# EUROPEAN PATENT APPLICATION

(11) **EP 3 916 019 A1**
(43) Date of publication of application: **01.12.2021**
(21) Application number: 19911196.4
(22) Date of filing: 16.12.2019
(51) Int. Cl.: C07K 16/30, C07K 14/705, C12N 5/0783, C12N 15/86, A61K 35/17, A61P 35/00

(54) **MESOTHELIN-SPECIFIC CHIMERIC ANTIGEN RECEPTOR AND T CELLS EXPRESSING SAME**

(30) Priority: 21.01.2019 KR 20190007422
(71) Applicant: Green Cross Cell Corporation, Giheung-gu Yongin-si, Gyeonggi-do 16924 (KR)
(72) Inventor: AHN, Jongseong, Yongin-si Gyeonggi-do 16924 (KR); KIM, Unkyo, Yongin-si Gyeonggi-do 16924 (KR); KIM, Irene, Yongin-si Gyeonggi-do 16924 (KR); KWON, Mi-Sun, Yongin-si Gyeonggi-do 16924 (KR); LEE, Hyeon Ho, Yongin-si Gyeonggi-do 16924 (KR)
(74) Representative: Mewburn Ellis LLP
(86) International application number: PCT/KR2019/017757
(87) International publication number: WO 2020/153605

(57) **Abstract**

The present invention relates to a chimeric antigen receptor and immune cells expressing same and, more specifically, to a chimeric antigen receptor and immune cells, preferably T cells, expressing same, the chimeric antigen receptor comprising: an anti-mesothelin antibody or a fragment thereof; a signal peptide of CD8α; a hinge of CD28 or CD8α; a transmembrane domain of CD28 or CD8α; and an intracellular signaling domain comprising intracellular signaling region sequences of CD28, 4-1BB, and CD3ζ. The chimeric antigen receptor and T cells expressing same according to the present invention can be effectively used for cancer treatment since the immune cells exhibit excellent anticancer activity.

## Description

### Technical Field

The present invention relates to a mesothelin-specific chimeric antigen receptor and T cells expressing the same.

### Background Art

T cells play important roles in adaptive immunity. T cells are activated through the stimulation of antigen-recognizing receptors (T-cell receptors, TCRs), co-stimulatory molecules, and cytokines. In addition, TCR signals are triggered by major histocompatibility complex (MHC) molecules bound to antigens leading to T cell activation but cancer cells evade immune system by downregulating the expression of the MHC molecule as a mechanism to avoid antigen recognition. Based on these characteristics of T cells, immune cell therapy (adoptive immune therapy, adoptive cellular therapy) is being developed.

Chimeric antigen receptors (CARs) are developed to allow T cells to directly recognize antigens without MHC molecules. A chimeric antigen receptor is composed of a single-chain variable fragment (scFv) to recognizes target antigen, a transmembrane domain, and a signaling domain that transmits signals into cells. Chimeric antigen receptors are artificially introduced into T cells, so that T cells (CAR-T cells) can recognize cancer cells with specific antigens. Currently, multinational pharmaceutical companies such as Novartis, Gilead, Juno Therapeutics, and Celgene in the United States developed CAR-T therapies for blood cancer as an indication, but studies on solid cancer associated therewith still remains a challenge.

Mesothelin (MSLN) is a 40-kDa glycoprotein expressed on the cell surface. MSLN is found to be seldom expressed in normal tissues, but to be over-expressed in several types of solid cancer, including mesothelioma, pancreatic cancer and ovarian cancer, and research thereon is underway as an anticancer target (Chang K 1996, Raffot. H 2004). Based thereon, a group led by Dr. Carl H. June at the University of Pennsylvania, USA, in collaboration with Novartis is researching mesothelin-targeting chimeric antigen receptors (ss1 CAR) containing mouse-derived anti-mesothelin scFv. ss1 CAR mRNA was delivered to T cells, and transiently expressed CAR T was administered to a patient. They found that a human anti-mouse antibody response (HAMA response) occurred in the patient (Beatty GL 2014).

The present inventors developed a chimeric antigen receptor comprising a human-derived scFv that specifically targets mesothelin, which can be used for the treatment of cancer expressing mesothelin to thereby solve the conventional problem of the human anti-mouse antibody response, and found that T cells expressing the chimeric antigen receptors comprising the human-derived mesothelin-specific scFv exhibit superior anti-tumor efficacy. Based on this finding, the present invention has been completed.

### Summary of the Invention

It is one object of the present invention to provide a novel chimeric antigen receptor comprising a mesothelin-binding domain that is capable of exhibiting excellent anticancer effects and solving the problem of an anticancer immune cell therapy using a conventional T cells expressing a chimeric antigen receptor comprising a mesothelin-binding domain, T cells expressing the chimeric antigen receptor, a composition for cancer treatment comprising the immune cells, and a method of treating cancer using the same.

It is another object of the present invention to provide the use of the T cells for cancer treatment.

It is another object of the present invention to provide the use of the T cells for the preparation of a therapeutic agent for treating cancer.

To achieve the above objects, the present invention provides a chimeric antigen receptor comprising a binding domain specifically binding to mesothelin (MSLN).

The binding domain to mesothelin (MSLN) according to the present invention is preferably an anti-mesothelin antibody or fragment thereof, but is not limited thereto.

The anti-mesothelin antibody or fragment thereof included in the chimeric antigen receptor according to the present invention comprises a heavy-chain variable region comprising a heavy-chain CDR1 having an amino acid sequence of SEQ ID NO: 45, 51, or 57, a heavy-chain CDR2 having an amino acid sequence of SEQ ID NO: 46, 52, or 58, and a heavy-chain CDR3 having an amino acid sequence of SEQ ID NO: 47, 53, or 59, and a light-chain variable region comprising a light-chain CDR1 having an amino acid sequence of SEQ ID NO: 48, 54, or 60, a light-chain CDR2 having an amino acid sequence of SEQ ID NO: 49, 55, or 61, and a light-chain CDR3 having an amino acid sequence of SEQ ID NO: 50, 56, or 62.

The present invention also provides a nucleic acid encoding a mesothelin-specific chimeric antigen receptor (CAR), an expression vector comprising the nucleic acid, immune cells expressing the chimeric antigen receptor, a composition for treating cancer comprising the immune cells, and a method for treating cancer using the same.

The present invention also provides the use of the immune cells for cancer treatment.

The present invention also provides the use of the immune cells for preparation of a therapeutic agent for treating cancer.

### Brief Description of Drawings

FIG. 1a shows the result of an enzyme-linked immunosorbent assay (ELISA), evaluating binding activity of MS501 scFv, MS503 scFv, or C2G4 scFv to recombinant human mesothelin (rhMSLN) according to an embodiment of the present invention.
FIG. 1b shows the result of flow cytometry analysis, evaluating cell binding activty of MS501 scFv, MS503 scFv, or C2G4 scFv to MIA PaCa2-MSLN cells according to an embodiment of the present invention.
FIG. 2a is a schematic diagram showing a CAR structure used in an *in-vitro* experiment according to an embodiment of the present invention.
FIG. 2b shows the result of flow cytometry analysis, identifying the CAR expression in Jurkat cells transduced with 501(28H)28z, 503(28H)28z or C2G4(28H)28z according to an embodiment of the present invention.
FIG. 2c shows the chimeric antigen receptor (CAR) and green fluorescent protein (GFP) expression of Jurkat cells transduced with 501(28H)28z, 503(28H)28z or C2G4(28H)28z according to an embodiment of the present invention.
FIG. 3a shows the result of flow cytometry analysis, determining the cellular activity of Jurkat cells transduced with 501(28H)28z, 503(28H)28z or C2G4(28H)28z according to an embodiment of the present invention upon response to target cells expressing mesothelin, namely, HeLa (cervical cancer) cells, OVCAR3 (ovarian cancer) cells and CAPAN1 (pancreatic cancer) cells.
FIG. 3b shows the result of an enzyme-linked immunoprecipitation assay, evaluating cytokine production capacity upon the mesothelin-specific activation of Jurkat cells transduced with 501(28H)28z, 503(28H)28z or C2G4(28H)28z in FIG. 3a.
FIG. 4a shows the result of flow cytometry analysis, determining cellular activity upon the mesothelin-specific activation of Jurkat cells transduced with 501(28H)28z, 503(28H)28z or C2G4(28H)28z according to an embodiment of the present invention.
FIG. 4b shows the result of an enzyme-linked immunoprecipitation assay, evaluating cytokine production capacity upon the mesothelin-specific activation of Jurkat cells transduced with 501(28H)28z, 503(28H)28z or C2G4(28H)28z in FIG. 4a.
FIG. 5a shows the result of flow cytometry analysis, determining the CAR expression of human T cells transduced with 501(28H)28z or C2G4(28H)28z according to an embodiment of the present invention.
FIG. 5b shows the cytotoxic activity upon mesothelin-specific response of the human T cells transduced with 501(28H)28z or C2G4(28H)28z in FIG. 5b.
FIG. 5c shows the result of an enzyme-linked immunoprecipitation assay, evaluating cytokine production capacity upon the mesothelin-specific activation of the human T cells transduced with 501(28H)28z, 503(28H)28z or C2G4(28H)28z in FIG. 5b.
FIG. 5d shows the cytotoxic activity of the human T cells transduced with 501(28H)28z, 503(28H)28z or C2G4(28H)28z against OVCAR3 (ovarian cancer) cells and CAPAN1 (pancreatic cancer) cells expressing mesothelin FIG. 5a.
FIG. 6a is a schematic diagram showing a CAR structure used in an *in-vivo* experiment according to an embodiment of the present invention.
FIG. 6b shows a change in tumor size after intratumoral injection of the human T cells transduced with 501(8H)Δ, 501(8H)z, 501(8H)BBz, 501(8H)28z or 501(28H)28z in FIG. 6a into a NCI-H226-xenografted mouse model.
FIG. 6c shows the weight of tumor tissue of a mouse harvested on the 20^{th} day after the intratumoral injection of the transduced human T cells into the tumor in FIG. 6b.
FIG. 6d shows the result of an enzyme-linked immunoprecipitation assay, evaluating cytokine production capacity of transduced human T cells in tumor tissues in FIG. 6c.
FIG. 6e shows body weight change of the mouse in FIG. 6b to investigate adverse effects.
FIG. 6f shows the survival (%) of mice at the end of the experiment in FIG. 6b.

### Detailed Description and Preferred Embodiments of the Invention

The present invention relates to a chimeric antigen receptor (CAR) comprising a binding domain specifically binding to mesothelin and T cells expressing the same. Hereinafter, the present invention will be described in detail.

The present invention is directed to a chimeric antigen receptor comprising a binding domain that specifically binds to mesothelin (MSLN).

The binding domain to mesothelin (MSLN) according to the present invention is preferably an anti-mesothelin antibody or a fragment thereof, but is not limited thereto.

The "fragment" of the antibody in the present invention refers to a fragment having an antigen-binding function, and includes scFv, Fab, F(ab')₂, Fv fragments, and the like.

The term "single chain Fv" or "scFv" antibody fragment includes the VH and VL domains of an antibody, and these domains are present in a single polypeptide chain. The Fv polypeptide may further include a polypeptide linker between the VH domain and the VL domain to allow the scFv to form a desired structure for antigen binding.

The "Fv" fragment is an antibody fragment containing complete antibody recognition and binding sites. Such a region includes a dimer that consists of one heavy-chain variable domain and one light-chain variable domain substantially tightly covalently linked to each other, for example scFv.

A "Fab" fragment contains a variable domain and a constant domain of the light chain and a variable domain and a first constant domain (CH1) of the heavy chain. A F(ab')₂ antibody fragment generally includes a pair of Fab fragments covalently linked near the carboxyl terminal thereof via a hinge cysteine therebetween.

The anti-mesothelin antibody or fragment thereof included in the chimeric antigen receptor according to the present invention comprises a heavy-chain variable region comprising a heavy-chain CDR1 having an amino acid sequence of SEQ ID NO: 45, 51, or 57, a heavy-chain CDR2 having an amino acid sequence of SEQ ID NO: 46, 52, or 58, and a heavy-chain CDR3 having an amino acid sequence of SEQ ID NO: 47, 53, or 59, and a light-chain variable region comprising a light-chain CDR1 having an amino acid sequence of SEQ ID NO: 48, 54, or 60, a light-chain CDR2 having an amino acid sequence of SEQ ID NO: 49, 55, or 61, and a light-chain CDR3 having an amino acid sequence of SEQ ID NO: 50, 56, or 62.

More specifically, the anti-mesothelin antibody or fragment thereof included in the chimeric antigen receptor according to the present invention may comprise: a heavy-chain CDR1 having an amino acid sequence of SEQ ID NO: 45, a heavy-chain CDR2 having an amino acid sequence of SEQ ID NO: 46, a heavy-chain CDR3 having an amino acid sequence of SEQ ID NO: 47, a light-chain CDR1 having an amino acid sequence of SEQ ID NO: 48, a light-chain CDR2 having an amino acid sequence of SEQ ID NO: 49, and a light-chain CDR3 having an amino acid sequence of SEQ ID NO: 50; a heavy-chain CDR1 having an amino acid sequence of SEQ ID NO: 51, a heavy-chain CDR2 having an amino acid sequence of SEQ ID NO: 52, a heavy-chain CDR3 having an amino acid sequence of SEQ ID NO: 53, a light-chain CDR1 having an amino acid sequence of SEQ ID NO: 54, a light-chain CDR2 having an amino acid sequence of SEQ ID NO: 55, and a light-chain CDR3 having an amino acid sequence of SEQ ID NO: 56; or a heavy-chain CDR1 having an amino acid sequence of SEQ ID NO: 57, a heavy-chain CDR2 having an amino acid sequence of SEQ ID NO: 58, a heavy-chain CDR3 having an amino acid sequence of SEQ ID NO: 59, a light-chain CDR1 having an amino acid sequence of SEQ ID NO: 60, a light-chain CDR2 having an amino acid sequence of SEQ ID NO: 61, and a light-chain CDR3 having an amino acid sequence of SEQ ID NO: 62.

Preferably, the anti-mesothelin antibody or fragment thereof included in the chimeric antigen receptor according to the present invention comprises a heavy-chain variable region having an amino acid sequence of SEQ ID NO: 39, 41, or 43, and a light-chain variable region having an amino acid sequence of SEQ ID NO: 40, 42, or 44.

More specifically, the anti-mesothelin antibody or fragment thereof included in the chimeric antigen receptor according to the present invention may comprise: a heavy-chain variable region having an amino acid sequence of SEQ ID NO: 39 and a light-chain variable region having an amino acid sequence of SEQ ID NO: 40; a heavy-chain variable region having an amino acid sequence of SEQ ID NO: 41 and a light-chain variable region having an amino acid sequence of SEQ ID NO: 42; or a heavy-chain variable region having an amino acid sequence of SEQ ID NO: 43 and a light-chain variable region having an amino acid sequence of SEQ ID NO: 44.

Most preferably, the anti-mesothelin antibody or fragment thereof included in the chimeric antigen receptor according to the present invention has an scFv form and preferably comprises an amino acid sequence represented by SEQ ID NO: 2, 4, or 6, but is not limited thereto.

The chimeric antigen receptor according to the present invention further comprises at least one selected from a signal peptide (SP), a hinge, a transmembrane domain, and an intracellular domain, along with the binding domain specifically binding to mesothelin.

The signal sequence that may be included in the chimeric antigen receptor according to the present invention is preferably a CD8α signal sequence, but is not limited thereto, and the CD8α signal sequence may have an amino acid sequence represented by SEQ ID NO: 8.

The signal sequence according to the present invention and the binding domain specifically binding to mesothelin constitute the extracellular domain of the chimeric antigen receptor. The extracellular domain is a site to which the main signal is transmitted, is present outside the cell membrane, and is a domain for specifically recognizing mesothelin.

Any transmembrane domain that may be included in the chimeric antigen receptor according to the present invention can be used, as long as it is capable of connecting the extracellular domain and the intracellular signaling domain via the cell membrane. Preferably, the transmembrane domain is composed of a transmembrane domain derived from CD28 and/or a transmembrane domain derived from CD8α, and comprises the entirety or part of the transmembrane domain derived from CD28 and/or the transmembrane domain derived from CD8α.

Preferably, the transmembrane domain may have an amino acid sequence represented by SEQ ID NO: 12 and/or 18, but is not limited thereto.

In an embodiment of the present invention, the extracellular domain and the transmembrane domain may be linked by a spacer domain.

Preferably, the spacer domain may be a hinge domain. The spacer domain that may be included in the chimeric antigen receptor according to the present invention may comprise a hinge domain derived from CD28 and/or a hinge domain derived from CD8α, and may comprise the entirety or part of the hinge domain derived from CD28 and/or the hinge domain derived from CD8α. In addition, the spacer domain may comprise the entirety or part of CD28 and/or CD8α.

Preferably, the hinge domain has an amino acid sequence represented by SEQ ID NO: 10 and/or 16, but is not limited thereto.

In a specific embodiment, in an *in-vivo* experiment using CAR structures of various combinations including MS501 scFv, combinations of MS501 scFv with a CD28 transmembrane domain and CD28-derived and CD3ζ-derived intracellular signaling domains (501(8H)28z and 501(28H)28z) exhibited unexpectedly superior anticancer effects of reducing the tumor size compared to other constructs (501(8H)z and 501(8H)BBz). In particular, 501(28H)28z with CD28 hinge showed more remarkable anticancer effect than 501(8H)28z with CD8α hinge.

In an embodiment of the present invention, the intracellular signaling domain is a part located inside the cell membrane of T cells, that is, in the cytoplasm, and is a site that mediates signal transduction for activation of immune cells when an antigen binding region of extracellular antibody domain recognizes a target antigen.

The chimeric antigen receptor according to the present invention may comprise two or more intracellular signaling domains. When two or more intracellular signaling domains are included, the intracellular signaling domains may be linked in tandem with each other. The intracellular signaling domain that may be included in the chimeric antigen receptor according to the present invention may have a structure in which an intracellular signaling domain derived from CD28 or 4-1BB is linked to an intracellular signaling domain derived from CD3ζ, and preferably has an amino acid sequence represented by SEQ ID NOS: 14, 20 and/or 22, but is not limited thereto.

In a specific embodiment, the chimeric antigen receptor according to the present invention may have at least one amino acid sequence selected from the group consisting of SEQ ID NOS: 24, 26, 28, 30, 32, 34 and 36, or a variant thereof having sequence identity of 80% or more, preferably 90% or more, more preferably 95% or more, and most preferably 99% or more with the amino acid sequence.

In addition, the present invention is directed to a nucleic acid encoding the chimeric antigen receptor according to the present invention described above. The polynucleotide (nucleic acid) encoding the antigen receptor of the present invention may be modified by codon optimization, which is due to codon degeneracy, and those skilled in the art will appreciate that there are many nucleotide sequences encoding polypeptides or variant fragments thereof. Some of these polynucleotides (nucleic acids) have minimal homology with the nucleotide sequence of any naturally occurring genes.

In particular, variable polynucleotides (nucleic acids) for example, polynucleotides (nucleic acids) optimized for codon selection of humans, primates and/or mammals) are preferred due to differences in codon utilization.

In an embodiment of the present invention, the nucleic acid sequence may comprise at least one nucleotide sequence selected from the group consisting of SEQ ID NOS: 23, 25, 27, 29, 31, 33 and 35, or a variant thereof having sequence identity of 80% or more, preferably 90% or more, more preferably 95% or more, and most preferably 99% or more with the nucleotide sequence.

The nucleic acid sequence encoding the CD8α signal sequence included in the chimeric antigen receptor according to the present invention may be represented by SEQ ID NO: 7; the nucleic acid sequence encoding the scFv included therein may be represented by SEQ ID NO: 1, 3, or 5; the nucleic acid sequence encoding the CD28 or CD8α hinge may be represented by SEQ ID NO: 9 or 15; the nucleic acid sequence encoding the CD28 or CD8α transmembrane domain may be represented by SEQ ID NO: 11 or 17; and the nucleic acid sequence encoding a CD28 intracellular signaling domain, a 4-1BB intracellular signaling domain, or a CD3ζ intracellular signaling domain may be represented by SEQ ID NO: 13, 19, or 21, but is not limited thereto.

The present invention is also directed to an expression vector comprising the nucleic acid and a virus comprising the expression vector.

As used herein, the term "vector" refers to a nucleic acid molecule capable of transferring or transporting nucleic acid molecule. The transferred nucleic acid is generally linked to a vector nucleic acid molecule, and is, for example, inserted into a vector nucleic acid molecule. The vector may include sequences directing autonomous replication in cells, or may include sequences sufficient to enable integration into the host cell DNA. The vector may be selected from the group consisting of DNA, RNA, plasmids, lentiviral vectors, adenovirus vectors, and retroviral vectors, but is not limited thereto.

As used herein, the term "virus" refers to a substance that is genetically modified to express the chimeric antigen receptor of the present invention for use in the treatment of cancer. The term "genetically modified" means the addition of foreign genetic material in the form of DNA or RNA to the total genetic material of a cell.

The present invention is directed to immune cells expressing the chimeric antigen receptor according to the present invention described above. The immune cells may be T cells, NK cells or NKT cells, but are not limited thereto, and are preferably T cells.

Therefore, the immune cells expressing the chimeric antigen receptor according to the present invention are CAR-T cells (chimeric antigen receptor T cells), CAR-NK cells (chimeric antigen receptor natural killer cells) or CAR-NKT cells (chimeric antigen receptor natural killer T cells).

In the present invention, the T cells are selected from the group consisting of T cells isolated from cytotoxic T lymphocytes (CTL), tumor-infiltrating lymphocytes (TIL), and peripheral blood mononuclear cells (PBMC).

The immune cells of the present invention may be toxic to tumor cells expressing mesothelin. In one embodiment, the immune cells (e.g., T cells) of the present invention are toxic to pancreatic cancer cells, cervical cancer cells, mesothelioma cells, or ovarian cancer cells. For example, the pancreatic cancer cells, cervical cancer cells, mesothelioma cells or ovarian cancer cells may express mesothelin.

In another aspect, the present invention is directed to a composition for treating cancer comprising the immune cells (for example, T cells) expressing the chimeric antigen receptor according to the present invention.

As used herein, the terms "cancer" and "tumor" are used interchangeably, and typically refer to or mean a physiological condition of a mammal characterized by unregulated cell growth/proliferation.

Examples of cancer (or tumors) include carcinoma, lymphoma (e.g., Hodgkin's and non-Hodgkin's lymphoma), blastoma, sarcoma, and leukemia. More preferably, examples of cancer include squamous cell cancer, small-cell lung cancer, non-small-cell lung cancer, pulmonary adenocarcinoma, pulmonary squamous cell carcinoma, peritoneal cancer, hepatocellular carcinoma, gastrointestinal cancer, pancreatic cancer, glioma, cervical cancer, ovarian cancer, liver cancer, bladder cancer, hepatocellular carcinoma, breast cancer, colon cancer, colorectal cancer, endometrial or uterine carcinoma, salivary gland carcinoma, kidney cancer, liver cancer, prostate cancer, vulvar cancer, thyroid cancer, liver carcinoma, mesothelioma, leukemia, other lymphoproliferative disorders, and various types of head and neck cancer, but are not limited thereto. In the present invention, the cancer is preferably mesothelin-positive cancer, and is selected from the group consisting of pancreatic cancer, ovarian cancer, lung cancer, gastric cancer, endometrial cancer and mesothelioma.

The composition according to the present invention is for preventing or treating cancer. As used herein, the term "preventing (or prevention)" refers to any action that inhibits the onset of cancer or delays the progression of cancer through administration of the composition according to the present invention, and the term "treating (or treatment)" refers to inhibition of the onset of cancer, or alleviation or elimination of symptoms of cancer through administration of the composition according to the present invention.

In the composition, the number of immune cells expressing the chimeric antigen receptor according to the present invention is preferably 1 to 10 times the number of tumor cells (e.g., pancreatic cancer, cervical cancer, mesothelioma or ovarian cancer) in a subject in need of treatment, but is not limited thereto.

The pharmaceutical composition comprising the immune cells expressing the chimeric antigen receptors according to the present invention may further comprise a pharmaceutically acceptable excipient. Examples of the excipient include: surfactants, preferably polysorbate nonionic surfactants; buffers such as neutral buffered saline and phosphate buffered saline; sugar or sugar alcohols such as glucose, mannose, sucrose, dextran, and mannitol; amino acids such as glycine and histidine, proteins, or polypeptides; antioxidants; chelating agents such as EDTA or glutathione; penetrants; adjuvants; and preservatives, but are not limited thereto.

In an embodiment of the present invention, the pharmaceutical composition of the present invention comprises the immune cells (e.g., T cells) in an amount corresponding to 1 to 10 times the number of tumor cells. For example, pancreatic cancer cells, cervical cancer cells, mesothelioma cells and ovarian cancer cells, within a single dose in the subject in need of treatment.

The present invention is directed to a method for treating cancer comprising administering the immune cells expressing the chimeric antigen receptor according to the present invention to a subject, and a method of preventing and treating tumor metastasis.

The present invention is directed to the use of the immune cells for cancer treatment.

The present invention is directed to the use of the immune cells for the preparation of a therapeutic agent for treating cancer.

The subject may be a tumor-bearing mammal, and specifically, may be a human, but is not limited thereto.

The immune cells expressing the chimeric antigen receptor or the composition comprising the same according to the present invention may be administered through oral administration, infusion, intravenous injection, intramuscular injection, subcutaneous injection, intraperitoneal administration, intrarectal administration, topical administration, intranasal injection, or the like, but the invention is not limited thereto.

Hereinafter, the present invention will be described in more detail with reference to examples. However, it will be obvious to those skilled in the art that these examples are provided only for illustration of the present invention, and should not be construed as limiting the scope of the present invention.

### Example 1: Methods and reagents

### 1.1 Cell lines and cell line culture

A human T-cell line Jurkat, a human pancreatic cancer cell line MIA PaCa2, a human cervical cancer cell line HeLa, a human ovarian cancer cell line OVCAR3, a human pancreatic cancer cell line CAPAN1, and a human mesothelioma cell line NCI-H226 were obtained from ATCC (American Type Culture Collection, Manassas, VA, USA). Jurkat cells and transformed Jurkat cells were cultured in RPMI-1640 (GIBCO, Grand Island, NY, USA) containing 10% FBS. MIA PaCa2 cells were cultured in DMEM (GIBCO, Grand Island, NY, USA) containing 10% FBS (fetal bovine serum; GIBCO, Grand Island, NY, USA). HeLa cells were cultured in DMEM (GIBCO) containing 10% FBS (GIBCO). OVCAR3 cells were cultured in RPMI-1640 (GIBCO) containing 20% FBS (GIBCO). CAPAN1 cells were cultured in IMDM (ATCC) containing 20% FBS (GIBCO). NCI-H226 cells were cultured in RPMI-1640 (GIBCO) containing 10% FBS (GIBCO). The HEK293T cell line, which is a human embryonic kidney fibroblast, was obtained from ATCC and used, and was cultured in DMEM (GIBCO) containing 10% FBS (GIBCO, Grand Island, NY, USA). Mesothelin-overexpressing human pancreatic cancer cell line MIA PaCa2-MSLN was obtained from MOGAM Institute for Biomedical Research (GC Green Cross, Korea) and used. MIA PaCa2-MSLN was cultured in DMEM (GIBCO) containing 10% FBS (GIBCO) and 200 µg/ml Hygromycin B (GIBCO).

### 1.2 Human T cell isolation and culture

Peripheral blood mononuclear cells (PBMC) were isolated from donors recruited under approval from the Institutional Review Board (IRB, Korea) using Ficoll-Hypaque (GE healthcare). The isolated PBMC were immediately frozen. Human T cells were isolated from the thawed PBMC with an EasySep (stem cells, Vancouver, BC, CA) kit using a negative selection method. Human T cells and anti-CD3/anti-CD28 magnetic beads (CD3/CD28 Dynabead, GIBCO) were mixed at a ratio of 1:3, followed by activation for 20 to 24 hours. Human T cells and transformed human T cells were stored in X-VIVO 15 (LONZA, Basel, Switzerland) containing 5% human serum (Sigma-Aldrich, Missouri, St. Louis, USA) and 50 IU/ml IL-2 (Proleukin, Novartis).

### 1.3 Identification of binding of scFv to recombinant human mesothelin

It was identified that the scFv according to the present invention binds to recombinant human mesothelin through the following procedure.
(i) MS501 scFv protein, MS503 scFv protein, and C2G4 scFv protein were coated on a 96-well immunoplate at 200 ng/ml for 24 hours.
(ii) The well was washed with a washing solution and blocked with a 1% BSA (bovine serum albumin, Sigma) solution for 1 hour.
(iii) The well was washed with a washing solution and treated with 100 ng/ml of recombinant human mesothelin protein for 2 hours.
(iv) The well was washed with a washing solution and seeded with a recombinant human mesothelin detection antibody diluted in accordance with a Certificate of Analysis and then treated for 1 hour.
(v) The well was washed with a washing solution and treated with streptavidin-HRP for 30 minutes.
(vi) The well was washed with the washing solution and then treated with a substrate solution for 15 minutes.
(vii) The well was treated with a stop solution and absorbance at 450 nm was measured.

1.4 Identification of binding to mesothelin-overexpressing cells

The degree of binding of the MS501 scFv protein, MS503 scFv protein, and C2G4 scFv protein to the mesothelin-overexpressing cell line, MIA PaCa2-MSLN, was detected. The MIA PaCa2-MSLN cell line suspended at 1 × 10⁵ cells/100 µl in FACS buffer was prepared. The MIA PaCa2-MSLN cell line was treated with 1 µg of each of MS501 scFv protein, MS503 scFv protein, and C2G4 scFv protein. The cells were washed twice using FACS buffer. The cells were stained with an anti-6x His tag antibody (BioLegend). Positive control samples were stained with an anti-mesothelin antibody (R&D system). The expression (%) and mean fluorescence intensity (MFI) of the stained cells were measured using BD LSRFortessa and analyzed using FlowJo software.

### 1.5 Construction of chimeric antigen receptor

In order to produce the chimeric antigen receptors of the present invention, the components set forth in Table 1 were artificially synthesized through SOE-PCR (splicing by overlapping extension by polymerase chain reaction). In more detail, (1) extracellular domains, namely, signal peptide of CD8α, scFv sequence (MS501 scFv sequence, MS503 scFv sequence or C2G4 scFv sequence), hinge of CD28 or hinge of CD8α, (2) transmembrane domains, namely, transmembrane domains of CD28 and CD8α, and (3) intracellular domains, namely intracellular signaling domains of CD28, 4-1BB and CD3ζ were artificially synthesized through SOE-PCR (splicing by overlapping extension through polymerase chain reaction). The gene recombination results were identified by direct sequencing. They cleaved with Nhe1 and EcoRI, and then ligated into the Nhe1 and EcoRI sites of the EF1α-MCS vector, which is a third-generation self-inactivating lentiviral expression vector.

The chimeric antigen receptor (CAR) according to an embodiment of the present invention is summarized in Table 1. All domains of CARs according to an embodiment of the present invention are linked in tandem to one another and are also linked in frame. 501(28H)28z has a structure in which the signal sequence domain of human CD8α (890-952 nucleotides, GenBank NM 001768.6), ScFv domain of MS501 IgG (Patent Application No.: 10-2015-0135755), human CD28-derived hinge, transmembrane domain, intracellular signaling domain (439-759 nucleotides, GenBank J02988.1), and CD3ζ-derived intracellular signaling domain (299-634 nucleotides, GenBank NM000734.3) are linked to a stop codon TGA. 503(28H)28z has a structure in which the signal sequence domain of human CD8α (890-952 nucleotides, GenBank NM 001768.6), ScFv domain of MS503 IgG (Patent Application No.: 10-2015-0135755), human CD28-derived hinge, transmembrane domain, intracellular signaling domain (439-759 nucleotides, GenBank J02988.1), and a CD3ζ-derived intracellular signaling domain (299-634 nucleotides, GenBank NM000734.3) are linked to a stop codon TGA. C2G4(28H)28z has a structure in which the signal sequence domain of human CD8α (890-952 nucleotides, GenBank NM 001768.6), ScFv domain of C2G4 IgG (Patent Application No.: 10-2015-0135755), human CD28-derived hinge, transmembrane domain and intracellular signaling domain (439-759 nucleotides, GenBank J02988.1), and a human CD3ζ-derived intracellular signaling domain (299-634 nucleotides, GenBank NM000734.3) are linked to a stop codon TGA. 501(8H)Δ has a structure in which the signal sequence domain of human CD8α (890-952 nucleotides, GenBank NM 001768.6), ScFv domain of MS501 IgG (Patent Application No.: 10-2015-0135755), and human CD8α-derived hinge and transmembrane domain (1292-1507 nucleotides, GenBank NM 001768.6) are linked to a stop codon TGA. 501(8H)z has a structure in which the signal sequence domain of human CD8α (890-952 nucleotides, GenBank NM 001768.6), ScFv domain of MS501 IgG (Patent Application No.: 10-2015-0135755), human CD8α-derived hinge and transmembrane domain (nucleotides 1292-1507, GenBank NM 001768.6), and a CD3ζ-derived intracellular signaling domain (299-634 nucleotides, GenBank NM000734.3) are linked to a stop codon TGA. 501(8H)BBz has a structure in which the signal sequence domain of human CD8α (890-952 nucleotides, GenBank NM 001768.6), ScFv domain of MS501 IgG (Patent Application No.: 10-2015-0135755), human CD8α-derived hinge and transmembrane domain (1292-1507 nucleotides, GenBank NM 001768.6) and human 4-1BB-derived intracellular signaling domain (901-1026 nucleotides, GenBank NM001561.5), and a CD3ζ-derived intracellular signaling domain (299-634 nucleotides, GenBank NM000734.3) are linked to a stop codon TGA. 501(8H)28z has a structure in which the signal sequence domain of human CD8α (890-952 nucleotides, GenBank NM 001768.6), ScFv domain of MS501 IgG (Patent Application No.: 10-2015-0135755), human CD8α-derived hinge (1292-1435 nucleotides, GenBank NM001768.6), human CD28-derived transmembrane domain and intracellular signaling domain (556-759 nucleotides, GenBank J02988.1), and a CD3ζ-derived intracellular signaling domain (299-634 nucleotides, GenBank NM000734.3) are linked to a stop codon TGA.

**[Table 1]**

| **Serial No.** | **Abbreviation** | **Signal sequence** | **scFv** | **Hinge** | **TM** | **Signal-1** | **Signal-2** |
|---|---|---|---|---|---|---|---|
| S1 | 501(28H)28z | CD8α | MS501 | CD28 | CD28 | CD28 | CD3ζ |
| S2 | 503(28H)28z | CD8α | MS503 | CD28 | CD28 | CD28 | CD3ζ |
| S3 | C2G4(28H)28z | CD8α | C2G4 | CD28 | CD28 | CD28 | CD3ζ |
| S4 | 501(8H)Δ | CD8α | MS501 | CD8a | CD8a | - | - |
| S5 | 501(8H)z | CD8α | MS501 | CD8a | CD8a | CD3ζ | - |
| S6 | 501(8H)BBz | CD8α | MS501 | CD8a | CD8a | 4-1BB | CD3ζ |
| S7 | 501(8H)28z | CD8α | MS501 | CD8a | CD8a | CD28 | CD3ζ |

The information of the sequences of the chimeric antigen receptors (CAR) and the domains used for production thereof according to an embodiment of the present invention are summarized in Tables 2 and 3.

**[Table 2]**

| **SEQ ID NO** | **Sequence name** | **Description of sequence** |
|---|---|---|
| 1 | MS501 scFv nucleotide | Patent Application No: 10-2015-0135755 |
| 2 | MS501 scFv amino acid | Amino acid sequence corresponding to SEQ ID NO: 1 |
| 3 | MS503 scFv nucleotide | Patent Application No.: 10-2015-0135755 |
| 4 | MS503 scFv amino acid | Amino acid sequence corresponding to SEQ ID NO: 3 |
| 5 | C2G4 scFv nucleotide | Patent Application No: 10-2015-0135755 |
| 6 | C2G4 scFv amino acid | Amino acid sequence corresponding to SEQ ID NO: 5 |
| 7 | CD8α signal sequence nucleotide | Signal sequence domain of CD8α (890-952 nucleotides, GenBank NM 001768.6) |
| 8 | CD8α signal sequence amino acid | Amino acid sequence corresponding to SEQ ID NO: 7 |
| 9 | CD28 hinge nucleotide | CD28-derived hinge (439-555 nucleotides, GenBank J02988.1) |
| 10 | CD28 hinge amino acid | Amino acid sequence corresponding to SEQ ID NO: 9 |
| 11 | CD28 TM nucleotide | CD28-derived transmembrane domain (TM)(556-636 nucleotides, GenBank J02988.1) |
| 12 | CD28 TM amino acid | Amino acid sequence corresponding to SEQ ID NO: 11 |
| 13 | CD28 SD nucleotide | CD28 -derived intracellular signaling domain (SD)(637-759 nucleotides, GenBank J02988.1) |
| 14 | CD28 SD amino acid | Amino acid sequence corresponding to SEQ ID NO: 13 |
| 15 | CD8α hinge nucleotide | CD8α-derived hinge (1292-1435 nucleotides, GenBank NM 001768.6) |
| 16 | CD8α hinge amino acid | Amino acid sequence corresponding to SEQ ID NO: 15 |
| 17 | CD8α TM nucleotide | CD8α-derived hinge (1436-1507 nucleotides, GenBank NM 001768.6) |
| 18 | CD8α TM amino acid | Amino acid sequence corresponding to SEQ ID NO: 17 |
| 19 | 4-IBB SD nucleotide | 4-1BB-derived intracellular signaling domain (SD) (901-1026 nucleotides, GenBank NM 001561.5) |
| 20 | 4-IBB SD amino acid | Amino acid sequence corresponding to SEQ ID NO: 19 |
| 21 | CD3ζ nucleotide | CD3ζ-derived intracellular signaling domain (299-634 nucleotides, GenBank NM 000734.3) |
| 22 | CD3ζ amino acid | Amino acid sequence corresponding to SEQ ID NO: 21 |
| 23 | 501(28H)28z nucleotide | [Table 1] nucleotide sequence linked in tandem in S1 |
| 24 | 501(28H)28z amino acid | Amino acid sequence corresponding to SEQ ID NO: 23 |
| 25 | 503(28H)28z nucleotide | [Table 1] nucleotide sequence linked in tandem in S2 |
| 26 | 503(28H)28z amino acid | Amino acid sequence corresponding to SEQ ID NO: 25 |
| 27 | C2G4(28H)28z nucleotide | [Table 1] nucleotide sequence linked in tandem in S3 |
| 28 | C2G4(28H)28z amino acid | Amino acid sequence corresponding to SEQ ID NO: 27 |
| 29 | 501(8H)Δ nucleotide | [Table 1] nucleotide sequence linked in tandem in S4 |
| 30 | 501(8H)Δ amino acid | Amino acid sequence corresponding to SEQ ID NO: 29 |
| 31 | 501(8H)z nucleotide | [Table 1] nucleotide sequence linked in tandem in S5 |
| 32 | 501(8H)z amino acid | Amino acid sequence corresponding to SEQ ID NO: 31 |
| 33 | 501(8H)BBz nucleotide | [Table 1] nucleotide sequence linked in tandem in S6 |
| 34 | 501(8H)BBz amino acid | Amino acid sequence corresponding to SEQ ID NO: 33 |
| 35 | 501(8H)28z nucleotide | [Table 1] nucleotide sequence linked in tandem in S7 |
| 36 | 501(8H)28z amino acid | Amino acid sequence corresponding to SEQ ID NO: 35 |
| 37 | Linker nucleotide | SEQ ID NO: 38-encoding nucleotide sequence |
| 38 | Linker amino acid | GGGGSGGGGSGGGGS |

**[Table 3]**

| **SEQ ID NO** | **Sequence name** | **Sequence** |
|---|---|---|
| 1 | MS501 scFv nucleotide | GAGGTGCAGCTGTTGGAGTCCGGGGGAGGCTTGGTACA GCCTGGGGGGTCCCTGAGACTCTCCTGTGCAGCCTCTGG ATTCACCTTTAGCAATTATGCTATGAGCTGGGTCCGCCA GGCTCCAGGGAAGGGGCTGGAGTGGGTCTCAGGGATCT ATCCCGATAGTGGTAGTACATATTACGCTGATTCTGTAA AAGGTCGGTTCACCATCTCCAGAGACAATTCCAAGAACA CGCTGTATCTGCAAATGAACAGCCTGAGAGCCGAGGAC ACGGCCGTGTATTACTGTGCGAGAAATATTTATACGTTC GACTACTGGGGCCAGGGTACACTGGTCACCGTGAGCTCA GGTGGAGGCGGTTCAGGCGGAGGTGGATCCGGCGGTGG CGGATCGCAGTCTGTGCTGACTCAGCCACCCTCAGCGTC TGGGACCCCCGGGCAGAGGGTCACCATCTCTTGTAGTGG CTCTTCATCTAATATTGGCAGTAATGCTGTCTCCTGGTAC CAGCAGCTCCCAGGAACGGCCCCCAAACTCCTCATCTAT TATAATAATCAGCGGCCAAGCGGGGTCCCTGACCGATTC TCTGGCTCCAAGTCTGGCACCTCAGCCTCCCTGGCCATC AGTGGGCTCCGGTCCGAGGATGAGGCTGATTATTACTGT GGTTCTTGGGATTCTAGCCTGAGTGGATATGTCTTCGGC GGAGGCACCAAGCTGACGGTCCTAGGC |
| 2 | MS501 scFv amino acid | EVQLLESGGGLVQPGGSLRLSCAASGFTFSNYAMSWVRQA PGKGLEWVSGIYPDSGSTYYADSVKGRFTISRDNSKNTLYL QMNSLRAEDTAVYYCARNIYTFDYWGQGTLVTVSSGGGG SGGGGSGGGGSQSVLTQPPSASGTPGQRVTISCSGSSSNIGS |
| | | NAVSWYQQLPGTAPKLLIYYNNQRPSGVPDRFSGSKSGTS ASLAISGLRSEDEADYYCGSWDSSLSGYVFGGGTKLTVLG |
| 3 | MS503 scFv nucleotide | GAGGTGCAGCTGTTGGAGTCCGGGGGAGGCTTGGTACA GCCTGGGGGGTCCCTGAGACTCTCCTGTGCAGCCTCTGG ATTCACCTTTAGCAATTATGCTATGAGCTGGGTCCGCCA GGCTCCAGGGAAGGGGCTGGAGTGGGTCTCATCGATCT ATCCTGGTGATGGTAGTACATATTACGCTGATTCTGTAA AAGGTCGGTTCACCATCTCCAGAGACAATTCCAAGAACA CGCTGTATCTGCAAATGAACAGCCTGAGAGCCGAGGAC ACGGCCGTGTATTACTGTGCGAAAAATGCGTTTACGTTC GACTACTGGGGCCAGGGTACACTGGTCACCGTGAGCTCA GGTGGAGGCGGTTCAGGCGGAGGTGGATCCGGCGGTGG CGGATCGCAGTCTGTGCTGACTCAGCCACCCTCAGCGTC TGGGACCCCCGGGCAGAGGGTCACCATCTCTTGTAGTGG CTCTTCATCTAATATTGGCAGTAATGCTGTCTCCTGGTAC CAGCAGCTCCCAGGAACGGCCCCCAAACTCCTCATCTAT TATAATAGTCATCGGCCAAGCGGGGTCCCTGACCGATTC TCTGGCTCCAAGTCTGGCACCTCAGCCTCCCTGGCCATC AGTGGGCTCCGGTCCGAGGATGAGGCTGATTATTACTGT GGTACTTGGGATTCTAGCCTGAGTGGTTATGTCTTCGGC GGAGGCACCAAGCTGACGGTCCTAGGC |
| 4 | MS503 scFv amino acid | EVQLLESGGGLVQPGGSLRLSCAASGFTFSNYAMSWVRQA PGKGLEWVSSIYPGDGSTYYADSVKGRFTISRDNSKNTLYL QMNSLRAEDTAVYYCAKNAFTFDYWGQGTLVTVSSGGG GSGGGGSGGGGSQSVLTQPPSASGTPGQRVTISCSGSSSNIG |
| | | SNAVSWYQQLPGTAPKLLIYYNSHRPSGVPDRFSGSKSGTS ASLAISGLRSEDEADYYCGTWDSSLSGYVFGGGTKLTVLG |
| 5 | C2G4 scFv nucleotide | GAGGTGCAGCTGTTGGAGTCTGGGGGAGGCTTGGTACA GCCTGGGGGGTCCCTGAGACTCTCCTGTGCGGCCTCTGG ATTCACCTTTAGCAATTATGCTATGAGCTGGGTCCGCCA GGCTCCAGGGAAGGGGCTGGAGTGGGTCTCAGGGATCC CTCCTGATAGTGGTAGTAAATATTACGCTGATTCTGTAA GAGGTCGGTTCACCGTCTCCAGAGACAATTCCAAGAACA CGCTGTATCTGCAAATGAACAGCCTGAGAGCCGAGGAC ACGGCCGTGTATTACTGTGCGAAAAATATGCTGTCGTTC GACTACTGGGGCCAGGGTACACTGGTCACCGTGAGCTCA GGTGGAGGCGGTTCAGGCGGAGGTGGATCCGGCGGTGG CGGATCGCAGTCTGTGCTGACTCAGCCACCCTCAGCGTC TGGGACCCCCGGGCAGAGGGTCACCATCTCTTGTACTGG CTCTTCATCTAATATTGGCTCCAATGCTGTCTCCTGGTAC CAGCAGCTCCCAGGAACGGCCCCCAAACTCCTCATCTAT TATAATAGTAAGCGGCCAAGCGGGGTCCCTGACCGATTC TCTGGCTCCAAGTCTGGCACCTCAGCCTCCCTGGCCATC AGTGGGCTCCGGTCCGAGGATGAGGCTGATTATTACTGT GGTTCTTGGGATCCTAGCCTGAATGGTTATGTCTTCGGC GGAGGCACCAAGGTGACCGTCCTAGGT |
| 6 | C2G4 scFv amino acid | EVQLLESGGGLVQPGGSLRLSCAASGFTFSNYAMSWVRQA PGKGLEWVSGIPPDSGSKYYADSVRGRFTVSRDNSKNTLY LQMNSLRAEDTAVYYCAKNMLSFDYWGQGTLVTVSSGG GGSGGGGSGGGGSQSVLTQPPSASGTPGQRVTISCTGSSSNI |
| | | GSNAVSWYQQLPGTAPKLLIYYNSKRPSGVPDRFSGSKSGT SASLAISGLRSEDEADYYCGSWDPSLNGYVFGGGTKVTVL G |
| 7 | CD8α signal sequence nucleotide | ATGGCCTTACCAGTGACCGCCTTGCTCCTGCCGCTGGCC TTGCTGCTCCACGCCGCCAGGCCG |
| 8 | CD8α signal sequence amino acid | MALPVTALLLPLALLLHAARP |
| 9 | CD28 hinge nucleotide | ATTGAAGTTATGTATCCTCCTCCTTACCTAGACAATGAG AAGAGCAATGGAACCATTATCCATGTGAAAGGAAACA CCTTTGTCCAAGTCCCCTATTTCCCGGACCTTCTAAGCCC |
| 10 | CD28 hinge amino acid | IEVMYPPPYLDNEKSNGTIIHVKGKHLCPSPLFPGPSKP |
| 11 | CD28 TM nucleotide | TTTTGGGTGCTGGTGGTGGTTGGTGGAGTCCTGGCTTGC TATAGCTTGCTAGTAACAGTGGCCTTTATTATTTCTGGG TG |
| 12 | CD28 TM amino acid | FWVLVVVGGVLACYSLLVTVAFIIFWV |
| 13 | CD28 SD nucleotide | AGGAGTAAGAGGAGCAGGCTCCTGCACAGTGACTACAT GAACATGACTCCCCGCCGCCCCGGGCCCACCCGCAAGC ATTACCAGCCCTATGCCCCACCACGCGACTTCGCAGCCT ATCGCTCC |
| 14 | CD28 SD amino acid | RSKRSRLLHSDYMNMTPRRPGPTRKHYQPYAPPRDFAAYR S |
| 15 | CD8α Hinge nucleotide | GCGAAGCCCACCACGACGCCAGCGCCGCGACCACCAAC ACCGGCGCCCACCATCGCGTCGCAGCCCCTGTCCCTGCG CCCAGAGGCGTGCCGGCCAGCGGCGGGGGCGCAGTGC ACACGAGGGGGCTGGACTTCGCCTGTGAT |
| 16 | CD8α hinge amino acid | AKPTTTPAPRPPTPAPTIASQPLSLRPEACRPAAGGAVHTRG LDFACD |
| 17 | CD8α TM nucleotide | ATCTACATCTGGGCGCCCTTGGCCGGGACTTGTGGGGTC CTTCTCCTGTCACTGGTTATCACCCTTTACTGC |
| 18 | CD8α TM amino acid | IYIWAPLAGTCGVLLLSLVITLYC |
| 19 | 4-1BB SD nucleotide | AAACGGGGCAGAAAGAAACTCCTGTATATATTCAAACA ACCATTTATGAGACCAGTACAAACTACTCAAGAGGAAG ATGGCTGTAGCTGCCGATTTCCAGAAGAAGAAGAAGGA GGATGTGAACTG |
| 20 | 4-IBB SD amino acid | KRGRKKLLYIFKQPFMRPVQTTQEEDGCSCRFPEEEEGGCE L |
| 21 | CD3ζ nucleotide | AGAGTGAAGTTCAGCAGGAGCGCAGACGCCCCCGCGTA CCAGCAGGGCCAGAACCAGCTCTATAACGAGCTCAATCT AGGACGAAGAGAGGAGTACGATGTTTTGGACAAGAGAC GTGGCCGGGACCCTGAGATGGGGGGAAAGCCGAGAAGG AAGAACCCTCAGGAAGGCCTGTACAATGAACTGCAGAA AGATAAGATGGCGGAGGCCTACAGTGAGATTGGGATGA AAGGCGAGCGCCGGAGGGGCAAGGGGCACGATGGCCTT TACCAGGGTCTCAGTACAGCCACCAAGGACACCTACGA CGCCCTTCACATGCAGGCCCTGCCCCCTCGC |
| 22 | CD3ζ amino acid | RVKFSRSADAPAYQQGQNQLYNELNLGRREEYDVLDKRR GRDPEMGGKPRRKNPQEGLYNELQKDKMAEAYSEIGMKG ERRRGKGHDGLYQGLSTATKDTYDALHMQALPPR |
| 23 | 501(28H)28z nucleotide | ATGGCCTTACCAGTGACCGCCTTGCTCCTGCCGCTGGCC TTGCTGCTCCACGCCGCCAGGCCGGAGGTGCAGCTGTTG GAGTCCGGGGGAGGCTTGGTACAGCCTGGGGGGTCCCT GAGACTCTCCTGTGCAGCCTCTGGATTCACCTTTAGCAA TTATGCTATGAGCTGGGTCCGCCAGGCTCCAGGGAAGGG GCTGGAGTGGGTCTCAGGGATCTATCCCGATAGTGGTAG TACATATTACGCTGATTCTGTAAAAGGTCGGTTCACCAT CTCCAGAGACAATTCCAAGAACACGCTGTATCTGCAAAT GAACAGCCTGAGAGCCGAGGACACGGCCGTGTATTACT GTGCGAGAAATATTTATACGTTCGACTACTGGGGCCAGG GTACACTGGTCACCGTGAGCTCAGGTGGAGGCGGTTCAG GCGGAGGTGGATCCGGCGGTGGCGGATCGCAGTCTGTG CTGACTCAGCCACCCTCAGCGTCTGGGACCCCCGGGCAG AGGGTCACCATCTCTTGTAGTGGCTCTTCATCTAATATTG GCAGTAATGCTGTCTCCTGGTACCAGCAGCTCCCAGGAA CGGCCCCCAAACTCCTCATCTATTATAATAATCAGCGGC CAAGCGGGGTCCCTGACCGATTCTCTGGCTCCAAGTCTG GCACCTCAGCCTCCCTGGCCATCAGTGGGCTCCGGTCCG AGGATGAGGCTGATTATTACTGTGGTTCTTGGGATTCTA GCCTGAGTGGATATGTCTTCGGCGGAGGCACCAAGCTGA CGGTCCTAGGCATTGAAGTTATGTATCCTCCTCCTTACCT AGACAATGAGAAGAGCAATGGAACCATTATCCATGTGA |
| | | AAGGGAAACACCTTTGTCCAAGTCCCCTATTTCCCGGAC CTTCTAAGCCCTTTTGGGTGCTGGTGGTTGGTGGAG TCCTGGCTTGCTATAGCTTGCTAGTAACAGTGGCCTTTAT TATTTTCTGGGTGAGGAGTAAGAGGAGCAGGCTCCTGCA CAGTGACTACATGAACATGACTCCCCGCCGCCCCGGGCC CACCCGCAAGCATTACCAGCCCTATGCCCCACCACGCGA CTTCGCAGCCTATCGCTCCAGAGTGAAGTTCAGCAGGAG CGCAGACGCCCCCGCGTACCAGCAGGGCCAGAACCAGC TCTATAACGAGCTCAATCTAGGACGAAGAGAGGAGTAC GATGTTTTGGACAAGAGACGTGGCCGGGACCCTGAGAT GGGGGGAAAGCCGAGAAGGAAGAACCCTCAGGAAGGC CTGTACAATGAACTGCAGAAAGATAAGATGGCGGAGGC CTACAGTGAGATTGGGATGAAAGGCGAGCGCCGGAGGG GCAAGGGGCACGATGGCCTTTACCAGGGTCTCAGTACA GCCACCAAGGACACCTACGACGCCCTTCACATGCAGGCC CTGCCCCCTCGCTGA |
| 24 | 501(28H)28z amino acid | MALPVTALLLPLALLLHAARPEVQLLESGGGLVQPGGSLR LSCAASGFTFSNYAMSWVRQAPGKGLEWVSGIYPDSGSTY YADSVKGRFTISRDNSKNTLYLQMNSLRAEDTAVYYCARN IYTFDYWGQGTLVTVSSGGGGSGGGGSGGGGSQSVLTQPP SASGTPGQRVTISCSGSSSNIGSNAVSWYQQLPGTAPKLLIY YNNQRPSGVPDRFSGSKSGTSASLAISGLRSEDEADYYCGS WDSSLSGYVFGGGTKLTVLGIEVMYPPPYLDNEKSNGTIIH VKGKHLCPSPLFPGPSKPFWVLVVVGGVLACYSLLVTVAFI IFWVRSKRSRLLHSDYMNMTPRRPGPTRKHYQPYAPPRDF |
| | | AAYRSRVKFSRSADAPAYQQGQNQLYNELNLGRREEYDV LDKRRGRDPEMGGKPRRKNPQEGLYNELQKDKMAEAYSE IGMKGERRRGKGHDGLYQGLSTATKDTYDALHMQALPPR |
| 25 | 503(28H)28z nucleotide | ATGGCCTTACCAGTGACCGCCTTGCTCCTGCCGCTGGCC TTGCTGCTCCACGCCGCCAGGCCGGAGGTGCAGCTGTTG GAGTCCGGGGGAGGCTTGGTACAGCCTGGGGGGTCCCT GAGACTCTCCTGTGCAGCCTCTGGATTCACCTTTAGCAA TTATGCTATGAGCTGGGTCCGCCAGGCTCCAGGGAAGGG GCTGGAGTGGGTCTCATCGATCTATCCTGGTGATGGTAG TACATATTACGCTGATTCTGTAAAAGGTCGGTTCACCAT CTCCAGAGACAATTCCAAGAACACGCTGTATCTGCAAAT GAACAGCCTGAGAGCCGAGGACACGGCCGTGTATTACT GTGCGAAAAATGCGTTTACGTTCGACTACTGGGGCCAGG GTACACTGGTCACCGTGAGCTCAGGTGGAGGCGGTTCAG GCGGAGGTGGATCCGGCGGTGGCGGATCGCAGTCTGTG CTGACTCAGCCACCCTCAGCGTCTGGGACCCCCGGGCAG AGGGTCACCATCTCTTGTAGTGGCTCTTCATCTAATATTG GCAGTAATGCTGTCTCCTGGTACCAGCAGCTCCCAGGAA CGGCCCCCAAACTCCTCATCTATTATAATAGTCATCGGC CAAGCGGGGTCCCTGACCGATTCTCTGGCTCCAAGTCTG GCACCTCAGCCTCCCTGGCCATCAGTGGGCTCCGGTCCG AGGATGAGGCTGATTATTACTGTGGTACTTGGGATTCTA GCCTGAGTGGTTATGTCTTCGGCGGAGGCACCAAGCTGA CGGTCCTAGGCATTGAAGTTATGTATCCTCCTCCTTACCT AGACAATGAGAAGAGCAATGGAACCATTATCCATGTGA |
| | | AAGGGAAACACCTTTGTCCAAGTCCCCTATTTCCCGGAC CTTCTAAGCCCTTTTGGGTGCTGGTGGTGGTTGGTGGAG TCCTGGCTTGCTATAGCTTGCTAGTAACAGTGGCCTTTAT TATTTTCTGGGTGAGGAGTAAGAGGAGCAGGCTCCTGCA CAGTGACTACATGAACATGACTCCCCGCCGCCCCGGGCC CACCCGCAAGCATTACCAGCCCTATGCCCCACCACGCGA CTTCGCAGCCTATCGCTCCAGAGTGAAGTTCAGCAGGAG CGCAGACGCCCCCGCGTACCAGCAGGGCCAGAACCAGC TCTATAACGAGCTCAATCTAGGACGAAGAGAGGAGTAC GATGTTTTGGACAAGAGACGTGGCCGGGACCCTGAGAT GGGGGGAAAGCCGAGAAGGAAGAACCCTCAGGAAGGC CTGTACAATGAACTGCAGAAAGATAAGATGGCGGAGGC CTACAGTGAGATTGGGATGAAAGGCGAGCGCCGGAGGG GCAAGGGGCACGATGGCCTTTACCAGGGTCTCAGTACA GCCACCAAGGACACCTACGACGCCCTTCACATGCAGGCC CTGCCCCCTCGCTGA |
| 26 | 503(28H)28z amino acid | MALPVTALLLPLALLLHAARPEVQLLESGGGLVQPGGSLR LSCAASGFTFSNYAMSWVRQAPGKGLEWVSSIYPGDGSTY YADSVKGRFTISRDNSKNTLYLQMNSLRAEDTAVYYCAK NAFTFDYWGQGTLVTVSSGGGGSGGGGSGGGGSQSVLTQ PPSASGTPGQRVTISCSGSSSNIGSNAVSWYQQLPGTAPKLL IYYNSHRPSGVPDRFSGSKSGTSASLAISGLRSEDEADYYC GTWDSSLSGYVFGGGTKLTVLGIEVMYPPPYLDNEKSNGTI IHVKGKHLCPSPLFPGPSKPFWVLVVVGGVLACYSLLVTV AFIIFWVRSKRSRLLHSDYMNMTPRRPGPTRKHYQPYAPPR |
| | | DFAAYRSRVKFSRSADAPAYQQGQNQLYNELNLGRREEY DVLDKRRGRDPEMGGKPRRKNPQEGLYNELQKDKMAEA YSEIGMKGERRRGKGHDGLYQGLSTATKDTYDALHMQAL PPR |
| 27 | C2G4(28H)28z nucleotide | ATGGCCTTACCAGTGACCGCCTTGCTCCTGCCGCTGGCC TTGCTGCTCCACGCCGCCAGGCCGGAGGTGCAGCTGTTG GAGTCTGGGGGAGGCTTGGTACAGCCTGGGGGGTCCCT GAGACTCTCCTGTGCGGCCTCTGGATTCACCTTTAGCAA TTATGCTATGAGCTGGGTCCGCCAGGCTCCAGGGAAGGG GCTGGAGTGGGTCTCAGGGATCCCTCCTGATAGTGGTAG TAAATATTACGCTGATTCTGTAAGAGGTCGGTTCACCGT CTCCAGAGACAATTCCAAGAACACGCTGTATCTGCAAAT GAACAGCCTGAGAGCCGAGGACACGGCCGTGTATTACT GTGCGAAAAATATGCTGTCGTTCGACTACTGGGGCCAGG GTACACTGGTCACCGTGAGCTCAGGTGGAGGCGGTTCAG GCGGAGGTGGATCCGGCGGTGGCGGATCGCAGTCTGTG CTGACTCAGCCACCCTCAGCGTCTGGGACCCCCGGGCAG AGGGTCACCATCTCTTGTACTGGCTCTTCATCTAATATTG GCTCCAATGCTGTCTCCTGGTACCAGCAGCTCCCAGGAA CGGCCCCCAAACTCCTCATCTATTATAATAGTAAGCGGC CAAGCGGGGTCCCTGACCGATTCTCTGGCTCCAAGTCTG GCACCTCAGCCTCCCTGGCCATCAGTGGGCTCCGGTCCG AGGATGAGGCTGATTATTACTGTGGTTCTTGGGATCCTA GCCTGAATGGTTATGTCTTCGGCGGAGGCACCAAGGTGA CCGTCCTAGGTATTGAAGTTATGTATCCTCCTCCTTACCT |
| | | AGACAATGAGAAGAGCAATGGAACCATTATCCATGTGA AAGGGAAACACCTTTGTCCAAGTCCCCTATTTCCCGGAC CTTCTAAGCCCTTTTGGGTGCTGGTGGTGGTTGGTGGAG TCCTGGCTTGCTATAGCTTGCTAGTAACAGTGGCCTTTAT TATTTTCTGGGTGAGGAGTAAGAGGAGCAGGCTCCTGCA CAGTGACTACATGAACATGACTCCCCGCCGCCCCGGGCC CACCCGCAAGCATTACCAGCCCTATGCCCCACCACGCGA CTTCGCAGCCTATCGCTCCAGAGTGAAGTTCAGCAGGAG CGCAGACGCCCCCGCGTACCAGCAGGGCCAGAACCAGC TCTATAACGAGCTCAATCTAGGACGAAGAGAGGAGTAC GATGTTTTGGACAAGAGACGTGGCCGGGACCCTGAGAT GGGGGGAAAGCCGAGAAGGAAGAACCCTCAGGAAGGC CTGTACAATGAACTGCAGAAAGATAAGATGGCGGAGGC CTACAGTGAGATTGGGATGAAAGGCGAGCGCCGGAGGG GCAAGGGGCACGATGGCCTTTACCAGGGTCTCAGTACA GCCACCAAGGACACCTACGACGCCCTTCACATGCAGGCC CTGCCCCCTCGCTGA |
| 28 | C2G4(28H)28z amino acid | MALPVTALLLPLALLLHAARPEVQLLESGGGLVQPGGSLR LSCAASGFTFSNYAMSWVRQAPGKGLEWVSGIPPDSGSKY YADSVRGRFTVSRDNSKNTLYLQMNSLRAEDTAVYYCAK NMLSFDYWGQGTLVTVSSGGGGSGGGGSGGGGSQSVLTQ PPSASGTPGQRVTISCTGSSSNIGSNAVSWYQQLPGTAPKLL IYYNSKRPSGVPDRFSGSKSGTSASLAISGLRSEDEADYYC GSWDPSLNGYVFGGGTKVTVLGIEVMYPPPYLDNEKSNGT IIHVKGKHLCPSPLFPGPSKPFWVLVVVGGVLACYSLLVTV |
| | | AFIIFWVRSKRSRLLHSDYMNMTPRRPGPTRKHYQPYAPPR DFAAYRSRVKFSRSADAPAYQQGQNQLYNELNLGRREEY DVLDKRRGRDPEMGGKPRRKNPQEGLYNELQKDKMAEA YSEIGMKGERRRGKGHDGLYQGLSTATKDTYDALHMQAL PPR |
| 29 | 501(8H)Δ nucleotide | ATGGCCTTACCAGTGACCGCCTTGCTCCTGCCGCTGGCC TTGCTGCTCCACGCCGCCAGGCCGGAGGTGCAGCTGTTG GAGTCCGGGGGAGGCTTGGTACAGCCTGGGGGGTCCCT GAGACTCTCCTGTGCAGCCTCTGGATTCACCTTTAGCAA TTATGCTATGAGCTGGGTCCGCCAGGCTCCAGGGAAGGG GCTGGAGTGGGTCTCAGGGATCTATCCCGATAGTGGTAG TACATATTACGCTGATTCTGTAAAAGGTCGGTTCACCAT CTCCAGAGACAATTCCAAGAACACGCTGTATCTGCAAAT GAACAGCCTGAGAGCCGAGGACACGGCCGTGTATTACT GTGCGAGAAATATTTATACGTTCGACTACTGGGGCCAGG GTACACTGGTCACCGTGAGCTCAGGTGGAGGCGGTTCAG GCGGAGGTGGATCCGGCGGTGGCGGATCGCAGTCTGTG CTGACTCAGCCACCCTCAGCGTCTGGGACCCCCGGGCAG AGGGTCACCATCTCTTGTAGTGGCTCTTCATCTAATATTG GCAGTAATGCTGTCTCCTGGTACCAGCAGCTCCCAGGAA CGGCCCCCAAACTCCTCATCTATTATAATAATCAGCGGC CAAGCGGGGTCCCTGACCGATTCTCTGGCTCCAAGTCTG GCACCTCAGCCTCCCTGGCCATCAGTGGGCTCCGGTCCG AGGATGAGGCTGATTATTACTGTGGTTCTTGGGATTCTA GCCTGAGTGGATATGTCTTCGGCGGAGGCACCAAGCTGA |
| | | CGGTCCTAGGCGCGAAGCCCACCACGACGCCAGCGCCG CGACCACCAACACCGGCGCCCACCATCGCGTCGCAGCCC CTGTCCCTGCGCCCAGAGGCGTGCCGGCCAGCGGCGGG GGGCGCAGTGCACACGAGGGGGCTGGACTTCGCCTGTG ATATCTACATCTGGGCGCCCTTGGCCGGGACTTGTGGGG TCCTTCTCCTGTCACTGGTTATCACCCTTTACTGCTGA |
| 30 | 501(8H)Δ amino acid | MALPVTALLLPLALLLHAARPEVQLLESGGGLVQPGGSLR LSCAASGFTFSNYAMSWVRQAPGKGLEWVSGIYPDSGSTY YADSVKGRFTISRDNSKNTLYLQMNSLRAEDTAVYYCARN IYTFDYWGQGTLVTVSSGGGGSGGGGSGGGGSQSVLTQPP SASGTPGQRVTISCSGSSSNIGSNAVSWYQQLPGTAPKLLIY YNNQRPSGVPDRFSGSKSGTSASLAISGLRSEDEADYYCGS WDSSLSGYVFGGGTKLTVLGAKPTTTPAPRPPTPAPTIASQ PLSLRPEACRPAAGGAVHTRGLDFACDIYIWAPLAGTCGV LLLSLVITLYC |
| 31 | 501(8H)z nucleotide | ATGGCCTTACCAGTGACCGCCTTGCTCCTGCCGCTGGCC TTGCTGCTCCACGCCGCCAGGCCGGAGGTGCAGCTGTTG GAGTCCGGGGGAGGCTTGGTACAGCCTGGGGGGTCCCT GAGACTCTCCTGTGCAGCCTCTGGATTCACCTTTAGCAA TTATGCTATGAGCTGGGTCCGCCAGGCTCCAGGGAAGGG GCTGGAGTGGGTCTCAGGGATCTATCCCGATAGTGGTAG TACATATTACGCTGATTCTGTAAAAGGTCGGTTCACCAT CTCCAGAGACAATTCCAAGAACACGCTGTATCTGCAAAT GAACAGCCTGAGAGCCGAGGACACGGCCGTGTATTACT GTGCGAGAAATATTTATACGTTCGACTACTGGGGCCAGG |
| | | GTACACTGGTCACCGTGAGCTCAGGTGGAGGCGGTTCAG GCGGAGGTGGATCCGGCGGTGGCGGATCGCAGTCTGTG CTGACTCAGCCACCCTCAGCGTCTGGGACCCCCGGGCAG AGGGTCACCATCTCTTGTAGTGGCTCTTCATCTAATATTG GCAGTAATGCTGTCTCCTGGTACCAGCAGCTCCCAGGAA CGGCCCCCAAACTCCTCATCTATTATAATAATCAGCGGC CAAGCGGGGTCCCTGACCGATTCTCTGGCTCCAAGTCTG GCACCTCAGCCTCCCTGGCCATCAGTGGGCTCCGGTCCG AGGATGAGGCTGATTATTACTGTGGTTCTTGGGATTCTA GCCTGAGTGGATATGTCTTCGGCGGAGGCACCAAGCTGA CGGTCCTAGGCGCGAAGCCCACCACGACGCCAGCGCCG CGACCACCAACACCGGCGCCCACCATCGCGTCGCAGCCC CTGTCCCTGCGCCCAGAGGCGTGCCGGCCAGCGGCGG GGGCGCAGTGCACACGAGGGGGCTGGACTTCGCCTGTG ATATCTACATCTGGGCGCCCTTGGCCGGGACTTGTGGG TCCTTCTCCTGTCACTGGTTATCACCCTTTACTGCAGAGT GAAGTTCAGCAGGAGCGCAGACGCCCCCGCGTACCAGC AGGGCCAGAACCAGCTCTATAACGAGCTCAATCTAGGA CGAAGAGAGGAGTACGATGTTTTGGACAAGAGACGTGG CCGGGACCCTGAGATGGGGGGAAAGCCGAGAAGGAAG AACCCTCAGGAAGGCCTGTACAATGAACTGCAGAAAGA TAAGATGGCGGAGGCCTACAGTGAGATTGGGATGAAAG GCGAGCGCCGGAGGGGCAAGGGGCACGATGGCCTTTAC CAGGGTCTCAGTACAGCCACCAAGGACACCTACGACGC CCTTCACATGCAGGCCCTGCCCCCTCGCTGA |
| 32 | 501(8H)z amino acid | MALPVTALLLPLALLLHAARPEVQLLESGGGLVQPGGSLR LSCAASGFTFSNYAMSWVRQAPGKGLEWVSGIYPDSGSTY YADSVKGRFTISRDNSKNTLYLQMNSLRAEDTAVYYCARN IYTFDYWGQGTLVTVSSGGGGSGGGGSGGGGSQSVLTQPP SASGTPGQRVTISCSGSSSNIGSNAVSWYQQLPGTAPKLLIY YNNQRPSGVPDRFSGSKSGTSASLAISGLRSEDEADYYCGS WDSSLSGYVFGGGTKLTVLGAKPTTTPAPRPPTPAPTIASQ PLSLRPEACRPAAGGAVHTRGLDFACDIYIWAPLAGTCGV LLLSLVITLYCRVKFSRSADAPAYQQGQNQLYNELNLGRR EEYDVLDKRRGRDPEMGGKPRRKNPQEGLYNELQKDKM AEAYSEIGMKGERRRGKGHDGLYQGLSTATKDTYDALHM QALPPR |
| 33 | 501(8H)BBz nucleotide | ATGGCCTTACCAGTGACCGCCTTGCTCCTGCCGCTGGCC TTGCTGCTCCACGCCGCCAGGCCGGAGGTGCAGCTGTTG GAGTCCGGGGGAGGCTTGGTACAGCCTGGGGGGTCCCT GAGACTCTCCTGTGCAGCCTCTGGATTCACCTTTAGCAA TTATGCTATGAGCTGGGTCCGCCAGGCTCCAGGGAAGGG GCTGGAGTGGGTCTCAGGGATCTATCCCGATAGTGGTAG TACATATTACGCTGATTCTGTAAAAGGTCGGTTCACCAT CTCCAGAGACAATTCCAAGAACACGCTGTATCTGCAAAT GAACAGCCTGAGAGCCGAGGACACGGCCGTGTATTACT GTGCGAGAAATATTTATACGTTCGACTACTGGGGCCAGG GTACACTGGTCACCGTGAGCTCAGGTGGAGGCGGTTCAG GCGGAGGTGGATCCGGCGGTGGCGGATCGCAGTCTGTG CTGACTCAGCCACCCTCAGCGTCTGGGACCCCCGGGCAG |
| | | AGGGTCACCATCTCTTGTAGTGGCTCTTCATCTAATATTG GCAGTAATGCTGTCTCCTGGTACCAGCAGCTCCCAGGAA CGGCCCCCAAACTCCTCATCTATTATAATAATCAGCGGC CAAGCGGGGTCCCTGACCGATTCTCTGGCTCCAAGTCTG GCACCTCAGCCTCCCTGGCCATCAGTGGGCTCCGGTCCG AGGATGAGGCTGATTATTACTGTGGTTCTTGGGATTCTA GCCTGAGTGGATATGTCTTCGGCGGAGGCACCAAGCTGA CGGTCCTAGGCGCGAAGCCCACCACGACGCCAGCGCCG CGACCACCAACACCGGCGCCCACCATCGCGTCGCAGCCC CTGTCCCTGCGCCCAGAGGCGTGCCGGCCAGCGGCGGG GGGCGCAGTGCACACGAGGGGGCTGGACTTCGCCTGTG ATATCTACATCTGGGCGCCCTTGGCCGGGACTTGTGGG TCCTTCTCCTGTCACTGGTTATCACCCTTTACTGCAAACG GGGCAGAAAGAAACTCCTGTATATATTCAAACAACCATT TATGAGACCAGTACAAACTACTCAAGAGGAAGATGGCT GTAGCTGCCGATTTCCAGAAGAAGAAGAAGGAGGATGT GAACTGAGAGTGAAGTTCAGCAGGAGCGCAGACGCCCC CGCGTACCAGCAGGGCCAGAACCAGCTCTATAACGAGC TCAATCTAGGACGAAGAGAGGAGTACGATGTTTTGGAC AAGAGACGTGGCCGGGACCCTGAGATGGGGGGAAAGCC GAGAAGGAAGAACCCTCAGGAAGGCCTGTACAATGAAC TGCAGAAAGATAAGATGGCGGAGGCCTACAGTGAGATT GGGATGAAAGGCGAGCGCCGGAGGGGCAAGGGGCACG ATGGCCTTTACCAGGGTCTCAGTACAGCCACCAAGGACA CCTACGACGCCCTTCACATGCAGGCCCTGCCCCCTCGCT |
| | | GA |
| 34 | 501(8H)BBz amino acid | MALPVTALLLPLALLLHAARPEVQLLESGGGLVQPGGSLR LSCAASGFTFSNYAMSWVRQAPGKGLEWVSGIYPDSGSTY YADSVKGRFTISRDNSKNTLYLQMNSLRAEDTAVYYCARN IYTFDYWGQGTLVTVSSGGGGSGGGGSGGGGSQSVLTQPP SASGTPGQRVTISCSGSSSNIGSNAVSWYQQLPGTAPKLLIY YNNQRPSGVPDRFSGSKSGTSASLAISGLRSEDEADYYCGS WDSSLSGYVFGGGTKLTVLGAKPTTTPAPRPPTPAPTIASQ PLSLRPEACRPAAGGAVHTRGLDFACDIYIWAPLAGTCGV LLLSLVITLYCKRGRKKLLYIFKQPFMRPVQTTQEEDGCSC RFPEEEEGGCELRVKFSRSADAPAYQQGQNQLYNELNLGR REEYDVLDKRRGRDPEMGGKPRRKNPQEGLYNELQKDKM AEAYSEIGMKGERRRGKGHDGLYQGLSTATKDTYDALHM QALPPR |
| 35 | 501(8H)28z nucleotide | ATGGCCTTACCAGTGACCGCCTTGCTCCTGCCGCTGGCC TTGCTGCTCCACGCCGCCAGGCCGGAGGTGCAGCTGTTG GAGTCCGGGGGAGGCTTGGTACAGCCTGGGGGGTCCCT GAGACTCTCCTGTGCAGCCTCTGGATTCACCTTTAGCAA TTATGCTATGAGCTGGGTCCGCCAGGCTCCAGGGAAGGG GCTGGAGTGGGTCTCAGGGATCTATCCCGATAGTGGTAG TACATATTACGCTGATTCTGTAAAAGGTCGGTTCACCAT CTCCAGAGACAATTCCAAGAACACGCTGTATCTGCAAAT GAACAGCCTGAGAGCCGAGGACACGGCCGTGTATTACT GTGCGAGAAATATTTATACGTTCGACTACTGGGGCCAGG GTACACTGGTCACCGTGAGCTCAGGTGGAGGCGGTTCAG |
| | | GCGGAGGTGGATCCGGCGGTGGCGGATCGCAGTCTGTG CTGACTCAGCCACCCTCAGCGTCTGGGACCCCCGGGCAG AGGGTCACCATCTCTTGTAGTGGCTCTTCATCTAATATTG GCAGTAATGCTGTCTCCTGGTACCAGCAGCTCCCAGGAA CGGCCCCCAAACTCCTCATCTATTATAATAATCAGCGGC CAAGCGGGGTCCCTGACCGATTCTCTGGCTCCAAGTCTG GCACCTCAGCCTCCCTGGCCATCAGTGGGCTCCGGTCCG AGGATGAGGCTGATTATTACTGTGGTTCTTGGGATTCTA GCCTGAGTGGATATGTCTTCGGCGGAGGCACCAAGCTGA CGGTCCTAGGCGCGAAGCCCACCACGACGCCAGCGCCG CGACCACCAACACCGGCGCCCACCATCGCGTCGCAGCCC CTGTCCCTGCGCCCAGAGGCGTGCCGGCCAGCGGCGGG GGGCGCAGTGCACACGAGGGGGCTGGACTTCGCCTGTG ATTTTTGGGTGCTGGTGGTGGTTGGTGGAGTCCTGGCTT GCTATAGCTTGCTAGTAACAGTGGCCTTTATTATTTTCTG GGTGAGGAGTAAGAGGAGCAGGCTCCTGCACAGTGACT ACATGAACATGACTCCCCGCCGCCCCGGGCCCACCCGCA AGCATTACCAGCCCTATGCCCCACCACGCGACTTCGCAG CCTATCGCTCCAGAGTGAAGTTCAGCAGGAGCGCAGAC GCCCCCGCGTACCAGCAGGGCCAGAACCAGCTCTATAA CGAGCTCAATCTAGGACGAAGAGAGGAGTACGATGTTT TGGACAAGAGACGTGGCCGGGACCCTGAGATGGGGGGA AAGCCGAGAAGGAAGAACCCTCAGGAAGGCCTGTACAA TGAACTGCAGAAAGATAAGATGGCGGAGGCCTACAGTG AGATTGGGATGAAAGGCGAGCGCCGGAGGGGCAAGGG |
| | | GCACGATGGCCTTTACCAGGGTCTCAGTACAGCCACCAA GGACACCTACGACGCCCTTCACATGCAGGCCCTGCCCCC TCGCTGA |
| 36 | 501(8H)28z amino acid | MALPVTALLLPLALLLHAARPEVQLLESGGGLVQPGGSLR LSCAASGFTFSNYAMSWVRQAPGKGLEWVSGIYPDSGSTY YADSVKGRFTISRDNSKNTLYLQMNSLRAEDTAVYYCARN IYTFDYWGQGTLVTVSSGGGGSGGGGSGGGGSQSVLTQPP SASGTPGQRVTISCSGSSSNIGSNAVSWYQQLPGTAPKLLIY YNNQRPSGVPDRFSGSKSGTSASLAISGLRSEDEADYYCGS WDSSLSGYVFGGGTKLTVLGAKPTTTPAPRPPTPAPTIASQ PLSLRPEACRPAAGGAVHTRGLDFACDFWVLVVVGGVLA CYSLLVTVAFIIFWVRSKRSRLLHSDYMNMTPRRPGPTRKH YQPYAPPRDFAAYRSRVKFSRSADAPAYQQGQNQLYNEL NLGRREEYDVLDKRRGRDPEMGGKPRRKNPQEGLYNELQ KDKMAEAYSEIGMKGERRRGKGHDGLYQGLSTATKDTYD ALHMQALPPR |
| 37 | Linker nucleotide | GGTGGAGGCGGTTCAGGCGGAGGTGGATCCGGCGGTGG CGGATCG |
| 38 | Linker amino acid | GGGGSGGGGSGGGGS |

The sequence list of the heavy chains, light chains, and CDRs of the scFv variable regions used in the production of the chimeric antigen receptor (CAR) according to an embodiment of the present invention are summarized in Tables 4 to 5.

**[Table 4]**

| SEQ ID NO | Clone | Variable region | Amino acid sequence |
|---|---|---|---|
| 39 | MS501 | Heavy chain | EVQLLESGGGLVQPGGSLRLSCAASGFTFSNYAMSWVRQAPG KGLEWVSGIYPDSGSTYYADSVKGRFTISRDNSKNTLYLQMN SLRAEDTAVYYCARNIYTFDYWGQGTLVTVSS |
| 40 | | Light chain | QSVLTQPPSASGTPGQRVTISCSGSSSNIGSNAVSWYQQLPGT APKLLIYYNNQRPSGVPDRFSGSKSGTSASLAISGLRSEDEAD YYCGSWDSSLSGYVFGGGTKLTVLG |
| 41 | MS503 | Heavy chain | EVQLLESGGGLVQPGGSLRLSCAASGFTFSNYAMSWVRQAPG KGLEWVSSIYPGDGSTYYADSVKGRFTISRDNSKNTLYLQMN SLRAEDTAVYYCAKNAFTFDYWGQGTLVTVSS |
| 42 | | Light chain | QSVLTQPPSASGTPGQRVTISCSGSSSNIGSNAVSWYQQLPGT APKLLIYYNSHRPSGVPDRFSGSKSGTSASLAISGLRSEDEADY YCGTWDSSLSGYVFGGGTKLTVLG |
| 43 | C2G4 | Heavy chain | EVQLLESGGGLVQPGGSLRLSCAASGFTFSNYAMSWVRQAPG KGLEWVSGIPPDSGSKYYADSVRGRFTVSRDNSKNTLYLQMN SLRAEDTAVYYCAKNMLSFDYWGQGTLVTVSS |
| 44 | | Light chain | QSVLTQPPSASGTPGQRVTISCTGSSSNIGSNAVSWYQQLPGT APKLLIYYNSKRPSGVPDRFSGSKSGTSASLAISGLRSEDEADY YCGSWDPSLNGYVFGGGTKVTVLG |

**[Table 5]**

| Clone | Variable region | SEQ ID NO | CDR1 | SEQ ID NO | CDR2 | SEQ ID NO | CDR3 |
|---|---|---|---|---|---|---|---|
| MS501 | Heavy chain | 45 | NYAMS | 46 | GIYPDSGSTYYAD SVKG | 47 | NIYTFDY |
| | Light chain | 48 | SGSSSNIGSN AVS | 49 | YNNQRPS | 50 | GSWDSSLSG YV |
| MS503 | Heavy chain | 51 | NYAMS | 52 | SIYPGDGSTYYAD SVKG | 53 | NAFTFDY |
| | Light chain | 54 | SGSSSNIGSN AVS | 55 | YNSHRPS | 56 | GTWDSSLSG YV |
| C2G4 | Heavy chain | 57 | NYAMS | 58 | GIPPDSGSKYYADS VRG | 59 | NMLSFDY |
| | Light chain | 60 | TGSSSNIGSN AVS | 61 | YNSKRPS | 62 | GSWDPSLNG YV |

### 1.6 Virus production and gene transfer

For gene delivery, pseudotype-VSVG lentivirus was produced using 293T cells. HEK293T cells were cultured in DMEM (GIBCO) medium containing 10% FBS (GIBCO). HEK293T cells were co-transfected with pCDH1-MSCV-MSLN CAR construct EF1α-copGFP vector, EF1α-MSLN CAR construct vector, or pCDH1-MSCV-EF1α―copGFP control vector along with HIV-based pPACKH1 Lentiviral Package Kit (System Biosciences). Lipofectamine 2000 (Invitrogen, Carlsbad, CA) was used for vector delivery. The MSLN CAR construct is as follows: 501(28H)28z, 503(28H)28z, C2G4(28H)28z, 501(8H)Δ, 501(8H)z, 501(8H)BBz or 501(8H)28z. 48 hours after transformation, the cell supernatant containing lentivirus was harvested. The cell debris was removed from the supernatant through a 0.45 µm filter unit (Millipore, Billerica, MA, USA). The virus was concentrated 1,500 times by ultracentrifugation at 10,600 rpm for 90 minutes. The concentrated virus was stored at - 80°C.

The concentration of Jurkat cells was adjusted to 5 x 10⁵ cells/ml using RPMI-1640 medium (GIBCO) containing 10% FBS, and 5 MOI (multiplicity of infection) or 10 MOI of lentivirus was added. The cells were centrifuged at 1,800 g and 32°C for 90 minutes. After centrifugation, the supernatant was removed and the medium was replaced with fresh medium. The cells were stored in a humidified incubator at 37°C and 5% CO₂. Control cells were transduced with the vector alone.

Human T cells were suspended at a concentration of 5 x 10⁵ cells/ml in X-VIVO15 (LONZA) medium containing 5% human serum (Sigma) and 50 IU/ml of IL-2 (Novartis). 5 or 10 MOI of lentivirus was added along with 8 µg/ml polybrene (Santa Cruz). The resulting mixture was centrifuged at 1,800 g and 32°C for 90 minutes. After centrifugation, the supernatant was removed and the medium was replaced with fresh medium. The cells were then allowed to stand in a humidified incubator at 37°C and 5% CO₂. Control cells were transduced with the vector alone.

### 1.7 Expression of receptor containing MSLN CAR and T cell activity marker analysis

501(28H)28z-transduced Jurkat cells, 503(28H)28z-transduced Jurkat cells, C2G4(28H)28z-transduced Jurkat cells, control-vector-transduced Jurkat cells and Jurkat cells were washed twice with FACS buffer. The cells were stained with anti-CD3 (BD Biosciences), anti-F(ab)₂ (Jackson Immuno research), and anti-CD69(BD)hAbs. The expression rate (%) and mean fluorescence intensity (MFI) of the stained cells were measured using a BD LSRFortessa and analyzed using FlowJo software.

501(28H)28z-transduced human T cells, C2G4(28H)28z-transduced human T cells, and control-vector-transduced human T cells were washed twice using FACS buffer. The cells were stained with anti-CD3 (BD Biosciences) and anti-F(ab)₂ (Jackson Immuno Research). The expression rate (%) and mean fluorescence intensity (MFI) of the stained cells were measured using a BD LSRFortessa and analyzed using FlowJo software.

### 1.8 Confirmation of ability of T cells expressing MSLN CAR to activate target cells

The ability against cancer cells that expressing mesothelin, the target cell of MSLN CAR-expressing T cells, was identified through the following process.
(i) Target cells were seeded at 1 x 10⁴ cells/well on a 96-well plate.
(ii) Effector cells (501(28H)28z-transduced Jurkat cells, 503(28H)28z-transduced Jurkat cells, C2G4(28H)28z-transduced Jurkat cells, control-vector-transduced Jurkat cells, and Jurkat cells) were harvested, washed with RPMI-1640 medium, and added under various E/T (effector-to-target) ratio conditions.
(iii) After 24 hours, the plates were centrifuged at 2,000 rpm for 3 minutes, 100 µL of supernatant was collected, the centrifuged cells were washed twice using FACS buffer, and then the activation markers were analyzed. After completion of cytotoxicity assay, the samples were stored at -20°C.

### 1.9 Calcein release cytotoxicity assay

Through the following process, the cytotoxicity of MSLN CAR-expressing T cells was detected.
(i) Target cells were labeled with 10 µM calceinacetoxymethyl ester (calcein-AM; cell-permeant dye, Invitrogen) at 37°C for 30 minutes.
(ii) After washing, the labeled target cells were seeded at 1 x 10⁴ cells/well on a 96-well plate.
(iii) Effector cells (501(28H)28z-transduced human T cells, C2G4(28H)28z-transduced human T cells and control-vector-transduced human T cells) were harvested, washed with an RPMI-1640 medium, and added under various E/T (effector-to-target) ratio conditions.
(iv) After 4 hours, the plates were centrifuged at 2,000 rpm for 3 minutes, 100 µL of the supernatant was collected, and calcein release was measured at a stimulating wavelength of 485 nm and a luminescent wavelength of 535 nm with a fluorescent microplate reader (Victor3, PerkinElmer). After completion of cytotoxicity assay, the samples were stored at -20°C.

### 1.10 Enzyme-linked immunosorbent assay for cytokine measurement

The cytokine in the supernatant stored at -20°C was measured using an enzyme-linked immunosorbent assay (ELISA, R&D systems).
(i) The capture antibody was diluted in accordance with the Certificate of Analysis (CoA) and coated on a 96-well plate for 24 hours.
(ii) Each well was washed with a washing solution and blocked with a 1% BSA (Bovine serum albumin, Sigma) solution for 1 hour.
(iii) Each well was washed with a washing solution and treated with the sample for 2 hours.
(iv) Each well was washed with the washing solution, and a detection antibody was diluted in accordance with the Certificate of Analysis (CoA), seeded into the well, followed by treatment for 2 hours.
(v) The well was washed with a washing solution and treated with streptavidin-HRP for 20 minutes.
(vi) The well was washed with the washing solution and treated with a substrate solution for 20 minutes.
(vii) The well was treated with a stop solution and absorbance at 450 nm was measured.

### 1.11 Establishment of xenograft mouse model and evaluation of antitumor activity of MSLN CAR-expressing T cells

An *in-vivo* experiment was conducted with the approval of the Institutional Animal Care and Use Committee (IACUC, GC Green Cross, Korea). 6-week-old female NSG (NOD scid gamma) mice were purchased from the Jackson laboratory (Bar Harbor, Maine, United States) and used. The NCI-H226 cells were suspended at a concentration of 5 x 10⁶ cells/100 µl in PBS (phosphate-buffered saline, GIBCO). A Matrigel basement membrane matrix (Corning, NY, United States) was added in the same volume as the suspended cells. 5 x 10⁶NCI-H226 cells were injected subcutaneously into the right flank of each mouse.

The prepared human T cells transduced with 501(8H)Δ, 501(8H)z, 501(8H)BBz, 501(8H)28z or 501(28H)28z were injected into the tumor twice at 5-day intervals when the tumor size reached 200-250 mm³. 10 mice were used for each group. CAR-T cells were injected, and then tumor size and weight were measured every 2 to 3 days. Tumor tissue was harvested from four animals in each group through a treatment method in accordance with IACUC regulations on the 20^{th} day after the first intratumoral injection. The harvested tumor tissue was weighed using an electronic scale. The tumor tissue was cut finely and washed with PBS, and the supernatant was used for IFN-γ cytokine ELISA. The mice were observed for 60 days after injection of CAR-T cells into the first tumor, and were considered dead when the length of the long axis of the tumor was 15 mm or more, or the body weight decreased by 20% or more.

### Example 2: Evaluation of Jurkat cells and human T cells expressing mesothelin-specific CAR as cancer immunotherapy

### 2.1 Evaluation of mesothelin-specific scFvs

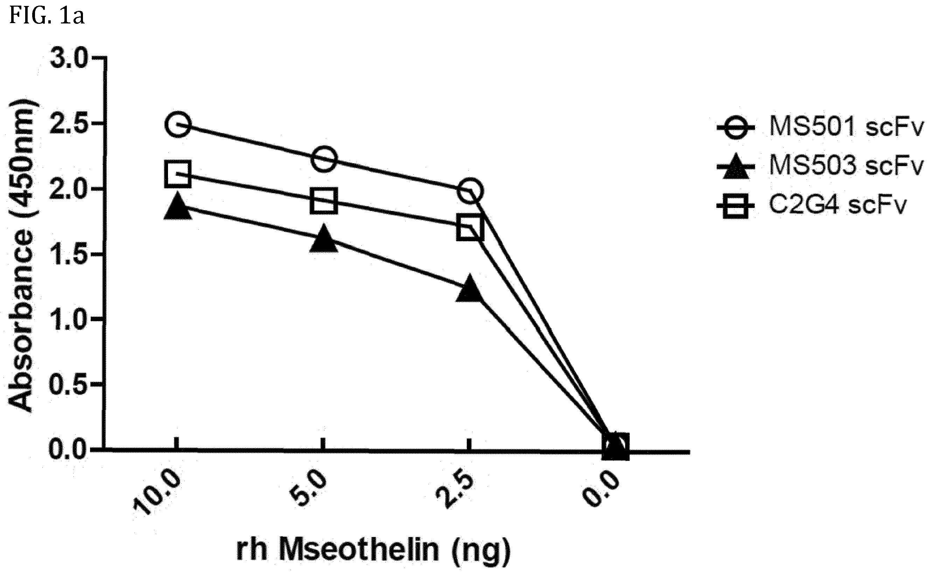

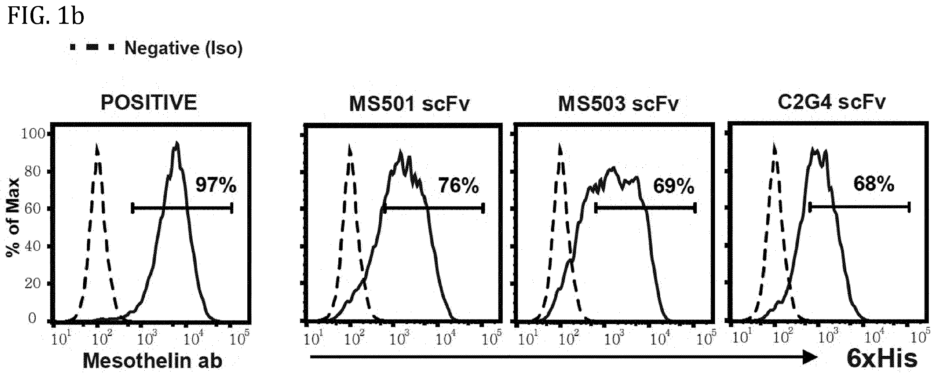

MS501 IgG, MS503 IgG, and C2G4 IgG were conceived in previous patents (Mokam Research Center, GC Green Cross, KOREA) and are known to be capable of specifically binding to mesothelin. The present inventors produced MS501 IgG, MS503 IgG, and C2G4 IgG in the form of scFv and identified through immunoprecipitation analysis that these scFv forms also bind to recombinant human mesothelin (FIG. 1a). The above scFv proteins were bound to mesothelin overexpressed cancer cell lines, and it was confirmed that the scFv proteins were more than 68% bound to mesothelin overexpressed cancer cell lines through flow cytometry (FIG. 1b).

### 2.2 Identification of mesothelin-specific CAR expression and evaluation of expression maintenance

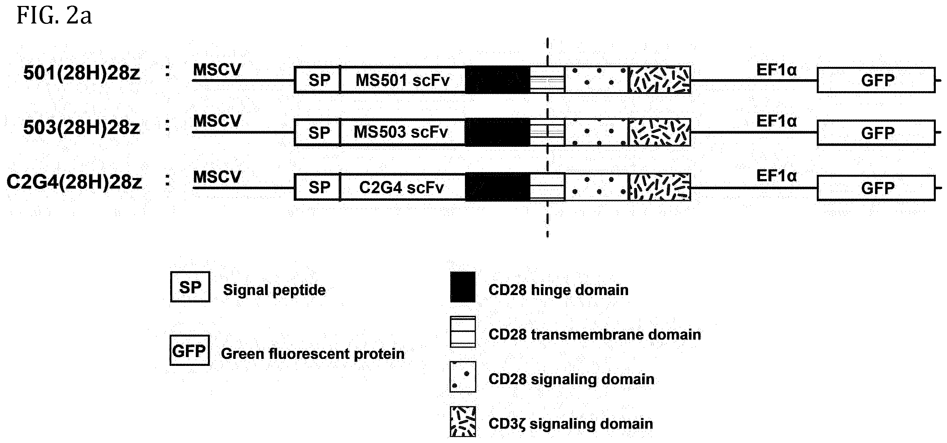

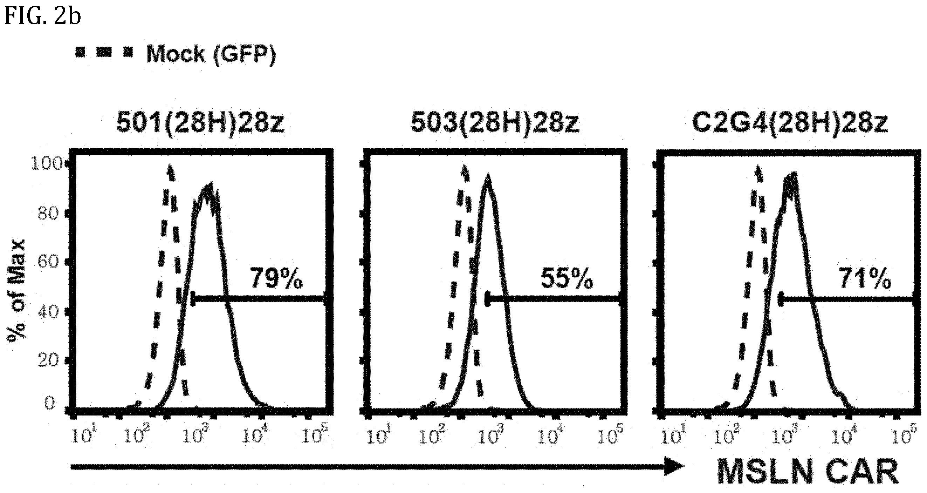

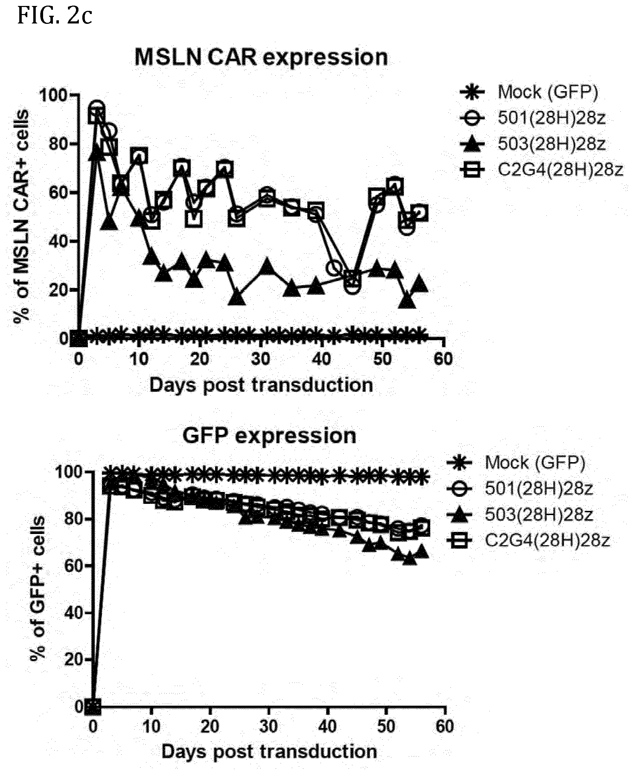

MS501 scFv, MS503 scFv and C2G4 scFv were linked to a CD8α-derived scFv signal sequence, CD28-derived hinge, transmembrane domain and intracellular signaling domain, and a CD3ζ-derived intracellular signaling domain [Table 1] (FIG. 2a). The recombined chimeric antigen receptor (501(28H)28z, 503(28H)28z, C2G4(28H)28z) genes were expressed in Jurkat cells using a lentiviral vector having an MSCV promoter. The amount of lentivirus used herein was 10 MOI (multiplicity of infection). Each CAR was detected using an anti-F(ab)₂ antibody. Flow cytometry result showed CAR transduction in Jurkat cells at an efficiency of 55% or more (FIG. 2b) and flow cytometry was conducted every 2-3 days to detect CAR expression. CAR expression was maintained for 50 days or longer, demonstrating that the expression of the transduced CAR genes can be maintained even upon extracellular proliferation (FIG. 2C).

### 2.3 Increased activity of Jurkat cells expressing mesothelin-specific CAR

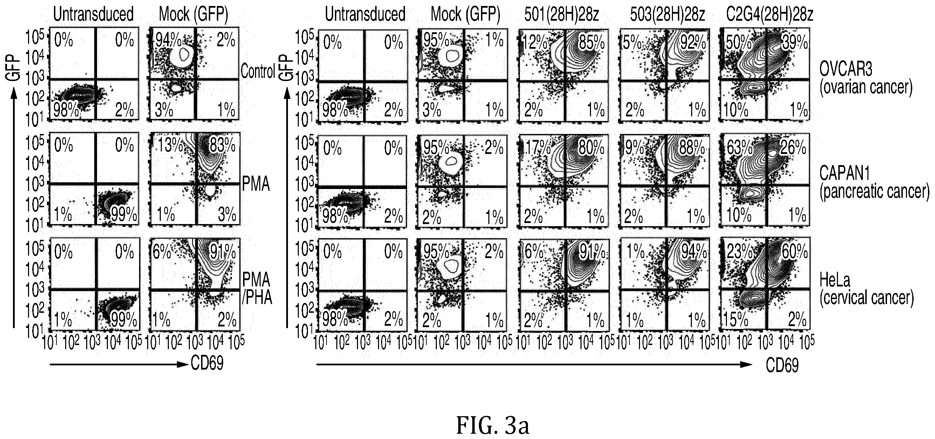

Because mesothelin is known to be overexpressed in cancer such as mesothelioma, uterine cancer and pancreatic cancer and to be related to the progression of cancer, we conducted a test to determine whether or not Jurkat cells expressing chimeric antigen receptors targeting mesothelin exhibit anticancer activity. Prior to the test, the activity of Jurkat cells was detected using PMA and PHA, which are known to activate T cells, in untransduced Jurkat cells and Jurkat cells transduced with the vector alone (mock). When treated with PMA and PHA, the activity of Jurkat cells was increased (FIG. 3A).

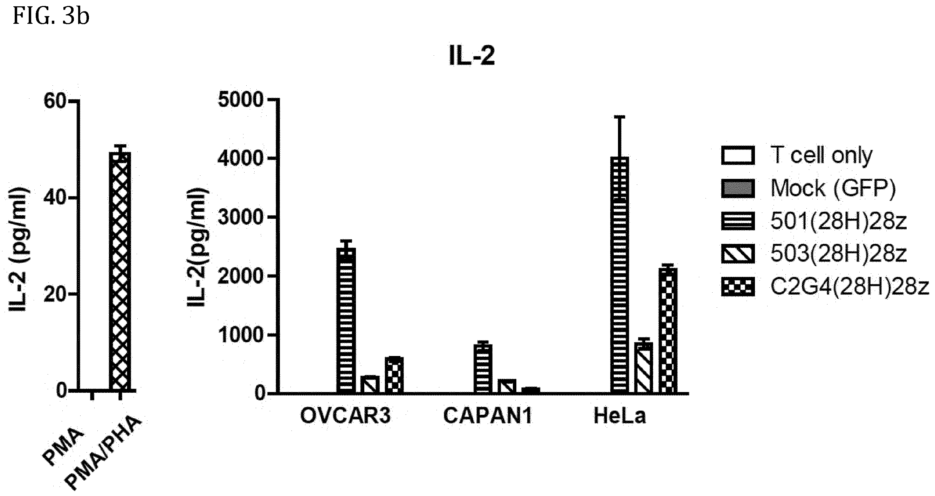

The gene encoding the mesothelin-specific chimeric antigen receptor was recombined, and the gene was transferred to Jurkat cells using a lentivirus. The Jurkat cells transduced with 501(28H)28z or C2G4(28H)28z mesothelin receptor exhibited high expression of chimeric antigen receptors compared to Jurkat cells transduced with 503(28H)28z (FIG. 2c). HeLa, OVCAR3 and CAPAN1 cells known to have high mesothelin expression and Jurkat cells transduced with the chimeric antigen receptor were co-cultured to evaluate the activity of Jurkat cells transduced with the mesothelin receptor. It was identified that CD69, which is a T cell activity marker, was expressed in Jurkat cells transduced with the mesothelin receptor, and in particular, CD69 was expressed at a high level in Jurkat cells transduced with MS501 and MS503 mesothelin receptors (FIG. 3a). In addition, it was found that cytokines were also secreted upon target cell response. In this case, the Jurkat cells transduced with 501(28H)28z secreted the greatest amount of cytokines (FIG. 3b). This demonstrated that, among the Jurkat cells expressing mesothelin-specific chimeric antigen receptors of the present invention, Jurkat cells transduced with a MS501 mesothelin receptor (501(28H)28z) exhibited higher activity than Jurkat cells transduced with MS503(28H)28z or C2G4(28H)28z.

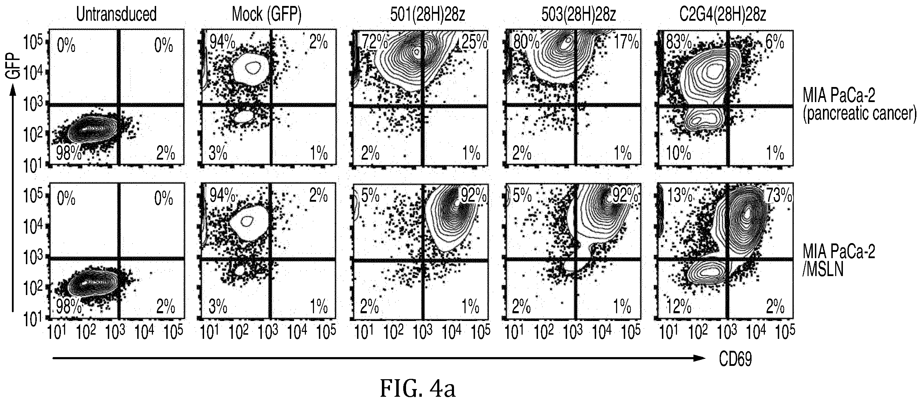

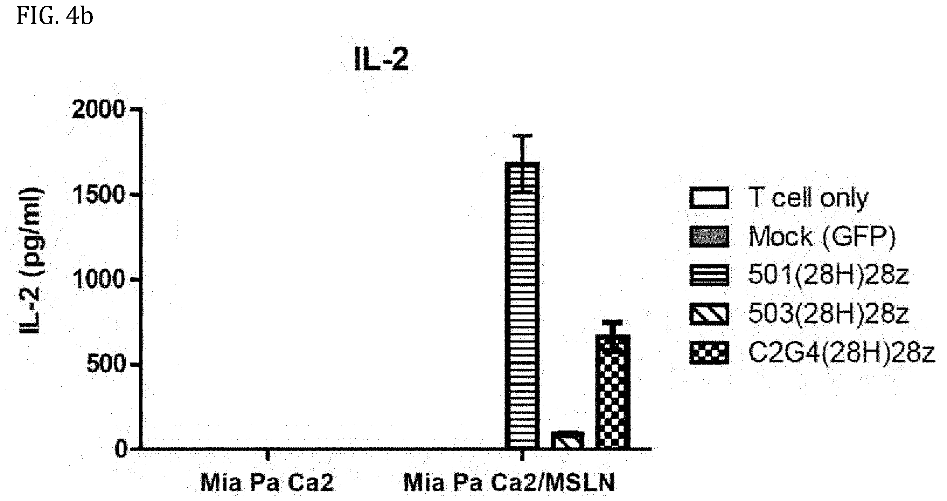

In order to ascertain the mesothelin-specific activity of Jurkat cells expressing three mesothelin receptors, namely, 501(28H)28z, 503(28H)28z and C2G4(28H)28z according to the present invention, each of MIA PaCa2 cells and MIA PaCa2-MSLN cells were co-cultured with the mesothelin receptor-transduced Jurkat cells, and then the activity was evaluated. CD69 was expressed at a high level in Jurkat cells transduced with 501(28H)28z, 503(28H)28z or C2G4(28H)28z, after co-culture of MIA PaCa2-MSLN cells which are mesothelin-overexpressing cells, with mesothelin receptor-transduced Jurkat cells. Among them, in particular, the Jurkat cells transduced with 501(28H)28z or 503(28H)28z expressed CD69 at higher levels compared to Jurkat cells transduced with C2G4(28H)28z (FIG. 4a). In addition, in order to ascertain the activity of Jurkat cells through cytokine-secreting ability, the amount of IL-2 was evaluated using ELISA. It was found that IL-2 cytokines were secreted in Jurkat cells transduced with 501(28H)28z, 503(28H)28z, or C2G4(28H)28z, and were secreted in high levels particularly in Jurkat cells transduced with 501(28H)28z (FIG. 4b). This demonstrated that the Jurkat cells expressing the mesothelin receptor of the present invention exhibit activity specific to mesothelin.

### 2.4 Evaluation of mesothelin-specific CAR expression in human T cells

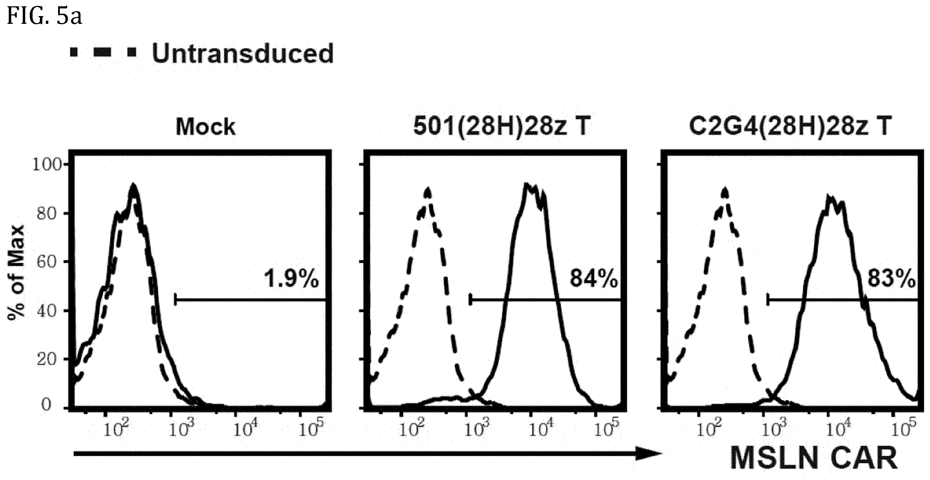

When compared with 501(28H)28z or C2G4(28H)28z-transformed Jurkat cells, the 503(28H)28z-transformed Jurkat cells exhibited low MSLN CAR expression maintenance (FIG. 2c) and insufficient cytokine secretion compared to the active marker expression when co-cultured with target cells (FIGS. 3a and 3b). We excluded 503(28H)28z from subsequent experiments. The recombinant chimeric antigen receptor (501(28H)28z or C2G4(28H)28z) gene was expressed in human T cells using a lentiviral vector having an MSCV promoter. The amount of lentivirus that was used was 5 to 10 MOI (multiplicity of infection). Each CAR was detected using an anti-F(ab)₂ antibody. Flow cytometry ascertained that the CAR was transduced with an efficiency of 80% or more into human T cells (FIG. 5a).

### 2.5 Evaluation of anticancer activity of human T cells expressing mesothelin-specific CAR

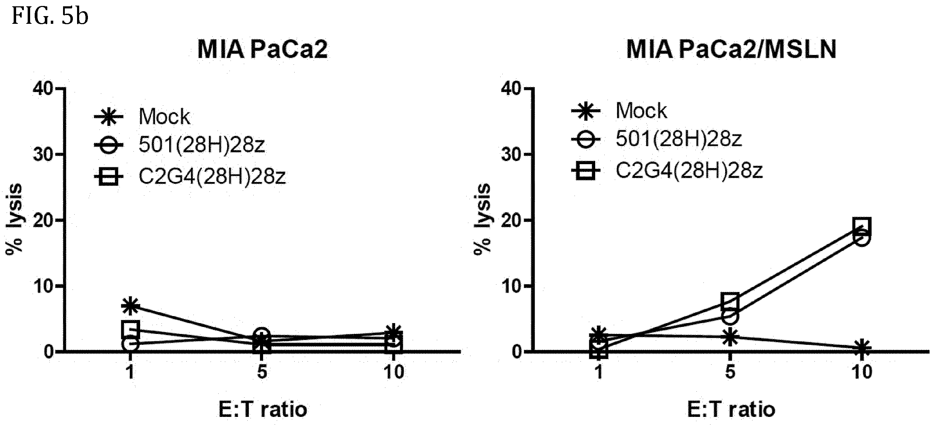

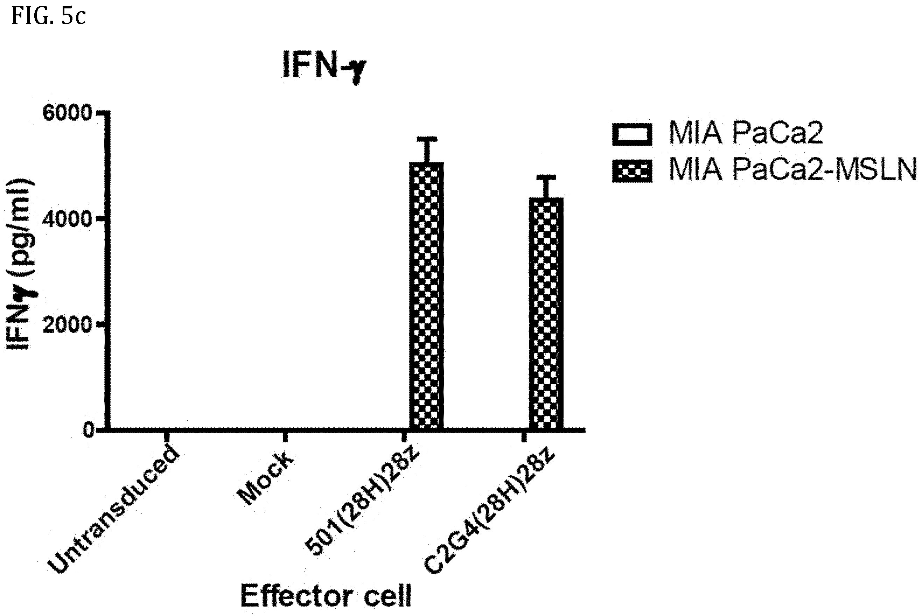

In order to evaluate the mesothelin-specific anticancer activity of human T cells expressing two mesothelin chimeric antigen receptors (501(28H)28z and C2G4(28H)28z) according to the present invention, the MIA PaCa2 cells or MIA PaCa2-MSLN cells was co-cultured with human T cells transduced with mesothelin receptors, and the ability to kill target cells was evaluated. Human T cells expressing the mesothelin receptor did not exhibit the ability to kill MIA PaCa2 cells, but MIA PaCa2-MSLN cells expressing mesothelin exhibited the killing ability (FIG. 5b). In addition, the activity of human T cells transformed with mesothelin CAR was determined through IFN-γ cytokine secretion ability (FIG. 5c). This ascertained that the human T cells expressing the mesothelin receptor of the present invention exhibited anticancer activity specifically for mesothelin.

OVCAR3 and CAPAN1 cells, which are known to have high mesothelin expression, were co-cultured with human T cells transduced with two mesothelin-specific chimeric antigen receptors (501(28H)28z and C2G4(28H)28z) according to the present invention, and the ability to kill target cells was evaluated. It was identified that human T cells expressing the mesothelin-specific chimeric antigen receptor have the ability to kill OVCAR3 and CAPAN1 cells (FIG. 5b).

### 2.6 Screening of mesothelin-specific CARs with various combinations and evaluation as anti-cancer immunotherapy in mouse models

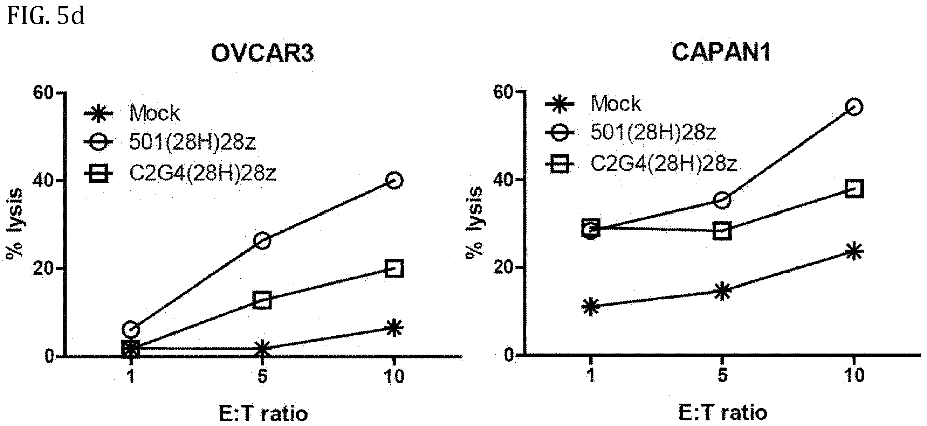

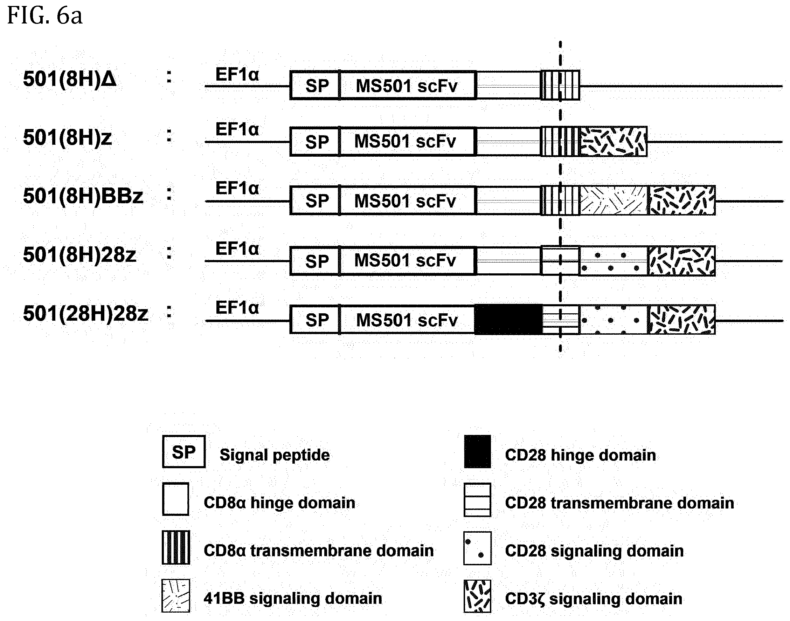

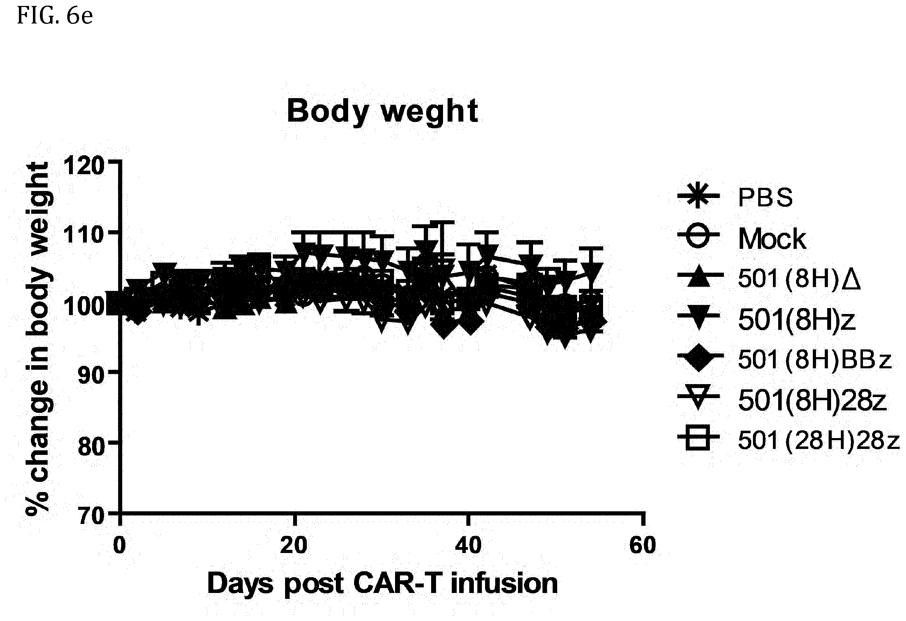

Compared to 501(28H)28z-transformed human T cells, C2G4(28H)28z-transformed human T cells exhibited insufficient ability to kill target cells expressing mesothelin (FIG. 5d). Thus, we excluded C2G4(28H)28z from subsequent experiments. For screening of mesothelin-specific CAR candidates with various combinations, a chimeric antigen receptor 501(8H)Δ, 501(8H)z, 501(8H)BBz, 501(8H)28z or 501(28H)28z gene was further reconstructed based on MS501 scFv [Table 1] (FIG. 6a) . The reconstructed CAR gene was expressed in human T cells using a lentiviral vector having an EF1α promoter. The amount of lentivirus that was used was 5 to 10 MOI (multiplicity of infection). Each CAR was detected using an anti-F(ab)₂ antibody. The number of CAR positive cells was calculated by detecting the CAR positive rate through flow cytometry.

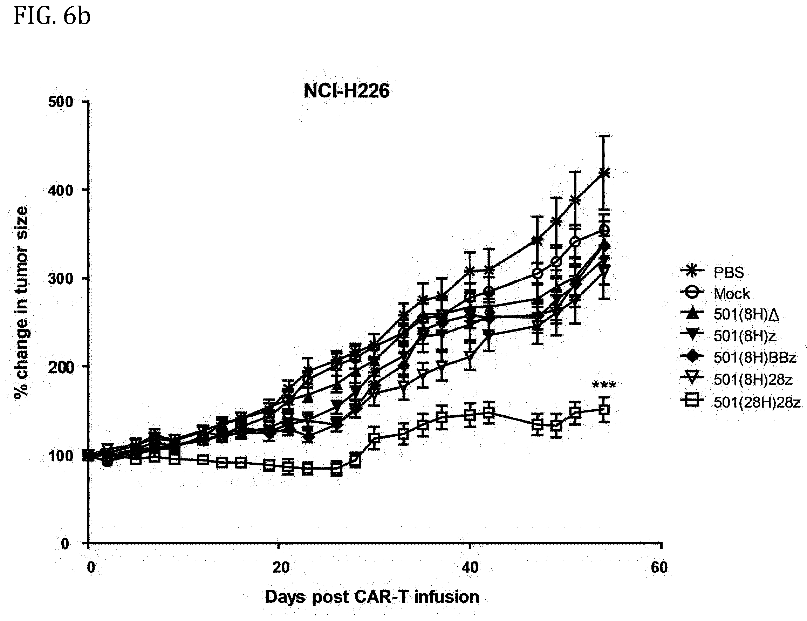

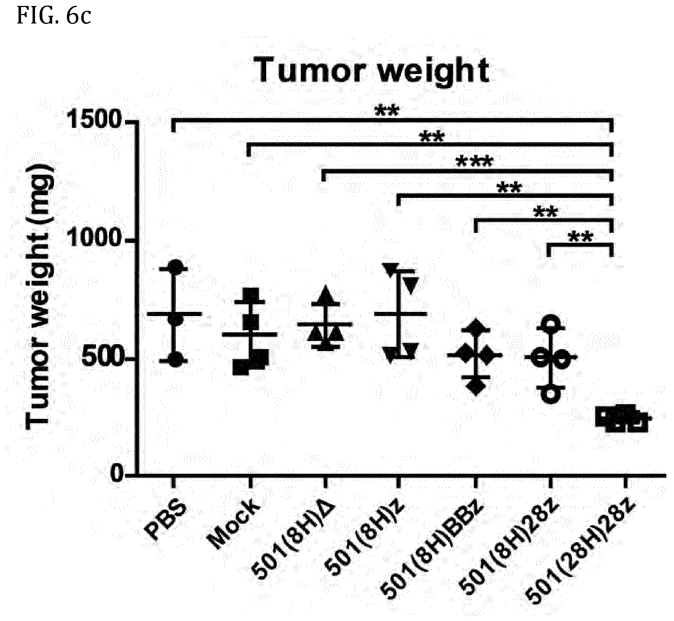

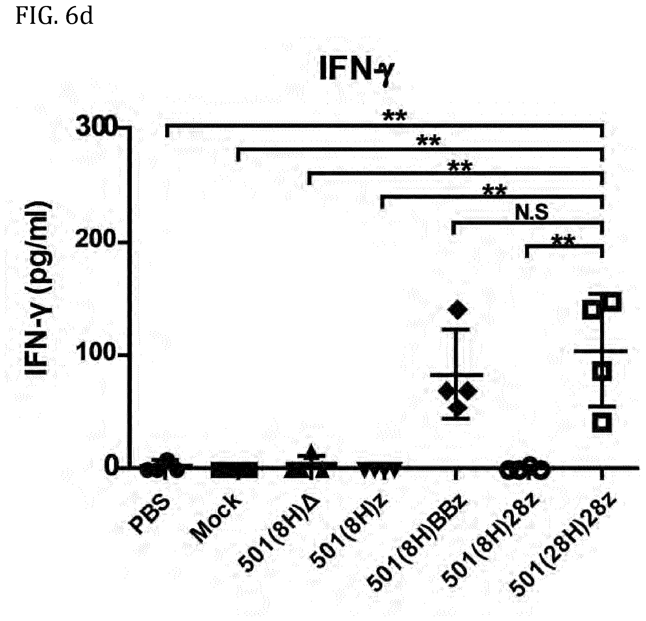

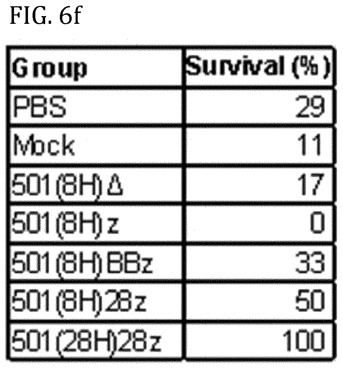

When the tumor size of NCI-H226 cell-xenografted mice reached 200 to 250 mm³, human T cells transduced with mesothelin-specific CARs having various combinations were injected into the tumors, and the anticancer activity thereof was evaluated in mouse models for two months. The increase or decrease in size compared to the tumor size before intratumoral injection were expressed as a percentage (FIG. 6b). When the human T cells transformed with the mesothelin-specific chimeric antigen receptor of the 501(28H)28z structure, among human T cells transformed with the mesothelin-specific chimeric antigen receptor having various combinations, were injected into the tumor, the tumor size was found to decrease by 270% at the end point, compared to the PBS control group. The 501(28H)28z group exhibited a 150% decrease in tumor size compared to the 501(8H)28z group. On the 20^{th} day after the first intratumoral injection of CAR-T cells, tumor tissues were isolated from 4 mice for each group, and the relative tumor tissue weights were compared between respective groups (FIG. 6c). The 501(28H)28z group exhibited lower tumor weight and better anticancer activity compared to other groups. In addition, an anticancer effect derived from the activity of 501(28H)28z-transformed human T cells was determined based on the ability to secrete IFN-γ cytokines in the tumor (FIG. 6d). Mesothelin-specific human CAR-T cells were injected into the tumors, and a side effect thereof was expressed as a body weight change (FIG. 6d). It was identified that there was no side effect after CAR-T cell injection. The survival (%) of the mouse model at the end point was determined (FIG. 6f). The PBS group exhibited 29% survival, the mock group exhibited 11% survival, 501(8H)Δ group exhibited 17% survival, the 501(8H)z group exhibited 0% survival, the 501(8H)BBz group exhibited 33% survival, and the 501(8H)28z group exhibited 50% survival, whereas the 501(28H)28z group exhibited 100% survival.

The above experimental results show that the present invention is useful as a mesothelin-specific cancer immunocytotherapy agent, and that, among the mesothelin chimeric antigen receptors of the present invention, T cells using the MS501 mesothelin receptor can exhibit superior anticancer effects, and in particular, T cells using 501(28H)28z can exhibit excellent anticancer effects.

### Industrial Applicability

The chimeric antigen receptor according to the present invention has advantages of exhibiting excellent mesothelin-specific targeting efficiency as well as excellent expression persistence, and the T cells expressing the chimeric antigen receptor according to the present invention have excellent cytotoxicity to cancer cells and thus are useful for anti-cancer immunotherapy.

Although specific configurations of the present invention have been described in detail, those skilled in the art will appreciate that this description is provided to set forth preferred embodiments for illustrative purposes and should not be construed as limiting the scope of the present invention. Therefore, the substantial scope of the present invention is defined by the accompanying claims and equivalents thereto.

### Sequence Listing Free Text

An electronic file is attached.

## Claims

1. A chimeric antigen receptor (CAR) comprising a binding domain specifically binding to mesothelin (MSLN).

2. The chimeric antigen receptor according to claim 1, further comprising at least one selected from a signal sequence, a hinge, a transmembrane domain, and an intracellular domain.

3. The chimeric antigen receptor according to claim 1, wherein the binding domain to mesothelin (MSLN) is an anti-mesothelin antibody or a fragment thereof, which comprises a heavy-chain variable region comprising the following heavy-chain CDRs and a light-chain variable region comprising the following light-chain CDRs:
a heavy-chain CDR1 comprising an amino acid sequence of SEQ ID NO: 45, a heavy-chain CDR2 comprising an amino acid sequence of SEQ ID NO: 46, a heavy-chain CDR3 comprising an amino acid sequence of SEQ ID NO: 47, a light-chain CDR1 comprising an amino acid sequence of SEQ ID NO: 48, a light-chain CDR2 comprising an amino acid sequence of SEQ ID NO: 49, and a light-chain CDR3 comprising an amino acid sequence of SEQ ID NO: 50;
a heavy-chain CDR1 comprising an amino acid sequence of SEQ ID NO: 51, a heavy-chain CDR2 comprising an amino acid sequence of SEQ ID NO: 52, a heavy-chain CDR3 comprising an amino acid sequence of SEQ ID NO: 53, a light-chain CDR1 comprising an amino acid sequence of SEQ ID NO: 54, a light-chain CDR2 comprising an amino acid sequence of SEQ ID NO: 55, and a light-chain CDR3 comprising an amino acid sequence of SEQ ID NO: 56; or
a heavy-chain CDR1 comprising an amino acid sequence of SEQ ID NO: 57, a heavy-chain CDR2 comprising an amino acid sequence of SEQ ID NO: 58, a heavy-chain CDR3 comprising an amino acid sequence of SEQ ID NO: 59, a light-chain CDR1 comprising an amino acid sequence of SEQ ID NO: 60, a light-chain CDR2 comprising an amino acid sequence of SEQ ID NO: 61, a light-chain CDR3 comprising an amino acid sequence of SEQ ID NO: 62.

4. The chimeric antigen receptor according to claim 1, wherein the binding domain to mesothelin (MSLN) is an anti-mesothelin antibody, or a fragment thereof, which comprises the following heavy-chain variable region and light-chain variable region:
a heavy-chain variable region comprising an amino acid sequence of SEQ ID NO: 39 and a light-chain variable region comprising an amino acid sequence of SEQ ID NO: 40;
a heavy-chain variable region comprising an amino acid sequence of SEQ ID NO: 41 and a light-chain variable region comprising an amino acid sequence of SEQ ID NO: 42; or
a heavy-chain variable region comprising an amino acid sequence of SEQ ID NO: 43 and a light-chain variable region comprising an amino acid sequence of SEQ ID NO: 44.

5. The chimeric antigen receptor according to claim 1, wherein the binding domain to mesothelin (MSLN) is a single-chain variable fragment (scFv) of the anti-mesothelin antibody.

6. The chimeric antigen receptor according to claim 5, wherein the single-chain variable fragment (scFv) of the anti-mesothelin antibody comprises an amino acid sequence represented by SEQ ID NO: 2, SEQ ID NO: 4 or SEQ ID NO: 6.

7. The chimeric antigen receptor according to claim 2, wherein the signal sequence comprises a signal sequence of CD8α.

8. The chimeric antigen receptor according to claim 7, wherein the signal sequence of CD8α comprises an amino acid sequence represented by SEQ ID NO: 8.

9. The chimeric antigen receptor according to claim 2, wherein the hinge comprises a hinge of CD28 or CD8α.

10. The chimeric antigen receptor according to claim 9, wherein a sequence of the hinge of CD28 or CD8α comprises an amino acid sequence represented by SEQ ID NO: 10 or SEQ ID NO: 16.

11. The chimeric antigen receptor according to claim 2, wherein the transmembrane domain (TM) comprises a transmembrane domain of CD28 or CD8α.

12. The chimeric antigen receptor according to claim 11, wherein the transmembrane domain of CD28 or CD8α comprises an amino acid sequence represented by SEQ ID NO: 12 or SEQ ID NO: 18.

13. The chimeric antigen receptor according to claim 2, wherein the intracellular domain comprises a sequence selected from the group consisting of intracellular signal region sequences of CD28, 4-1BB and CD3ζ, and combinations thereof.

14. The chimeric antigen receptor according to claim 13, wherein the intracellular signal region sequences of CD28, 4-1BB and CD3ζ comprise an amino acid sequence represented by SEQ ID NO: 14, SEQ ID NO: 20, or SEQ ID NO: 22.

15. The chimeric antigen receptor according to claim 2, wherein the chimeric antigen receptor comprises an amino acid sequence represented by SEQ ID NO: 24, 26, 28, 30, 32, 34, or 36.

16. A nucleic acid encoding the chimeric antigen receptor according to any one of claims 1 to 15.

17. The nucleic acid according to claim 16, wherein a nucleotide sequence encoding a CD8α signal sequence of the nucleic acid encoding the chimeric antigen receptor is represented by SEQ ID NO: 7.

18. The nucleic acid according to claim 16, wherein a nucleotide sequence encoding a single-chain variable fragment (scFv) of an anti-mesothelin antibody of the nucleic acid encoding the chimeric antigen receptor is represented by SEQ ID NO: 1, SEQ ID NO: 3, or SEQ ID NO: 5.

19. The nucleic acid according to claim 16, wherein a nucleotide sequence encoding a CD28 or CD8α hinge of the nucleic acid encoding the chimeric antigen receptor is represented by SEQ ID NO: 9 or SEQ ID NO: 15.

20. The nucleic acid according to claim 16, wherein a nucleotide sequence encoding a CD28 or CD8α transmembrane domain of the nucleic acid encoding the chimeric antigen receptor is represented by SEQ ID NO: 11 or SEQ ID NO: 17.

21. The nucleic acid according to claim 16, wherein a nucleotide sequence encoding a CD28, 4-1BB, or CD3ζ intracellular signal region of the nucleic acid encoding the chimeric antigen receptor is represented by SEQ ID NO: 13, SEQ ID NO: 19, or SEQ ID NO: 21.

22. The nucleic acid according to claim 16, wherein the nucleic acid encoding the chimeric antigen receptor is represented by SEQ ID NO: 23, 25, 27, 29, 31, 33, or 35.

23. An expression vector comprising the nucleic acid encoding the chimeric antigen receptor according to any one of claims 16 to 25.

24. A virus comprising the expression vector according to claim 23.

25. An immune cell expressing the chimeric antigen receptor according to any one of claims 1 to 15 on a surface thereof.

26. The immune cell according to claim 25, wherein the immune cell is a T cell, an NK cell or an NKT cell.

27. A composition for treating cancer comprising the immune cell according to claim 25.

28. The composition according to claim 27, wherein the cancer is selected from the group consisting of squamous cell cancer, small-cell lung cancer, non-small-cell lung cancer, pulmonary adenocarcinoma, pulmonary squamous cell carcinoma, peritoneal cancer, hepatocellular carcinoma, gastrointestinal cancer, pancreatic cancer, glioma, cervical cancer, ovarian cancer, liver cancer, bladder cancer, hepatocellular carcinoma, breast cancer, colon cancer, colorectal cancer, endometrial or uterine carcinoma, salivary gland carcinoma, kidney cancer, liver cancer, prostate cancer, vulvar cancer, thyroid cancer, liver carcinoma, leukemia, other lymphoproliferative disorders, and various types of head and neck cancer.


<110>     Green Cross Cell

<120>     Mesothelin-specific chimeric antigen receptor and T lymphocyte
          expressing  this chimeric antigen receptor

<130>     PP-B2331

<150>     KR 2019-0007422
<151>     2019-01-21

<160>     62

<170>     KoPatentIn 3.0

<210>     1
<211>     727
<212>     DNA
<213>     Artificial Sequence

<220>
<223>     MS501 scFv nucleotide


<400>     1
gaggtgcagc tgttggagtc cgggggaggc ttggtacagc ctggggggtc cctgagactc          60

tcctgtgcag cctctggatt cacctttagc aattatgcta tgagctgggt ccgccaggct         120

ccagggaagg ggctggagtg ggtctcaggg atctatcccg atagtggtag tacatattac         180

gctgattctg taaaaggtcg gttcaccatc tccagagaca attccaagaa cacgctgtat         240

ctgcaaatga acagcctgag agccgaggac acggccgtgt attactgtgc gagaaatatt         300

tatacgttcg actactgggg ccagggtaca ctggtcaccg tgagctcagg tggaggcggt         360

tcaggcggag gtggatccgg cggtggcgga tcgcagtctg tgctgactca gccaccctca         420

gcgtctggga cccccgggca gagggtcacc atctcttgta gtggctcttc atctaatatt         480

ggcagtaatg ctgtctcctg gtaccagcag ctcccaggaa cggcccccaa actcctcatc         540

tattataata atcagcggcc aagcggggtc cctgaccgat tctctggctc caagtctggc         600

acctcagcct ccctggccat cagtgggctc cggtccgagg atgaggctga ttattactgt         660

ggttcttggg attctagcct gagtggatat gtcttcggcg gaggcaccaa gctgacggtc         720

ctaggca                                                                   727


<210>     2
<211>     242
<212>     PRT
<213>     Artificial Sequence

<220>
<223>     MS501 scFv amino acid


<400>     2
Glu Val Gln Leu Leu Glu Ser Gly Gly Gly Leu Val Gln Pro Gly Gly
  1               5                  10                  15

Ser Leu Arg Leu Ser Cys Ala Ala Ser Gly Phe Thr Phe Ser Asn Tyr
            20                  25                  30

Ala Met Ser Trp Val Arg Gln Ala Pro Gly Lys Gly Leu Glu Trp Val
        35                  40                  45

Ser Gly Ile Tyr Pro Asp Ser Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Asn Ile Tyr Thr Phe Asp Tyr Trp Gly Gln Gly Thr Leu Val
            100                 105                 110

Thr Val Ser Ser Gly Gly Gly Gly Ser Gly Gly Gly Gly Ser Gly Gly
        115                 120                 125

Gly Gly Ser Gln Ser Val Leu Thr Gln Pro Pro Ser Ala Ser Gly Thr
    130                 135                 140

Pro Gly Gln Arg Val Thr Ile Ser Cys Ser Gly Ser Ser Ser Asn Ile
145                 150                 155                 160

Gly Ser Asn Ala Val Ser Trp Tyr Gln Gln Leu Pro Gly Thr Ala Pro
                165                 170                 175

Lys Leu Leu Ile Tyr Tyr Asn Asn Gln Arg Pro Ser Gly Val Pro Asp
            180                 185                 190

Arg Phe Ser Gly Ser Lys Ser Gly Thr Ser Ala Ser Leu Ala Ile Ser
        195                 200                 205

Gly Leu Arg Ser Glu Asp Glu Ala Asp Tyr Tyr Cys Gly Ser Trp Asp
    210                 215                 220

Ser Ser Leu Ser Gly Tyr Val Phe Gly Gly Gly Thr Lys Leu Thr Val
225                 230                 235                 240

Leu Gly

<210>    3
<211>    726
<212>    DNA
<213>    Artificial Sequence

<220>
<223>    MS503 scFv nucleotide

<400>    3
gaggtgcagc tgttggagtc cgggggaggc ttggtacagc ctggggggtc cctgagactc        60

tcctgtgcag cctctggatt cacctttagc aattatgcta tgagctgggt ccgccaggct       120

ccagggaagg ggctggagtg ggtctcatcg atctatcctg gtgatggtag tacatattac       180

gctgattctg taaaaggtcg gttcaccatc tccagagaca attccaagaa cacgctgtat 240

ctgcaaatga acagcctgag agccgaggac acggccgtgt attactgtgc gaaaaatgcg 300

tttacgttcg actactgggg ccagggtaca ctggtcaccg tgagctcagg tggaggcggt 360

tcaggcggag gtggatccgg cggtggcgga tcgcagtctg tgctgactca gccaccctca 420

gcgtctggga cccccgggca gagggtcacc atctcttgta gtggctcttc atctaatatt 480

ggcagtaatg ctgtctcctg gtaccagcag ctcccaggaa cggcccccaa actcctcatc 540

tattataata gtcatcggcc aagcggggtc cctgaccgat tctctggctc caagtctggc 600

acctcagcct ccctggccat cagtgggctc cggtccgagg atgaggctga ttattactgt 660

ggtacttggg attctagcct gagtggttat gtcttcggcg gaggcaccaa gctgacggtc 720

ctaggc 726

<210> 4
<211> 242
<212> PRT
<213> Artificial Sequence

<220>
<223> MS503 scFv amino acid

<400> 4
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asn Tyr
            20                  25                  30

Ala Met Ser Trp Val Arg Gln Ala Pro Gly Lys Gly Leu Glu Trp Val
        35                  40                  45

Ser Ser Ile Tyr Pro Gly Asp Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Asn Ala Phe Thr Phe Asp Tyr Trp Gly Gln Gly Thr Leu Val
            100                 105                 110

Thr Val Ser Ser Gly Gly Gly Gly Ser Gly Gly Gly Gly Ser Gly Gly
        115                 120                 125

Gly Gly Ser Gln Ser Val Leu Thr Gln Pro Pro Ser Ala Ser Gly Thr
    130                 135                 140

Pro Gly Gln Arg Val Thr Ile Ser Cys Ser Gly Ser Ser Ser Asn Ile
145                 150                 155                 160

Gly Ser Asn Ala Val Ser Trp Tyr Gln Gln Leu Pro Gly Thr Ala Pro
                165                 170                 175

Lys Leu Leu Ile Tyr Tyr Asn Ser His Arg Pro Ser Gly Val Pro Asp
            180                 185                 190

Arg Phe Ser Gly Ser Lys Ser Gly Thr Ser Ala Ser Leu Ala Ile Ser
        195                 200                 205

Gly Leu Arg Ser Glu Asp Glu Ala Asp Tyr Tyr Cys Gly Thr Trp Asp
    210                 215                 220

Ser Ser Leu Ser Gly Tyr Val Phe Gly Gly Gly Thr Lys Leu Thr Val
225                 230                 235                 240

Leu Gly

<210>    5
<211>    726
<212>    DNA
<213>    Artificial Sequence

<220>
<223>    C2G4 scFv nucleotide

<400>    5
gaggtgcagc tgttggagtc tgggggaggc ttggtacagc ctggggggtc cctgagactc      60

tcctgtgcgg cctctggatt cacctttagc aattatgcta tgagctgggt ccgccaggct     120

ccagggaagg ggctggagtg ggtctcaggg atccctcctg atagtggtag taaatattac     180

gctgattctg taagaggtcg gttcaccgtc tccagagaca attccaagaa cacgctgtat     240

ctgcaaatga acagcctgag agccgaggac acggccgtgt attactgtgc gaaaaatatg     300

ctgtcgttcg actactgggg ccagggtaca ctggtcaccg tgagctcagg tggaggcggt     360

tcaggcggag gtggatccgg cggtggcgga tcgcagtctg tgctgactca gccaccctca     420

gcgtctggga cccccgggca gagggtcacc atctcttgta ctggctcttc atctaatatt     480

ggctccaatg ctgtctcctg gtaccagcag ctcccaggaa cggcccccaa actcctcatc     540

tattataata gtaagcggcc aagcggggtc cctgaccgat tctctggctc caagtctggc     600

acctcagcct ccctggccat cagtgggctc cggtccgagg atgaggctga ttattactgt     660

ggttcttggg atcctagcct gaatggttat gtcttcggcg gaggcaccaa ggtgaccgtc     720

ctaggt                                                                726

<210>    6
<211>    242
<212>    PRT
<213>    Artificial Sequence

<220>
<223>    C2G4 scFv amino acid

<400>    6
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asn Tyr
            20                  25                  30

Ala Met Ser Trp Val Arg Gln Ala Pro Gly Lys Gly Leu Glu Trp Val
        35                  40                  45

Ser Gly Ile Pro Pro Asp Ser Gly Ser Lys Tyr Tyr Ala Asp Ser Val
    50                  55                  60

Arg Gly Arg Phe Thr Val Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Asn Met Leu Ser Phe Asp Tyr Trp Gly Gln Gly Thr Leu Val
            100                 105                 110

Thr Val Ser Ser Gly Gly Gly Gly Ser Gly Gly Gly Gly Ser Gly Gly
        115                 120                 125

Gly Gly Ser Gln Ser Val Leu Thr Gln Pro Pro Ser Ala Ser Gly Thr
    130                 135                 140

Pro Gly Gln Arg Val Thr Ile Ser Cys Thr Gly Ser Ser Ser Asn Ile
145                 150                 155                 160

Gly Ser Asn Ala Val Ser Trp Tyr Gln Gln Leu Pro Gly Thr Ala Pro
                165                 170                 175

Lys Leu Leu Ile Tyr Tyr Asn Ser Lys Arg Pro Ser Gly Val Pro Asp
            180                 185                 190

Arg Phe Ser Gly Ser Lys Ser Gly Thr Ser Ala Ser Leu Ala Ile Ser
        195                 200                 205

Gly Leu Arg Ser Glu Asp Glu Ala Asp Tyr Tyr Cys Gly Ser Trp Asp
    210                 215                 220

Pro Ser Leu Asn Gly Tyr Val Phe Gly Gly Gly Thr Lys Val Thr Val
225                 230                 235                 240

Leu Gly



<210>    7
<211>    63
<212>    DNA
<213>    Artificial Sequence

<220>
<223>    CD8 alpha signal sequence nucleotide


<400>    7
atggccttac cagtgaccgc cttgctcctg ccgctggcct tgctgctcca cgccgccagg        60

ccg                                                                      63

<210>    8
<211>    21
<212>    PRT
<213>    Artificial Sequence

<220>
<223>    CD8 alpha signal sequence amino acid


<400>    8
Met Ala Leu Pro Val Thr Ala Leu Leu Leu Pro Leu Ala Leu Leu Leu
1               5                   10                  15

His Ala Ala Arg Pro
            20


<210>    9
<211>    402
<212>    DNA
<213>    Artificial Sequence

<220>
<223>    CD28 Hinge nucleotide


<400>    9
aacaagattt tggtgaagca gtcgcccatg cttgtagcgt acgacaatgc ggtcaacctt      60

agctgcaagt attcctacaa tctcttctca agggagttcc gggcatccct tcacaaagga     120

ctggatagtg ctgtggaagt ctgtgttgta tatgggaatt actcccagca gcttcaggtt     180

tactcaaaaa cggggttcaa ctgtgatggg aaattgggca atgaatcagt gacattctac     240

ctccagaatt tgtatgttaa ccaaacagat atttacttct gcaaaattga agttatgtat     300

cctcctcctt acctagacaa tgagaagagc aatggaacca ttatccatgt gaaagggaaa     360

cacctttgtc caagtcccct atttcccgga ccttctaagc cc                        402


<210>    10
<211>    39
<212>    PRT
<213>    Artificial Sequence

<220>
<223>    CD28 Hinge amino acid


<400>    10
Ile Glu Val Met Tyr Pro Pro Pro Tyr Leu Asp Asn Glu Lys Ser Asn
1               5                   10                  15

Gly Thr Ile Ile His Val Lys Gly Lys His Leu Cys Pro Ser Pro Leu
            20                  25                  30

Phe Pro Gly Pro Ser Lys Pro
        35

<210>	11
<211>	81
<212>	DNA
<213>	Artificial Sequence

<220>
<223>	CD28 TM nucleotide


<400>	11
ttttgggtgc tggtggtggt tggtggagtc ctggcttgct atagcttgct agtaacagtg	60

gcctttatta ttttctgggt g	81


<210>	12
<211>	27
<212>	PRT
<213>	Artificial Sequence

<220>
<223>	CD28 TM amino acid


<400>	12
Phe Trp Val Leu Val Val Val Gly Gly Val Leu Ala Cys Tyr Ser Leu
1               5                   10                  15

Leu Val Thr Val Ala Phe Ile Ile Phe Trp Val
            20                  25


<210>	13
<211>	123
<212>	DNA
<213>	Artificial Sequence

<220>
<223>	CD28 SD nucleotide


<400>	13
aggagtaaga ggagcaggct cctgcacagt gactacatga acatgactcc ccgccgcccc	60

gggcccaccc gcaagcatta ccagccctat gccccaccac gcgacttcgc agcctatcgc	120

tcc	123


<210>	14
<211>	41
<212>	PRT
<213>	Artificial Sequence

<220>
<223>	CD28 SD amino acid


<400>	14
Arg Ser Lys Arg Ser Arg Leu Leu His Ser Asp Tyr Met Asn Met Thr
1               5                   10                  15

Pro Arg Arg Pro Gly Pro Thr Arg Lys His Tyr Gln Pro Tyr Ala Pro
            20                  25                  30

Pro Arg Asp Phe Ala Ala Tyr Arg Ser
        35                  40

<210>    15
<211>    144
<212>    DNA
<213>    Artificial Sequence

<220>
<223>    CD8 alpha Hinge nucleotide

<400>    15
gcgaagccca ccacgacgcc agcgccgcga ccaccaacac cggcgcccac catcgcgtcg        60

cagcccctgt ccctgcgccc agaggcgtgc cggccagcgg cggggggcgc agtgcacacg       120

agggggctgg acttcgcctg tgat                                              144

<210>    16
<211>    48
<212>    PRT
<213>    Artificial Sequence

<220>
<223>    CD8 alpha Hinge amino acid

<400>    16
Ala Lys Pro Thr Thr Thr Pro Ala Pro Arg Pro Pro Thr Pro Ala Pro
 1               5                  10                  15

Thr Ile Ala Ser Gln Pro Leu Ser Leu Arg Pro Glu Ala Cys Arg Pro
            20                  25                  30

Ala Ala Gly Gly Ala Val His Thr Arg Gly Leu Asp Phe Ala Cys Asp
        35                  40                  45

<210>    17
<211>    72
<212>    DNA
<213>    Artificial Sequence

<220>
<223>    CD8 alpha TM nucleotide

<400>    17
atctacatct gggcgccctt ggccgggact tgtggggtcc ttctcctgtc actggttatc        60

accctttact gc                                                            72

<210>    18
<211>    24

<212>    PRT
<213>    Artificial Sequence

<220>
<223>    CD8 alpha TM amino acid


<400>    18
Ile Tyr Ile Trp Ala Pro Leu Ala Gly Thr Cys Gly Val Leu Leu Leu
1               5                   10                  15

Ser Leu Val Ile Thr Leu Tyr Cys
            20


<210>    19
<211>    126
<212>    DNA
<213>    Artificial Sequence

<220>
<223>    4-1BB SD nucleotide


<400>    19
aaacggggca gaaagaaact cctgtatata ttcaaacaac catttatgag accagtacaa        60

actactcaag aggaagatgg ctgtagctgc cgatttccag aagaagaaga aggaggatgt       120

gaactg                                                                  126


<210>    20
<211>    42
<212>    PRT
<213>    Artificial Sequence

<220>
<223>    4-1BB SD amino acid


<400>    20
Lys Arg Gly Arg Lys Lys Leu Leu Tyr Ile Phe Lys Gln Pro Phe Met
1               5                   10                  15

Arg Pro Val Gln Thr Thr Gln Glu Glu Asp Gly Cys Ser Cys Arg Phe
            20                  25                  30

Pro Glu Glu Glu Glu Gly Gly Cys Glu Leu
        35                  40


<210>    21
<211>    336
<212>    DNA
<213>    Artificial Sequence

<220>
<223>    CD3 zeta nucleotide


<400>    21
agagtgaagt tcagcaggag cgcagacgcc cccgcgtacc agcagggcca gaaccagctc        60

tataacgagc tcaatctagg acgaagagag gagtacgatg ttttggacaa gagacgtggc 120

cgggaccctg agatgggggg aaagccgaga aggaagaacc ctcaggaagg cctgtacaat 180

gaactgcaga aagataagat ggcggaggcc tacagtgaga ttgggatgaa aggcgagcgc 240

cggaggggca aggggcacga tggcctttac cagggtctca gtacagccac caaggacacc 300

tacgacgccc ttcacatgca ggccctgccc cctcgc 336

<210> 22
<211> 112
<212> PRT
<213> Artificial Sequence

<220>
<223> CD3 zeta amino acid

<400> 22
Arg Val Lys Phe Ser Arg Ser Ala Asp Ala Pro Ala Tyr Gln Gln Gly
1               5                   10                  15

Gln Asn Gln Leu Tyr Asn Glu Leu Asn Leu Gly Arg Arg Glu Glu Tyr
            20                  25                  30

Asp Val Leu Asp Lys Arg Arg Gly Arg Asp Pro Glu Met Gly Gly Lys
        35                  40                  45

Pro Arg Arg Lys Asn Pro Gln Glu Gly Leu Tyr Asn Glu Leu Gln Lys
    50                  55                  60

Asp Lys Met Ala Glu Ala Tyr Ser Glu Ile Gly Met Lys Gly Glu Arg
65                  70                  75                  80

Arg Arg Gly Lys Gly His Asp Gly Leu Tyr Gln Gly Leu Ser Thr Ala
                85                  90                  95

Thr Lys Asp Thr Tyr Asp Ala Leu His Met Gln Ala Leu Pro Pro Arg
            100                 105                 110

<210> 23
<211> 1734
<212> DNA
<213> Artificial Sequence

<220>
<223> 501(28H)28z nucleotide

<400> 23
atggccttac cagtgaccgc cttgctcctg ccgctggcct tgctgctcca cgccgccagg 60

ccggaggtgc agctgttgga gtccggggga ggcttggtac agcctggggg gtccctgaga 120

ctctcctgtg cagcctctgg attcaccttt agcaattatg ctatgagctg ggtccgccag 180

gctccaggga aggggctgga gtgggtctca gggatctatc ccgatagtgg tagtacatat 240

tacgctgatt ctgtaaaagg tcggttcacc atctccagag acaattccaa gaacacgctg 300

tatctgcaaa tgaacagcct gagagccgag gacacggccg tgtattactg tgcgagaaat 360

atttatacgt tcgactactg gggccagggt acactggtca ccgtgagctc aggtggaggc 420

ggttcaggcg gaggtggatc cggcggtggc ggatcgcagt ctgtgctgac tcagccaccc 480

tcagcgtctg ggacccccgg gcagagggtc accatctctt gtagtggctc ttcatctaat 540

attggcagta atgctgtctc ctggtaccag cagctcccag gaacggcccc caaactcctc 600

atctattata ataatcagcg gccaagcggg gtccctgacc gattctctgg ctccaagtct 660

ggcacctcag cctccctggc catcagtggg ctccggtccg aggatgaggc tgattattac 720

tgtggttctt gggattctag cctgagtgga tatgtcttcg gcggaggcac caagctgacg 780

gtcctaggca acaagatttt ggtgaagcag tcgcccatgc ttgtagcgta cgacaatgcg 840

gtcaacctta gctgcaagta ttcctacaat ctcttctcaa gggagttccg ggcatccctt 900

cacaaaggac tggatagtgc tgtggaagtc tgtgttgtat atgggaatta ctcccagcag 960

cttcaggttt actcaaaaac ggggttcaac tgtgatggga aattgggcaa tgaatcagtg 1020

acattctacc tccagaattt gtatgttaac caaacagata tttacttctg caaaattgaa 1080

gttatgtatc ctcctcctta cctagacaat gagaagagca atggaaccat tatccatgtg 1140

aaagggaaac acctttgtcc aagtccccta tttcccggac cttctaagcc cttttgggtg 1200

ctggtggtgg ttggtggagt cctggcttgc tatagcttgc tagtaacagt ggcctttatt 1260

attttctggg tgaggagtaa gaggagcagg ctcctgcaca gtgactacat gaacatgact 1320

ccccgccgcc ccgggcccac ccgcaagcat taccagccct atgccccacc acgcgacttc 1380

gcagcctatc gctccagagt gaagttcagc aggagcgcag acgcccccgc gtaccagcag 1440

ggccagaacc agctctataa cgagctcaat ctaggacgaa gagaggagta cgatgttttg 1500

gacaagagac gtggccggga ccctgagatg gggggaaagc cgagaaggaa gaaccctcag 1560

gaaggcctgt acaatgaact gcagaaagat aagatggcgg aggcctacag tgagattggg 1620

atgaaaggcg agcgccggag gggcaagggg cacgatggcc tttaccaggg tctcagtaca 1680

gccaccaagg acacctacga cgcccttcac atgcaggccc tgccccctcg ctga 1734

<210> 24
<211> 482
<212> PRT
<213> Artificial Sequence

<220>
<223> 501(28H)28z amino acid

<400> 24
Met Ala Leu Pro Val Thr Ala Leu Leu Leu Pro Leu Ala Leu Leu Leu

1               5                   10                  15

His Ala Ala Arg Pro Glu Val Gln Leu Leu Glu Ser Gly Gly Gly Leu
                20                  25                  30

Val Gln Pro Gly Gly Ser Leu Arg Leu Ser Cys Ala Ala Ser Gly Phe
            35                  40                  45

Thr Phe Ser Asn Tyr Ala Met Ser Trp Val Arg Gln Ala Pro Gly Lys
    50                  55                  60

Gly Leu Glu Trp Val Ser Gly Ile Tyr Pro Asp Ser Gly Ser Thr Tyr
65                  70                  75                  80

Tyr Ala Asp Ser Val Lys Gly Arg Phe Thr Ile Ser Arg Asp Asn Ser
                85                  90                  95

Lys Asn Thr Leu Tyr Leu Gln Met Asn Ser Leu Arg Ala Glu Asp Thr
            100                 105                 110

Ala Val Tyr Tyr Cys Ala Arg Asn Ile Tyr Thr Phe Asp Tyr Trp Gly
        115                 120                 125

Gln Gly Thr Leu Val Thr Val Ser Ser Gly Gly Gly Gly Ser Gly Gly
    130                 135                 140

Gly Gly Ser Gly Gly Gly Gly Ser Gln Ser Val Leu Thr Gln Pro Pro
145                 150                 155                 160

Ser Ala Ser Gly Thr Pro Gly Gln Arg Val Thr Ile Ser Cys Ser Gly
                165                 170                 175

Ser Ser Ser Asn Ile Gly Ser Asn Ala Val Ser Trp Tyr Gln Gln Leu
            180                 185                 190

Pro Gly Thr Ala Pro Lys Leu Leu Ile Tyr Tyr Asn Asn Gln Arg Pro
        195                 200                 205

Ser Gly Val Pro Asp Arg Phe Ser Gly Ser Lys Ser Gly Thr Ser Ala
    210                 215                 220

Ser Leu Ala Ile Ser Gly Leu Arg Ser Glu Asp Glu Ala Asp Tyr Tyr
225                 230                 235                 240

Cys Gly Ser Trp Asp Ser Ser Leu Ser Gly Tyr Val Phe Gly Gly Gly
                245                 250                 255

Thr Lys Leu Thr Val Leu Gly Ile Glu Val Met Tyr Pro Pro Pro Tyr
            260                 265                 270

Leu Asp Asn Glu Lys Ser Asn Gly Thr Ile Ile His Val Lys Gly Lys
        275                 280                 285

His Leu Cys Pro Ser Pro Leu Phe Pro Gly Pro Ser Lys Pro Phe Trp
    290                 295                 300

Val Leu Val Val Val Gly Gly Val Leu Ala Cys Tyr Ser Leu Leu Val
305                 310                 315                 320

Thr Val Ala Phe Ile Ile Phe Trp Val Arg Ser Lys Arg Ser Arg Leu
                325                 330                 335

Leu His Ser Asp Tyr Met Asn Met Thr Pro Arg Arg Pro Gly Pro Thr

340                 345                 350

Arg Lys His Tyr Gln Pro Tyr Ala Pro Pro Arg Asp Phe Ala Ala Tyr
        355                 360                 365

Arg Ser Arg Val Lys Phe Ser Arg Ser Ala Asp Ala Pro Ala Tyr Gln
    370                 375                 380

Gln Gly Gln Asn Gln Leu Tyr Asn Glu Leu Asn Leu Gly Arg Arg Glu
385                 390                 395                 400

Glu Tyr Asp Val Leu Asp Lys Arg Arg Gly Arg Asp Pro Glu Met Gly
                405                 410                 415

Gly Lys Pro Arg Arg Lys Asn Pro Gln Glu Gly Leu Tyr Asn Glu Leu
            420                 425                 430

Gln Lys Asp Lys Met Ala Glu Ala Tyr Ser Glu Ile Gly Met Lys Gly
        435                 440                 445

Glu Arg Arg Arg Gly Lys Gly His Asp Gly Leu Tyr Gln Gly Leu Ser
    450                 455                 460

Thr Ala Thr Lys Asp Thr Tyr Asp Ala Leu His Met Gln Ala Leu Pro
465                 470                 475                 480

Pro Arg

<210>    25
<211>    1734
<212>    DNA
<213>    Artificial Sequence

<220>
<223>    503(28H)28z nucleotide

<400>    25
atggccttac cagtgaccgc cttgctcctg ccgctggcct tgctgctcca cgccgccagg      60

ccggaggtgc agctgttgga gtccggggga ggcttggtac agcctggggg gtccctgaga     120

ctctcctgtg cagcctctgg attcaccttt agcaattatg ctatgagctg ggtccgccag     180

gctccaggga aggggctgga gtgggtctca tcgatctatc ctggtgatgg tagtacatat     240

tacgctgatt ctgtaaaagg tcggttcacc atctccagag acaattccaa gaacacgctg     300

tatctgcaaa tgaacagcct gagagccgag gacacggccg tgtattactg tgcgaaaaat     360

gcgtttacgt tcgactactg gggccagggt acactggtca ccgtgagctc aggtggaggc     420

ggttcaggcg gaggtggatc cggcggtggc ggatcgcagt ctgtgctgac tcagccaccc     480

tcagcgtctg ggacccccgg gcagagggtc accatctctt gtagtggctc ttcatctaat     540

attggcagta atgctgtctc ctggtaccag cagctcccag gaacggcccc caaactcctc     600

atctattata atagtcatcg gccaagcggg gtccctgacc gattctctgg ctccaagtct     660

ggcacctcag cctccctggc catcagtggg ctccggtccg aggatgaggc tgattattac     720

tgtggtactt gggattctag cctgagtggt tatgtcttcg gcggaggcac caagctgacg 780

gtcctaggca acaagatttt ggtgaagcag tcgcccatgc ttgtagcgta cgacaatgcg 840

gtcaacctta gctgcaagta ttcctacaat ctcttctcaa gggagttccg ggcatccctt 900

cacaaaggac tggatagtgc tgtggaagtc tgtgttgtat atgggaatta ctcccagcag 960

cttcaggttt actcaaaaac ggggttcaac tgtgatggga aattgggcaa tgaatcagtg 1020

acattctacc tccagaattt gtatgttaac caaacagata tttacttctg caaaattgaa 1080

gttatgtatc ctcctcctta cctagacaat gagaagagca atggaaccat tatccatgtg 1140

aaagggaaac acctttgtcc aagtccccta tttcccggac cttctaagcc cttttgggtg 1200

ctggtggtgg ttggtggagt cctggcttgc tatagcttgc tagtaacagt ggcctttatt 1260

attttctggg tgaggagtaa gaggagcagg ctcctgcaca gtgactacat gaacatgact 1320

ccccgccgcc ccgggcccac ccgcaagcat taccagccct atgccccacc acgcgacttc 1380

gcagcctatc gctccagagt gaagttcagc aggagcgcag acgcccccgc gtaccagcag 1440

ggccagaacc agctctataa cgagctcaat ctaggacgaa gagaggagta cgatgttttg 1500

gacaagagac gtggccggga ccctgagatg gggggaaagc cgagaaggaa gaaccctcag 1560

gaaggcctgt acaatgaact gcagaaagat aagatggcgg aggcctacag tgagattggg 1620

atgaaaggcg agcgccggag gggcaagggg cacgatggcc tttaccaggg tctcagtaca 1680

gccaccaagg acacctacga cgcccttcac atgcaggccc tgccccctcg ctga 1734

<210> 26
<211> 482
<212> PRT
<213> Artificial Sequence

<220>
<223> 503(28H)28z amino acid

<400> 26
Met Ala Leu Pro Val Thr Ala Leu Leu Leu Pro Leu Ala Leu Leu Leu
1               5                   10                  15

His Ala Ala Arg Pro Glu Val Gln Leu Leu Glu Ser Gly Gly Gly Leu
            20                  25                  30

Val Gln Pro Gly Gly Ser Leu Arg Leu Ser Cys Ala Ala Ser Gly Phe
        35                  40                  45

Thr Phe Ser Asn Tyr Ala Met Ser Trp Val Arg Gln Ala Pro Gly Lys
    50                  55                  60

Gly Leu Glu Trp Val Ser Ser Ile Tyr Pro Gly Asp Gly Ser Thr Tyr
65                  70                  75                  80

Tyr Ala Asp Ser Val Lys Gly Arg Phe Thr Ile Ser Arg Asp Asn Ser
                85                  90                  95

Lys Asn Thr Leu Tyr Leu Gln Met Asn Ser Leu Arg Ala Glu Asp Thr
            100                 105                 110

Ala Val Tyr Tyr Cys Ala Lys Asn Ala Phe Thr Phe Asp Tyr Trp Gly
        115                 120                 125

Gln Gly Thr Leu Val Thr Val Ser Ser Gly Gly Gly Gly Ser Gly Gly
    130                 135                 140

Gly Gly Ser Gly Gly Gly Gly Ser Gln Ser Val Leu Thr Gln Pro Pro
145                 150                 155                 160

Ser Ala Ser Gly Thr Pro Gly Gln Arg Val Thr Ile Ser Cys Ser Gly
                165                 170                 175

Ser Ser Ser Asn Ile Gly Ser Asn Ala Val Ser Trp Tyr Gln Gln Leu
            180                 185                 190

Pro Gly Thr Ala Pro Lys Leu Leu Ile Tyr Tyr Asn Ser His Arg Pro
        195                 200                 205

Ser Gly Val Pro Asp Arg Phe Ser Gly Ser Lys Ser Gly Thr Ser Ala
    210                 215                 220

Ser Leu Ala Ile Ser Gly Leu Arg Ser Glu Asp Glu Ala Asp Tyr Tyr
225                 230                 235                 240

Cys Gly Thr Trp Asp Ser Ser Leu Ser Gly Tyr Val Phe Gly Gly Gly
                245                 250                 255

Thr Lys Leu Thr Val Leu Gly Ile Glu Val Met Tyr Pro Pro Pro Tyr
            260                 265                 270

Leu Asp Asn Glu Lys Ser Asn Gly Thr Ile Ile His Val Lys Gly Lys
        275                 280                 285

His Leu Cys Pro Ser Pro Leu Phe Pro Gly Pro Ser Lys Pro Phe Trp
    290                 295                 300

Val Leu Val Val Val Gly Gly Val Leu Ala Cys Tyr Ser Leu Leu Val
305                 310                 315                 320

Thr Val Ala Phe Ile Ile Phe Trp Val Arg Ser Lys Arg Ser Arg Leu
                325                 330                 335

Leu His Ser Asp Tyr Met Asn Met Thr Pro Arg Arg Pro Gly Pro Thr
            340                 345                 350

Arg Lys His Tyr Gln Pro Tyr Ala Pro Pro Arg Asp Phe Ala Ala Tyr
        355                 360                 365

Arg Ser Arg Val Lys Phe Ser Arg Ser Ala Asp Ala Pro Ala Tyr Gln
    370                 375                 380

Gln Gly Gln Asn Gln Leu Tyr Asn Glu Leu Asn Leu Gly Arg Arg Glu
385                 390                 395                 400

Glu Tyr Asp Val Leu Asp Lys Arg Arg Gly Arg Asp Pro Glu Met Gly
                405                 410                 415

Gly Lys Pro Arg Arg Lys Asn Pro Gln Glu Gly Leu Tyr Asn Glu Leu
            420                 425                 430

Gln Lys Asp Lys Met Ala Glu Ala Tyr Ser Glu Ile Gly Met Lys Gly
        435                 440                 445

Glu Arg Arg Arg Gly Lys Gly His Asp Gly Leu Tyr Gln Gly Leu Ser
    450                 455                 460

Thr Ala Thr Lys Asp Thr Tyr Asp Ala Leu His Met Gln Ala Leu Pro
465                 470                 475                 480

Pro Arg

<210>    27
<211>    1734
<212>    DNA
<213>    Artificial Sequence

<220>
<223>    C2G4(28H)28z  nucleotide

<400>    27
atggccttac cagtgaccgc cttgctcctg ccgctggcct tgctgctcca cgccgccagg      60

ccggaggtgc agctgttgga gtctggggga ggcttggtac agcctggggg gtccctgaga     120

ctctcctgtg cggcctctgg attcaccttt agcaattatg ctatgagctg ggtccgccag     180

gctccaggga aggggctgga gtgggtctca gggatccctc ctgatagtgg tagtaaatat     240

tacgctgatt ctgtaagagg tcggttcacc gtctccagag acaattccaa gaacacgctg     300

tatctgcaaa tgaacagcct gagagccgag gacacggccg tgtattactg tgcgaaaaat     360

atgctgtcgt tcgactactg gggccagggt acactggtca ccgtgagctc aggtggaggc     420

ggttcaggcg gaggtggatc cggcggtggc ggatcgcagt ctgtgctgac tcagccaccc     480

tcagcgtctg ggacccccgg gcagagggtc accatctctt gtactggctc ttcatctaat     540

attggctcca atgctgtctc ctggtaccag cagctcccag gaacggcccc caaactcctc     600

atctattata atagtaagcg gccaagcggg gtccctgacc gattctctgg ctccaagtct     660

ggcacctcag cctccctggc catcagtggg ctccggtccg aggatgaggc tgattattac     720

tgtggttctt gggatcctag cctgaatggt tatgtcttcg gcggaggcac caaggtgacc     780

gtcctaggta acaagatttt ggtgaagcag tcgcccatgc ttgtagcgta cgacaatgcg     840

gtcaacctta gctgcaagta ttcctacaat ctcttctcaa gggagttccg ggcatccctt     900

cacaaaggac tggatagtgc tgtggaagtc tgtgttgtat atgggaatta ctcccagcag     960

cttcaggttt actcaaaaac ggggttcaac tgtgatggga aattgggcaa tgaatcagtg    1020

acattctacc tccagaattt gtatgttaac caaacagata tttacttctg caaaattgaa    1080

gttatgtatc ctcctcctta cctagacaat gagaagagca atggaaccat tatccatgtg    1140

aaagggaaac acctttgtcc aagtccccta tttcccggac cttctaagcc cttttgggtg    1200

ctggtggtgg ttggtggagt cctggcttgc tatagcttgc tagtaacagt ggcctttatt 1260

atttctgggg tgaggagtaa gaggagcagg ctcctgcaca gtgactacat gaacatgact 1320

ccccgccgcc ccgggcccac ccgcaagcat taccagccct atgccccacc acgcgacttc 1380

gcagcctatc gctccagagt gaagttcagc aggagcgcag acgcccccgc gtaccagcag 1440

ggccagaacc agctctataa cgagctcaat ctaggacgaa gagaggagta cgatgttttg 1500

gacaagagac gtggccggga ccctgagatg gggggaaagc cgagaaggaa gaaccctcag 1560

gaaggcctgt acaatgaact gcagaaagat aagatggcgg aggcctacag tgagattggg 1620

atgaaaggcg agcgccggag gggcaagggg cacgatggcc tttaccaggg tctcagtaca 1680

gccaccaagg acacctacga cgcccttcac atgcaggccc tgccccctcg ctga 1734

<210> 28
<211> 482
<212> PRT
<213> Artificial Sequence

<220>
<223> C2G4(28H)28z amino acid

<400> 28
Met Ala Leu Pro Val Thr Ala Leu Leu Leu Pro Leu Ala Leu Leu Leu
1               5                   10                  15

His Ala Ala Arg Pro Glu Val Gln Leu Leu Glu Ser Gly Gly Gly Leu
            20                  25                  30

Val Gln Pro Gly Gly Ser Leu Arg Leu Ser Cys Ala Ala Ser Gly Phe
        35                  40                  45

Thr Phe Ser Asn Tyr Ala Met Ser Trp Val Arg Gln Ala Pro Gly Lys
    50                  55                  60

Gly Leu Glu Trp Val Ser Gly Ile Pro Pro Asp Ser Gly Ser Lys Tyr
65                  70                  75                  80

Tyr Ala Asp Ser Val Arg Gly Arg Phe Thr Val Ser Arg Asp Asn Ser
                85                  90                  95

Lys Asn Thr Leu Tyr Leu Gln Met Asn Ser Leu Arg Ala Glu Asp Thr
            100                 105                 110

Ala Val Tyr Tyr Cys Ala Lys Asn Met Leu Ser Phe Asp Tyr Trp Gly
        115                 120                 125

Gln Gly Thr Leu Val Thr Val Ser Ser Gly Gly Gly Gly Ser Gly Gly
    130                 135                 140

Gly Gly Ser Gly Gly Gly Gly Ser Gln Ser Val Leu Thr Gln Pro Pro
145                 150                 155                 160

Ser Ala Ser Gly Thr Pro Gly Gln Arg Val Thr Ile Ser Cys Thr Gly
                165                 170                 175

Ser Ser Ser Asn Ile Gly Ser Asn Ala Val Ser Trp Tyr Gln Gln Leu
        180                 185                 190

Pro Gly Thr Ala Pro Lys Leu Leu Ile Tyr Tyr Asn Ser Lys Arg Pro
    195                 200                 205

Ser Gly Val Pro Asp Arg Phe Ser Gly Ser Lys Ser Gly Thr Ser Ala
    210                 215                 220

Ser Leu Ala Ile Ser Gly Leu Arg Ser Glu Asp Glu Ala Asp Tyr Tyr
225                 230                 235                 240

Cys Gly Ser Trp Asp Pro Ser Leu Asn Gly Tyr Val Phe Gly Gly Gly
                245                 250                 255

Thr Lys Val Thr Val Leu Gly Ile Glu Val Met Tyr Pro Pro Pro Tyr
        260                 265                 270

Leu Asp Asn Glu Lys Ser Asn Gly Thr Ile Ile His Val Lys Gly Lys
    275                 280                 285

His Leu Cys Pro Ser Pro Leu Phe Pro Gly Pro Ser Lys Pro Phe Trp
    290                 295                 300

Val Leu Val Val Val Gly Gly Val Leu Ala Cys Tyr Ser Leu Leu Val
305                 310                 315                 320

Thr Val Ala Phe Ile Ile Phe Trp Val Arg Ser Lys Arg Ser Arg Leu
                325                 330                 335

Leu His Ser Asp Tyr Met Asn Met Thr Pro Arg Arg Pro Gly Pro Thr
        340                 345                 350

Arg Lys His Tyr Gln Pro Tyr Ala Pro Pro Arg Asp Phe Ala Ala Tyr
    355                 360                 365

Arg Ser Arg Val Lys Phe Ser Arg Ser Ala Asp Ala Pro Ala Tyr Gln
    370                 375                 380

Gln Gly Gln Asn Gln Leu Tyr Asn Glu Leu Asn Leu Gly Arg Arg Glu
385                 390                 395                 400

Glu Tyr Asp Val Leu Asp Lys Arg Arg Gly Arg Asp Pro Glu Met Gly
                405                 410                 415

Gly Lys Pro Arg Arg Lys Asn Pro Gln Glu Gly Leu Tyr Asn Glu Leu
        420                 425                 430

Gln Lys Asp Lys Met Ala Glu Ala Tyr Ser Glu Ile Gly Met Lys Gly
    435                 440                 445

Glu Arg Arg Arg Gly Lys Gly His Asp Gly Leu Tyr Gln Gly Leu Ser
    450                 455                 460

Thr Ala Thr Lys Asp Thr Tyr Asp Ala Leu His Met Gln Ala Leu Pro
465                 470                 475                 480

Pro Arg

<210>   29
<211>   1008

<212>     DNA
<213>     Artificial Sequence

<220>
<223>     501(8H) delta nucleotide


<400>     29
atggccttac cagtgaccgc cttgctcctg ccgctggcct tgctgctcca cgccgccagg          60

ccggaggtgc agctgttgga gtccggggga ggcttggtac agcctggggg gtccctgaga         120

ctctcctgtg cagcctctgg attcaccttt agcaattatg ctatgagctg ggtccgccag         180

gctccaggga aggggctgga gtgggtctca gggatctatc ccgatagtgg tagtacatat         240

tacgctgatt ctgtaaaagg tcggttcacc atctccagag acaattccaa gaacacgctg         300

tatctgcaaa tgaacagcct gagagccgag gacacggccg tgtattactg tgcgagaaat         360

atttatacgt tcgactactg gggccagggt acactggtca ccgtgagctc aggtggaggc         420

ggttcaggcg gaggtggatc cggcggtggc ggatcgcagt ctgtgctgac tcagccaccc         480

tcagcgtctg ggacccccgg gcagagggtc accatctctt gtagtggctc ttcatctaat         540

attggcagta atgctgtctc ctggtaccag cagctcccag gaacggcccc caaactcctc         600

atctattata ataatcagcg gccaagcggg gtccctgacc gattctctgg ctccaagtct         660

ggcacctcag cctccctggc catcagtggg ctccggtccg aggatgaggc tgattattac         720

tgtggttctt gggattctag cctgagtgga tatgtcttcg gcggaggcac caagctgacg         780

gtcctaggcg cgaagcccac cacgacgcca gcgccgcgac caccaacacc ggcgcccacc         840

atcgcgtcgc agcccctgtc cctgcgccca gaggcgtgcc ggccagcggc ggggggcgca         900

gtgcacacga gggggctgga cttcgcctgt gatatctaca tctgggcgcc cttggccggg         960

acttgtgggg tccttctcct gtcactggtt atcacccttt actgctga                     1008


<210>     30
<211>     335
<212>     PRT
<213>     Artificial Sequence

<220>
<223>     501(8H) delta amino acid


<400>     30
Met Ala Leu Pro Val Thr Ala Leu Leu Leu Pro Leu Ala Leu Leu Leu
1               5                   10                  15

His Ala Ala Arg Pro Glu Val Gln Leu Leu Glu Ser Gly Gly Gly Leu
            20                  25                  30

Val Gln Pro Gly Gly Ser Leu Arg Leu Ser Cys Ala Ala Ser Gly Phe
        35                  40                  45

Thr Phe Ser Asn Tyr Ala Met Ser Trp Val Arg Gln Ala Pro Gly Lys

50                  55                  60

Gly Leu Glu Trp Val Ser Gly Ile Tyr Pro Asp Ser Gly Ser Thr Tyr
65                  70                  75                  80

Tyr Ala Asp Ser Val Lys Gly Arg Phe Thr Ile Ser Arg Asp Asn Ser
                85                  90                  95

Lys Asn Thr Leu Tyr Leu Gln Met Asn Ser Leu Arg Ala Glu Asp Thr
            100                 105                 110

Ala Val Tyr Tyr Cys Ala Arg Asn Ile Tyr Thr Phe Asp Tyr Trp Gly
        115                 120                 125

Gln Gly Thr Leu Val Thr Val Ser Ser Gly Gly Gly Gly Ser Gly Gly
    130                 135                 140

Gly Gly Ser Gly Gly Gly Gly Ser Gln Ser Val Leu Thr Gln Pro Pro
145                 150                 155                 160

Ser Ala Ser Gly Thr Pro Gly Gln Arg Val Thr Ile Ser Cys Ser Gly
                165                 170                 175

Ser Ser Ser Asn Ile Gly Ser Asn Ala Val Ser Trp Tyr Gln Gln Leu
            180                 185                 190

Pro Gly Thr Ala Pro Lys Leu Leu Ile Tyr Tyr Asn Asn Gln Arg Pro
        195                 200                 205

Ser Gly Val Pro Asp Arg Phe Ser Gly Ser Lys Ser Gly Thr Ser Ala
    210                 215                 220

Ser Leu Ala Ile Ser Gly Leu Arg Ser Glu Asp Glu Ala Asp Tyr Tyr
225                 230                 235                 240

Cys Gly Ser Trp Asp Ser Ser Leu Ser Gly Tyr Val Phe Gly Gly Gly
                245                 250                 255

Thr Lys Leu Thr Val Leu Gly Ala Lys Pro Thr Thr Thr Pro Ala Pro
            260                 265                 270

Arg Pro Pro Thr Pro Ala Pro Thr Ile Ala Ser Gln Pro Leu Ser Leu
        275                 280                 285

Arg Pro Glu Ala Cys Arg Pro Ala Ala Gly Gly Ala Val His Thr Arg
    290                 295                 300

Gly Leu Asp Phe Ala Cys Asp Ile Tyr Ile Trp Ala Pro Leu Ala Gly
305                 310                 315                 320

Thr Cys Gly Val Leu Leu Leu Ser Leu Val Ile Thr Leu Tyr Cys
                325                 330                 335

<210>   31
<211>   1344
<212>   DNA
<213>   Artificial Sequence

<220>
<223>   501(8H)z nucleotide

<400>    31
atggccttac cagtgaccgc cttgctcctg ccgctggcct tgctgctcca cgccgccagg      60

ccggaggtgc agctgttgga gtccggggga ggcttggtac agcctggggg gtccctgaga     120

ctctcctgtg cagcctctgg attcaccttt agcaattatg ctatgagctg ggtccgccag     180

gctccaggga aggggctgga gtgggtctca gggatctatc ccgatagtgg tagtacatat     240

tacgctgatt ctgtaaaagg tcggttcacc atctccagag acaattccaa gaacacgctg     300

tatctgcaaa tgaacagcct gagagccgag gacacggccg tgtattactg tgcgagaaat     360

atttatacgt tcgactactg gggccagggt acactggtca ccgtgagctc aggtggaggc     420

ggttcaggcg gaggtggatc cggcggtggc ggatcgcagt ctgtgctgac tcagccaccc     480

tcagcgtctg ggacccccgg gcagagggtc accatctctt gtagtggctc ttcatctaat     540

attggcagta atgctgtctc ctggtaccag cagctcccag gaacggcccc caaactcctc     600

atctattata ataatcagcg gccaagcggg gtccctgacc gattctctgg ctccaagtct     660

ggcacctcag cctccctggc catcagtggg ctccggtccg aggatgaggc tgattattac     720

tgtggttctt gggattctag cctgagtgga tatgtcttcg gcggaggcac caagctgacg     780

gtcctaggcg cgaagcccac cacgacgcca gcgccgcgac caccaacacc ggcgcccacc     840

atcgcgtcgc agcccctgtc cctgcgccca gaggcgtgcc ggccagcggc ggggggcgca     900

gtgcacacga gggggctgga cttcgcctgt gatatctaca tctgggcgcc cttggccggg     960

acttgtgggg tccttctcct gtcactggtt atcacccttt actgcagagt gaagttcagc    1020

aggagcgcag acgcccccgc gtaccagcag ggccagaacc agctctataa cgagctcaat    1080

ctaggacgaa gagaggagta cgatgttttg gacaagagac gtggccggga ccctgagatg    1140

gggggaaagc cgagaaggaa gaaccctcag gaaggcctgt acaatgaact gcagaaagat    1200

aagatggcgg aggcctacag tgagattggg atgaaaggcg agcgccggag gggcaagggg    1260

cacgatggcc tttaccaggg tctcagtaca gccaccaagg acacctacga cgcccttcac    1320

atgcaggccc tgccccctcg ctga                                           1344

<210>    32
<211>    447
<212>    PRT
<213>    Artificial Sequence

<220>
<223>    501(8H)z amino acid

<400>    32
Met Ala Leu Pro Val Thr Ala Leu Leu Leu Pro Leu Ala Leu Leu Leu
1               5                   10                  15

His Ala Ala Arg Pro Glu Val Gln Leu Leu Glu Ser Gly Gly Gly Leu
            20                  25                  30

Val Gln Pro Gly Gly Ser Leu Arg Leu Ser Cys Ala Ala Ser Gly Phe
        35                  40                  45

Thr Phe Ser Asn Tyr Ala Met Ser Trp Val Arg Gln Ala Pro Gly Lys
    50                  55                  60

Gly Leu Glu Trp Val Ser Gly Ile Tyr Pro Asp Ser Gly Ser Thr Tyr
65                  70                  75                  80

Tyr Ala Asp Ser Val Lys Gly Arg Phe Thr Ile Ser Arg Asp Asn Ser
                85                  90                  95

Lys Asn Thr Leu Tyr Leu Gln Met Asn Ser Leu Arg Ala Glu Asp Thr
            100                 105                 110

Ala Val Tyr Tyr Cys Ala Arg Asn Ile Tyr Thr Phe Asp Tyr Trp Gly
        115                 120                 125

Gln Gly Thr Leu Val Thr Val Ser Ser Gly Gly Gly Gly Ser Gly Gly
    130                 135                 140

Gly Gly Ser Gly Gly Gly Gly Ser Gln Ser Val Leu Thr Gln Pro Pro
145                 150                 155                 160

Ser Ala Ser Gly Thr Pro Gly Gln Arg Val Thr Ile Ser Cys Ser Gly
                165                 170                 175

Ser Ser Ser Asn Ile Gly Ser Asn Ala Val Ser Trp Tyr Gln Gln Leu
            180                 185                 190

Pro Gly Thr Ala Pro Lys Leu Leu Ile Tyr Tyr Asn Asn Gln Arg Pro
        195                 200                 205

Ser Gly Val Pro Asp Arg Phe Ser Gly Ser Lys Ser Gly Thr Ser Ala
    210                 215                 220

Ser Leu Ala Ile Ser Gly Leu Arg Ser Glu Asp Glu Ala Asp Tyr Tyr
225                 230                 235                 240

Cys Gly Ser Trp Asp Ser Ser Leu Ser Gly Tyr Val Phe Gly Gly Gly
                245                 250                 255

Thr Lys Leu Thr Val Leu Gly Ala Lys Pro Thr Thr Thr Pro Ala Pro
            260                 265                 270

Arg Pro Pro Thr Pro Ala Pro Thr Ile Ala Ser Gln Pro Leu Ser Leu
        275                 280                 285

Arg Pro Glu Ala Cys Arg Pro Ala Ala Gly Gly Ala Val His Thr Arg
    290                 295                 300

Gly Leu Asp Phe Ala Cys Asp Ile Tyr Ile Trp Ala Pro Leu Ala Gly
305                 310                 315                 320

Thr Cys Gly Val Leu Leu Leu Ser Leu Val Ile Thr Leu Tyr Cys Arg
                325                 330                 335

Val Lys Phe Ser Arg Ser Ala Asp Ala Pro Ala Tyr Gln Gln Gly Gln
            340                 345                 350

Asn Gln Leu Tyr Asn Glu Leu Asn Leu Gly Arg Arg Glu Glu Tyr Asp
        355                 360                 365

Val Leu Asp Lys Arg Arg Gly Arg Asp Pro Glu Met Gly Gly Lys Pro
    370                 375                 380

Arg Arg Lys Asn Pro Gln Glu Gly Leu Tyr Asn Glu Leu Gln Lys Asp
385                 390                 395                 400

Lys Met Ala Glu Ala Tyr Ser Glu Ile Gly Met Lys Gly Glu Arg Arg
                405                 410                 415

Arg Gly Lys Gly His Asp Gly Leu Tyr Gln Gly Leu Ser Thr Ala Thr
            420                 425                 430

Lys Asp Thr Tyr Asp Ala Leu His Met Gln Ala Leu Pro Pro Arg
        435                 440                 445

<210>    33
<211>    1470
<212>    DNA
<213>    Artificial Sequence

<220>
<223>    501(8H)BBz nucleotide

<400>    33
atggccttac cagtgaccgc cttgctcctg ccgctggcct tgctgctcca cgccgccagg      60

ccggaggtgc agctgttgga gtccggggga ggcttggtac agcctggggg gtccctgaga     120

ctctcctgtg cagcctctgg attcaccttt agcaattatg ctatgagctg ggtccgccag     180

gctccaggga aggggctgga gtgggtctca gggatctatc ccgatagtgg tagtacatat     240

tacgctgatt ctgtaaaagg tcggttcacc atctccagag acaattccaa gaacacgctg     300

tatctgcaaa tgaacagcct gagagccgag gacacggccg tgtattactg tgcgagaaat     360

atttatacgt tcgactactg gggccagggt acactggtca ccgtgagctc aggtggaggc     420

ggttcaggcg gaggtggatc cggcggtggc ggatcgcagt ctgtgctgac tcagccaccc     480

tcagcgtctg ggacccccgg gcagagggtc accatctctt gtagtggctc ttcatctaat     540

attggcagta atgctgtctc ctggtaccag cagctcccag gaacggcccc caaactcctc     600

atctattata ataatcagcg gccaagcggg gtccctgacc gattctctgg ctccaagtct     660

ggcacctcag cctccctggc catcagtggg ctccggtccg aggatgaggc tgattattac     720

tgtggttctt gggattctag cctgagtgga tatgtcttcg gcggaggcac caagctgacg     780

gtcctaggcg cgaagcccac cacgacgcca gcgccgcgac caccaacacc ggcgcccacc     840

atcgcgtcgc agcccctgtc cctgcgccca gaggcgtgcc ggccagcggc ggggggcgca     900

gtgcacacga gggggctgga cttcgcctgt gatatctaca tctgggcgcc cttggccggg     960

acttgtgggg tccttctcct gtcactggtt atcacccttt actgcaaacg gggcagaaag    1020

aaactcctgt atatattcaa acaaccattt atgagaccag tacaaactac tcaagaggaa 1080

gatggctgta gctgccgatt tccagaagaa gaagaaggag gatgtgaact gagagtgaag 1140

ttcagcagga gcgcagacgc ccccgcgtac cagcagggcc agaaccagct ctataacgag 1200

ctcaatctag gacgaagaga ggagtacgat gttttggaca agagacgtgg ccgggaccct 1260

gagatggggg gaaagccgag aaggaagaac cctcaggaag gcctgtacaa tgaactgcag 1320

aaagataaga tggcggaggc ctacagtgag attgggatga aaggcgagcg ccggaggggc 1380

aaggggcacg atggccttta ccagggtctc agtacagcca ccaaggacac ctacgacgcc 1440

cttcacatgc aggccctgcc ccctcgctga 1470

<210> 34
<211> 489
<212> PRT
<213> Artificial Sequence

<220>
<223> 501(8H)BBz amino acid

<400> 34
Met Ala Leu Pro Val Thr Ala Leu Leu Leu Pro Leu Ala Leu Leu Leu
1               5                   10                  15

His Ala Ala Arg Pro Glu Val Gln Leu Leu Glu Ser Gly Gly Gly Leu
            20                  25                  30

Val Gln Pro Gly Gly Ser Leu Arg Leu Ser Cys Ala Ala Ser Gly Phe
        35                  40                  45

Thr Phe Ser Asn Tyr Ala Met Ser Trp Val Arg Gln Ala Pro Gly Lys
    50                  55                  60

Gly Leu Glu Trp Val Ser Gly Ile Tyr Pro Asp Ser Gly Ser Thr Tyr
65                  70                  75                  80

Tyr Ala Asp Ser Val Lys Gly Arg Phe Thr Ile Ser Arg Asp Asn Ser
                85                  90                  95

Lys Asn Thr Leu Tyr Leu Gln Met Asn Ser Leu Arg Ala Glu Asp Thr
            100                 105                 110

Ala Val Tyr Tyr Cys Ala Arg Asn Ile Tyr Thr Phe Asp Tyr Trp Gly
        115                 120                 125

Gln Gly Thr Leu Val Thr Val Ser Ser Gly Gly Gly Gly Ser Gly Gly
    130                 135                 140

Gly Gly Ser Gly Gly Gly Gly Ser Gln Ser Val Leu Thr Gln Pro Pro
145                 150                 155                 160

Ser Ala Ser Gly Thr Pro Gly Gln Arg Val Thr Ile Ser Cys Ser Gly
                165                 170                 175

Ser Ser Ser Asn Ile Gly Ser Asn Ala Val Ser Trp Tyr Gln Gln Leu
            180                 185                 190

Pro Gly Thr Ala Pro Lys Leu Leu Ile Tyr Tyr Asn Asn Gln Arg Pro
        195                 200                 205

Ser Gly Val Pro Asp Arg Phe Ser Gly Ser Lys Ser Gly Thr Ser Ala
    210                 215                 220

Ser Leu Ala Ile Ser Gly Leu Arg Ser Glu Asp Glu Ala Asp Tyr Tyr
225                 230                 235                 240

Cys Gly Ser Trp Asp Ser Ser Leu Ser Gly Tyr Val Phe Gly Gly Gly
                245                 250                 255

Thr Lys Leu Thr Val Leu Gly Ala Lys Pro Thr Thr Thr Pro Ala Pro
            260                 265                 270

Arg Pro Pro Thr Pro Ala Pro Thr Ile Ala Ser Gln Pro Leu Ser Leu
        275                 280                 285

Arg Pro Glu Ala Cys Arg Pro Ala Ala Gly Gly Ala Val His Thr Arg
    290                 295                 300

Gly Leu Asp Phe Ala Cys Asp Ile Tyr Ile Trp Ala Pro Leu Ala Gly
305                 310                 315                 320

Thr Cys Gly Val Leu Leu Leu Ser Leu Val Ile Thr Leu Tyr Cys Lys
                325                 330                 335

Arg Gly Arg Lys Lys Leu Leu Tyr Ile Phe Lys Gln Pro Phe Met Arg
            340                 345                 350

Pro Val Gln Thr Thr Gln Glu Glu Asp Gly Cys Ser Cys Arg Phe Pro
        355                 360                 365

Glu Glu Glu Glu Gly Gly Cys Glu Leu Arg Val Lys Phe Ser Arg Ser
    370                 375                 380

Ala Asp Ala Pro Ala Tyr Gln Gln Gly Gln Asn Gln Leu Tyr Asn Glu
385                 390                 395                 400

Leu Asn Leu Gly Arg Arg Glu Glu Tyr Asp Val Leu Asp Lys Arg Arg
                405                 410                 415

Gly Arg Asp Pro Glu Met Gly Gly Lys Pro Arg Arg Lys Asn Pro Gln
            420                 425                 430

Glu Gly Leu Tyr Asn Glu Leu Gln Lys Asp Lys Met Ala Glu Ala Tyr
        435                 440                 445

Ser Glu Ile Gly Met Lys Gly Glu Arg Arg Arg Gly Lys Gly His Asp
    450                 455                 460

Gly Leu Tyr Gln Gly Leu Ser Thr Ala Thr Lys Asp Thr Tyr Asp Ala
465                 470                 475                 480

Leu His Met Gln Ala Leu Pro Pro Arg
                485


<210>    35
<211>    1476
<212>    DNA
<213>    Artificial Sequence

<220>
<223>    501(8H)28z nucleotide

<400>    35
atggccttac cagtgaccgc cttgctcctg ccgctggcct tgctgctcca cgccgccagg      60

ccggaggtgc agctgttgga gtccggggga ggcttggtac agcctggggg gtccctgaga     120

ctctcctgtg cagcctctgg attcaccttt agcaattatg ctatgagctg ggtccgccag     180

gctccaggga aggggctgga gtgggtctca gggatctatc ccgatagtgg tagtacatat     240

tacgctgatt ctgtaaaagg tcggttcacc atctccagag acaattccaa gaacacgctg     300

tatctgcaaa tgaacagcct gagagccgag gacacggccg tgtattactg tgcgagaaat     360

atttatacgt tcgactactg gggccagggt acactggtca ccgtgagctc aggtggaggc     420

ggttcaggcg gaggtggatc cggcggtggc ggatcgcagt ctgtgctgac tcagccaccc     480

tcagcgtctg ggacccccgg gcagagggtc accatctctt gtagtggctc ttcatctaat     540

attggcagta atgctgtctc ctggtaccag cagctcccag gaacggcccc caaactcctc     600

atctattata ataatcagcg gccaagcggg gtccctgacc gattctctgg ctccaagtct     660

ggcacctcag cctccctggc catcagtggg ctccggtccg aggatgaggc tgattattac     720

tgtggttctt gggattctag cctgagtgga tatgtcttcg gcggaggcac caagctgacg     780

gtcctaggcg cgaagcccac cacgacgcca gcgccgcgac caccaacacc ggcgcccacc     840

atcgcgtcgc agcccctgtc cctgcgccca gaggcgtgcc ggccagcggc ggggggcgca     900

gtgcacacga gggggctgga cttcgcctgt gattttgggg tgctggtggt ggttggtgga     960

gtcctggctt gctatagctt gctagtaaca gtggccttta ttattttctg ggtgaggagt    1020

aagaggagca ggctcctgca cagtgactac atgaacatga ctccccgccg ccccgggccc    1080

acccgcaagc attaccagcc ctatgcccca ccacgcgact tcgcagccta tcgctccaga    1140

gtgaagttca gcaggagcgc agacgccccc gcgtaccagc agggccagaa ccagctctat    1200

aacgagctca atctaggacg aagagaggag tacgatgttt tggacaagag acgtggccgg    1260

gaccctgaga tggggggaaa gccgagaagg aagaaccctc aggaaggcct gtacaatgaa    1320

ctgcagaaag ataagatggc ggaggcctac agtgagattg ggatgaaagg cgagcgccgg    1380

aggggcaagg ggcacgatgg cctttaccag ggtctcagta cagccaccaa ggacacctac    1440

gacgcccttc acatgcaggc cctgccccct cgctga                              1476

<210>    36
<211>    491
<212>    PRT
<213>    Artificial Sequence

<220>
<223>    501(8H)28z amino acid

<400>    36
Met Ala Leu Pro Val Thr Ala Leu Leu Leu Pro Leu Ala Leu Leu Leu
1               5                   10                  15

His Ala Ala Arg Pro Glu Val Gln Leu Leu Glu Ser Gly Gly Gly Leu
            20                  25                  30

Val Gln Pro Gly Gly Ser Leu Arg Leu Ser Cys Ala Ala Ser Gly Phe
        35                  40                  45

Thr Phe Ser Asn Tyr Ala Met Ser Trp Val Arg Gln Ala Pro Gly Lys
    50                  55                  60

Gly Leu Glu Trp Val Ser Gly Ile Tyr Pro Asp Ser Gly Ser Thr Tyr
65                  70                  75                  80

Tyr Ala Asp Ser Val Lys Gly Arg Phe Thr Ile Ser Arg Asp Asn Ser
                85                  90                  95

Lys Asn Thr Leu Tyr Leu Gln Met Asn Ser Leu Arg Ala Glu Asp Thr
            100                 105                 110

Ala Val Tyr Tyr Cys Ala Arg Asn Ile Tyr Thr Phe Asp Tyr Trp Gly
        115                 120                 125

Gln Gly Thr Leu Val Thr Val Ser Ser Gly Gly Gly Gly Ser Gly Gly
    130                 135                 140

Gly Gly Ser Gly Gly Gly Gly Ser Gln Ser Val Leu Thr Gln Pro Pro
145                 150                 155                 160

Ser Ala Ser Gly Thr Pro Gly Gln Arg Val Thr Ile Ser Cys Ser Gly
                165                 170                 175

Ser Ser Ser Asn Ile Gly Ser Asn Ala Val Ser Trp Tyr Gln Gln Leu
            180                 185                 190

Pro Gly Thr Ala Pro Lys Leu Leu Ile Tyr Tyr Asn Asn Gln Arg Pro
        195                 200                 205

Ser Gly Val Pro Asp Arg Phe Ser Gly Ser Lys Ser Gly Thr Ser Ala
    210                 215                 220

Ser Leu Ala Ile Ser Gly Leu Arg Ser Glu Asp Glu Ala Asp Tyr Tyr
225                 230                 235                 240

Cys Gly Ser Trp Asp Ser Ser Leu Ser Gly Tyr Val Phe Gly Gly Gly
                245                 250                 255

Thr Lys Leu Thr Val Leu Gly Ala Lys Pro Thr Thr Thr Pro Ala Pro
            260                 265                 270

Arg Pro Pro Thr Pro Ala Pro Thr Ile Ala Ser Gln Pro Leu Ser Leu
        275                 280                 285

Arg Pro Glu Ala Cys Arg Pro Ala Ala Gly Gly Ala Val His Thr Arg
    290                 295                 300

Gly Leu Asp Phe Ala Cys Asp Phe Trp Val Leu Val Val Val Gly Gly
305                 310                 315                 320

Val Leu Ala Cys Tyr Ser Leu Leu Val Thr Val Ala Phe Ile Ile Phe
                325                 330                 335

Trp Val Arg Ser Lys Arg Ser Arg Leu Leu His Ser Asp Tyr Met Asn
            340                 345                 350

Met Thr Pro Arg Arg Pro Gly Pro Thr Arg Lys His Tyr Gln Pro Tyr
        355                 360                 365

Ala Pro Pro Arg Asp Phe Ala Ala Tyr Arg Ser Arg Val Lys Phe Ser
    370                 375                 380

Arg Ser Ala Asp Ala Pro Ala Tyr Gln Gln Gly Gln Asn Gln Leu Tyr
385                 390                 395                 400

Asn Glu Leu Asn Leu Gly Arg Arg Glu Glu Tyr Asp Val Leu Asp Lys
                405                 410                 415

Arg Arg Gly Arg Asp Pro Glu Met Gly Gly Lys Pro Arg Arg Lys Asn
            420                 425                 430

Pro Gln Glu Gly Leu Tyr Asn Glu Leu Gln Lys Asp Lys Met Ala Glu
        435                 440                 445

Ala Tyr Ser Glu Ile Gly Met Lys Gly Glu Arg Arg Arg Gly Lys Gly
    450                 455                 460

His Asp Gly Leu Tyr Gln Gly Leu Ser Thr Ala Thr Lys Asp Thr Tyr
465                 470                 475                 480

Asp Ala Leu His Met Gln Ala Leu Pro Pro Arg
                485                 490


<210>    37
<211>    45
<212>    DNA
<213>    Artificial Sequence

<220>
<223>    Linker nucleotide


<400>    37
ggtggaggcg gttcaggcgg aggtggatcc ggcggtggcg gatcg                    45


<210>    38
<211>    15
<212>    PRT
<213>    Artificial Sequence

<220>
<223>    Linker amino acid


<400>    38
Gly Gly Gly Gly Ser Gly Gly Gly Gly Ser Gly Gly Gly Gly Ser
1               5                   10                  15


<210>    39
<211>    116

<212>    PRT
<213>    Artificial Sequence

<220>
<223>    MS501 scFv_heavy chain


<400>    39
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asn Tyr
            20                  25                  30

Ala Met Ser Trp Val Arg Gln Ala Pro Gly Lys Gly Leu Glu Trp Val
        35                  40                  45

Ser Gly Ile Tyr Pro Asp Ser Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Asn Ile Tyr Thr Phe Asp Tyr Trp Gly Gln Gly Thr Leu Val
            100                 105                 110

Thr Val Ser Ser
        115


<210>    40
<211>    111
<212>    PRT
<213>    Artificial Sequence

<220>
<223>    MS501 scFv_light chain


<400>    40
Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Ala Val Ser Trp Tyr Gln Gln Leu Pro Gly Thr Ala Pro Lys Leu Leu
        35                  40                  45

Ile Tyr Tyr Asn Asn Gln Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Gly Ser Trp Asp Ser Ser Leu
                85                  90                  95

Ser Gly Tyr Val Phe Gly Gly Gly Thr Lys Leu Thr Val Leu Gly
            100                 105                 110

<210>    41
<211>    116
<212>    PRT
<213>    Artificial Sequence

<220>
<223>    MS503 scFv_heavy chain

<400>    41
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asn Tyr
            20                  25                  30

Ala Met Ser Trp Val Arg Gln Ala Pro Gly Lys Gly Leu Glu Trp Val
        35                  40                  45

Ser Ser Ile Tyr Pro Gly Asp Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Asn Ala Phe Thr Phe Asp Tyr Trp Gly Gln Gly Thr Leu Val
            100                 105                 110

Thr Val Ser Ser
        115

<210>    42
<211>    111
<212>    PRT
<213>    Artificial Sequence

<220>
<223>    MS503 scFv_light chain

<400>    42
Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Ala Val Ser Trp Tyr Gln Gln Leu Pro Gly Thr Ala Pro Lys Leu Leu
        35                  40                  45

Ile Tyr Tyr Asn Ser His Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Gly Thr Trp Asp Ser Ser Leu
                85                  90                  95

Ser Gly Tyr Val Phe Gly Gly Gly Thr Lys Leu Thr Val Leu Gly
            100                 105                 110

<210>  43
<211>  116
<212>  PRT
<213>  Artificial Sequence

<220>
<223>  C2G4 scFv_heavy chain

<400>  43
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asn Tyr
            20                  25                  30

Ala Met Ser Trp Val Arg Gln Ala Pro Gly Lys Gly Leu Glu Trp Val
        35                  40                  45

Ser Gly Ile Pro Pro Asp Ser Gly Ser Lys Tyr Tyr Ala Asp Ser Val
    50                  55                  60

Arg Gly Arg Phe Thr Val Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Asn Met Leu Ser Phe Asp Tyr Trp Gly Gln Gly Thr Leu Val
            100                 105                 110

Thr Val Ser Ser
        115

<210>  44
<211>  111
<212>  PRT
<213>  Artificial Sequence

<220>
<223>  C2G4 scFv_light chain

<400>  44
Gln Ser Val Leu Thr Gln Pro Pro Ser Ala Ser Gly Thr Pro Gly Gln
1               5                   10                  15

Arg Val Thr Ile Ser Cys Thr Gly Ser Ser Ser Asn Ile Gly Ser Asn
            20                  25                  30

Ala Val Ser Trp Tyr Gln Gln Leu Pro Gly Thr Ala Pro Lys Leu Leu
        35                  40                  45

Ile Tyr Tyr Asn Ser Lys Arg Pro Ser Gly Val Pro Asp Arg Phe Ser

50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Gly Ser Trp Asp Pro Ser Leu
                85                  90                  95

Asn Gly Tyr Val Phe Gly Gly Gly Thr Lys Val Thr Val Leu Gly
            100                 105                 110

<210>   45
<211>   5
<212>   PRT
<213>   Artificial Sequence

<220>
<223>   MS501_variable heavy chain CDR1

<400>   45
Asn Tyr Ala Met Ser
1               5

<210>   46
<211>   17
<212>   PRT
<213>   Artificial Sequence

<220>
<223>   MS501_variable heavy chain CDR2

<400>   46
Gly Ile Tyr Pro Asp Ser Gly Ser Thr Tyr Tyr Ala Asp Ser Val Lys
1               5                   10                  15

Gly

<210>   47
<211>   7
<212>   PRT
<213>   Artificial Sequence

<220>
<223>   MS501_variable heavy chain CDR3

<400>   47
Asn Ile Tyr Thr Phe Asp Tyr
1               5

<210>   48
<211>   13
<212>   PRT
<213>   Artificial Sequence

<220>
<223>    MS501_variable light chain CDR1

<400>    48
Ser Gly Ser Ser Ser Asn Ile Gly Ser Asn Ala Val Ser
1               5                   10

<210>    49
<211>    7
<212>    PRT
<213>    Artificial Sequence

<220>
<223>    MS501_variable light chain CDR2

<400>    49
Tyr Asn Asn Gln Arg Pro Ser
1               5

<210>    50
<211>    11
<212>    PRT
<213>    Artificial Sequence

<220>
<223>    MS501_variable light chain CDR3

<400>    50
Gly Ser Trp Asp Ser Ser Leu Ser Gly Tyr Val
1               5                   10

<210>    51
<211>    5
<212>    PRT
<213>    Artificial Sequence

<220>
<223>    MS503_variable heavy chain CDR1

<400>    51
Asn Tyr Ala Met Ser
1               5

<210>    52
<211>    17
<212>    PRT
<213>    Artificial Sequence

<220>
<223>    MS503_variable heavy chain CDR2

<400>    52
Ser Ile Tyr Pro Gly Asp Gly Ser Thr Tyr Tyr Ala Asp Ser Val Lys
1               5                   10                  15

Gly

<210>	53
<211>	7
<212>	PRT
<213>	Artificial Sequence

<220>
<223>	MS503_variable heavy chain CDR3

<400>	53
Asn Ala Phe Thr Phe Asp Tyr
1               5

<210>	54
<211>	13
<212>	PRT
<213>	Artificial Sequence

<220>
<223>	MS503_variable light chain CDR1

<400>	54
Ser Gly Ser Ser Ser Asn Ile Gly Ser Asn Ala Val Ser
1               5                   10

<210>	55
<211>	7
<212>	PRT
<213>	Artificial Sequence

<220>
<223>	MS503_variable light chain CDR2

<400>	55
Tyr Asn Ser His Arg Pro Ser
1               5

<210>	56
<211>	11
<212>	PRT
<213>	Artificial Sequence

<220>
<223>	MS503_variable light chain CDR3

<400>	56
Gly Thr Trp Asp Ser Ser Leu Ser Gly Tyr Val
1               5                   10

<210>	57
<211>	5

<212>    PRT
<213>    Artificial Sequence

<220>
<223>    C2G4_variable heavy chain CDR1


<400>    57
Asn Tyr Ala Met Ser
1               5


<210>    58
<211>    17
<212>    PRT
<213>    Artificial Sequence

<220>
<223>    C2G4_variable heavy chain CDR2


<400>    58
Gly Ile Pro Pro Asp Ser Gly Ser Lys Tyr Tyr Ala Asp Ser Val Arg
1               5                   10                  15

Gly



<210>    59
<211>    7
<212>    PRT
<213>    Artificial Sequence

<220>
<223>    C2G4_variable heavy chain CDR3


<400>    59
Asn Met Leu Ser Phe Asp Tyr
1               5


<210>    60
<211>    13
<212>    PRT
<213>    Artificial Sequence

<220>
<223>    C2G4_variable light chain CDR1


<400>    60
Thr Gly Ser Ser Ser Asn Ile Gly Ser Asn Ala Val Ser
1               5                   10


<210>    61
<211>    7
<212>    PRT
<213>    Artificial Sequence

<220>

<223>    C2G4_variable light chain CDR2

<400>    61
Tyr Asn Ser Lys Arg Pro Ser
1               5

<210>    62
<211>    11
<212>    PRT
<213>    Artificial Sequence

<220>
<223>    C2G4_variable light chain CDR3

<400>    62
Gly Ser Trp Asp Pro Ser Leu Asn Gly Tyr Val
1               5                   10